# EUROPEAN PATENT SPECIFICATION

(11) **EP 1 542 725 B1**
(45) Date of publication and mention of the grant of the patent: **09.10.2013**
(21) Application number: 03791650.9
(22) Date of filing: 06.08.2003
(51) Int. Cl.: A61K 39/395, C12P 21/04, C12P 21/08, C07H 21/04, C07K 16/18, C07K 16/28, C07K 16/46

(54) **METHOD OF HUMANIZING IMMUNE SYSTEM MOLECULES**
VERFAHREN ZUR HUMANISIERUNG VON IMMUNSYSTEM-MOLEKÜLEN
PROCEDE D'HUMANISATION DE MOLECULES DU SYSTEME IMMUNITAIRE

(30) Priority: 29.08.2002 US 230880
(43) Date of publication of application: 22.06.2005
(73) Proprietor: Altor BioScience Corporation, Miramar, FL 33025-3958 (US)
(72) Inventor: WONG, Hing C., Fort Lauderdale, FL 33332 (US); STINSON, Jeffrey R., Brookeville, MD 20877 (US); MOSQUERA, Luis A., Miami, FL 33196 (US)
(74) Representative: Smart, Peter John
(86) International application number: PCT/US2003/024637
(87) International publication number: WO 2004/020579

(56) References cited:
- WO-A-98/40408
- WO-A-98/45331
- WO-A-03/002607
- US-A- 5 530 101
- US-A- 5 889 157
- US-B1- 6 180 370
- US-B1- 6 610 293
- LEUNG S-O ET AL: "Construction and characterization of a humanized, internalizing, B-cell (CD22)-specific, leukemia/lymphoma antibody, LL2" MOLECULAR IMMUNOLOGY, ELMSFORD, NY, US, vol. 32, no. 17/18, December 1995 (1995-12), pages 1413-1421,1423, XP002046722 ISSN: 0161-5890
- SATO K ET AL: "HUMANIZATION OF A MOUSE ANTI-HUMAN INTERLEUKIN-6 RECEPTOR ANTIBODY COMPARING TWO METHODS FOR SELECTING HUMAN FRAMEWORK REGIONS" MOLECULAR IMMUNOLOGY, ELMSFORD, NY, US, vol. 31, no. 5, April 1994 (1994-04), pages 371-381, XP001069947 ISSN: 0161-5890
- KIPRIYANOV S M ET AL: "GENERATION OF RECOMBINANT ANTIBODIES" MOLECULAR BIOTECHNOLOGY, TOTOWA, NJ, US, vol. 12, no. 2, September 1999 (1999-09), pages 173-201, XP009006067 ISSN: 1073-6085
- BENHAR I. ET AL: 'Rapid humanization of the Fv of monoclonal antibody B3 by using framework exchange of the recombinant immunotoxin B3(Fv)-PE38' PROC. NATL. ACAD. SCI. USA vol. 91, December 1994, pages 12051 - 12055, XP002978443
- COUTO J.R. ET AL: 'Anti-BA46 Monoclonal Antibody Mc3: Humanization Using a Novel Positional Consensus and in Vivo and in Vitro Characterization' CANCER RESEARCH vol. 55, 15 April 1995, pages 1717 - 1722, XP000919432
- RIPPMANN J.F. ET AL: 'Fusion of the Tissue factor Extracellular Domain to a Tumor stroma Specific Single-Chain Fragment Variable Antibody Results in an Antigen-Specific Coagulation-Promoting Molecule' BIOCHEM. J. vol. 349, 2000, pages 805 - 812, XP001021508

### SEQUENCE LISTING

(1) GENERAL INFORMATION
   (i) APPLICANT:
   (ii) TITLE OF THE INVENTION: Method of Humanizing Immune System Molecules
   (iii) NUMBER OF SEQUENCES: 26
   (iv) CORRESPONDENCE ADDRESS:
      (A) ADDRESSEE:
      (B) STREET:
      (C) CITY:
      (D) STATE:
      (E) COUNTRY:
      (F) ZIP:
   (v) COMPUTER READABLE FORM:
      (A) MEDIUM TYPE: Diskette
      (B) COMPUTER: IBM Compatible
      (C) OPERATING SYSTEM: DOS
      (D) SOFTWARE: FastSEQ Version 1.5
   (vi) CURRENT APPLICATION DATA:
      (A) APPLICATION NUMBER:
      (B) FILING DATE:
      (C) CLASSIFICATION:
   (vii) PRIOR APPLICATION DATA:
      (A) APPLICATION NUMBER:
      (B) FILING DATE:
   (viii) ATTORNEY/AGENT INFORMATION:
      (A) NAME:
      (B) REGISTRATION NUMBER:
      (C) REFERENCE/DOCKET NUMBER:
   (ix) TELECOMMUNICATION INFORMATION:
      (A) TELEPHONE:
      (B) TELEFAX:
      (C) TELEX:
(2) INFORMATION FOR SEQ ID N:1:
   (i) SEQUENCE CHARACTERISTICS:
      (A) LENGTH: 321 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTISENSE: NO
   (v) FRAGMENT TYPE:
   (vi) ORIGINAL SOURCE:
   (xi) SEQUENCE DESCRIPTION: SEQ ID NO:1:
   GACATTCAGA TGACCCAGTC TCCTGCCTCC CAGTCTGCAT CTCTGGGAGA AAGTGTCACC   60
   ATCACATGCC TGGCAAGTCA GACCATTGAT ACATGGTTAG CATGGTATCA GCAGAAACCA  120
   GGGAAATCTC CTCAGCTCCT GATTTATGCT GCCACCAACT TGGCAGATGG GGTCCCATCA  180
   AGGTTCAGTG GCAGTGGATC TGGCACAAAA TTTTCTTTCA AGATCAGCAG CCTACAGGCT  240
   GAAGATTTTG TAAATTATTA CTGTCAACAA GTTTACAGTT CTCCATTCAC GTTCGGTGCT  300
   GGGACCAAGC TGGAGCTGAA A                                            321
(2) INFORMATION FOR SEQ ID NO:2:
   (i) SEQUENCE CHARACTERISTICS:
      (A) LENGTH: 107 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (iv) ANTISENSE: NO
   (v) FRAGMENT TYPE: N-terminal
   (vi) ORIGINAL SOURCE:
   (xi) SEQUENCE DESCRIPTION: SEQ ID NO:2:
   Asp Ile Gln Met Thr Gln Ser Pro Ala Ser Gln Ser Ala Ser Leu Gly
    1               5                  10                  15
   Glu Ser Val Thr Ile Thr Cys Leu Ala Ser Gln Thr Ile Asp Thr Trp
               20                  25                  30
   Leu Ala Trp Tyr Gln Gln Lys Pro Gly Lys Ser Pro Gln Leu Leu Ile
           35                  40                  45
   Tyr Ala Ala Thr Asn Leu Ala Asp Gly Val Pro Ser Arg Phe Ser Gly
       50                  55                  60
   Ser Gly Ser Gly Thr Lys Phe Ser Phe Lys Ile Ser Ser Leu Gln Ala
   65                  70                  75                  80
   Glu Asp Phe Val Asn Tyr Tyr Cys Gln Gln Val Tyr Ser Ser Pro Phe
                   85                  90                  95
   Thr Phe Gly Ala Gly Thr Lys Leu Glu Leu Lys
               100                 105
(2) INFORMATION FOR SEQ ID NO:3:
   (i) SEQUENCE CHARACTERISTICS:
      (A) LENGTH: 351 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTISENSE: NO
   (v) FRAGMENT TYPE:
   (vi) ORIGINAL SOURCE:
   (xi) SEQUENCE DESCRIPTION: SEQ ID NO:3:
   GAGATCCAGC TGCAGCAGTC TGGACCTGAG CTGGTGAAGC CTGGGGCTTC AGTGCAGGTA    60
   TCCTGCAAGA CTTCTGGTTA CTCATTCACT GACTACAACG TGTACTGGGT GAGGCAGAGC   120
   CATGGAAAGA GCCTTGAGTG GATTGGATAT ATTGATCCTT ACAATGGTAT TACTATCTAC   180
   GACCAGAACT TCAAGGGCAA GGCCACATTG ACTGTTGACA AGTCTTCCAC CACAGCCTTC   240
   ATGCATCTCA ACAGCCTGAC ATCTGACGAC TCTGCAGTTT ATTTCTGTGC AAGAGATGTG   300
   ACTACGGCCC TTGACTTCTG GGGCCAAGGC ACCACTCTCA CAGTCTCCTC A            351
(2) INFORMATION FOR SEQ ID NO:4:
   (i) SEQUENCE CHARACTERISTICS:
      (A) LENGTH: 117 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (iv) ANTISENSE: NO
   (v) FRAGMENT TYPE: N-terminal
   (vi) ORIGINAL SOURCE:
   (xi) SEQUENCE DESCRIPTION: SEQ ID NO:4:
   Glu Ile Gln Leu Gln Gln Ser Gly Pro Glu Leu Val Lys Pro Gly Ala
    1               5                  10                  15
   Ser Val Gln Val Ser Cys Lys Thr Xaa Gly Tyr Ser Phe Thr Asp Tyr
               20                  25                  30
   Asn Val Tyr Trp Val Arg Gln Ser His Gly Lys Ser Leu Glu Trp Ile
           35                  40                  45
   Gly Tyr Ile Asp Pro Tyr Asn Gly Ile Thr Ile Tyr Asp Gln Asn Phe
       50                  55                  60
   Lys Gly Lys Ala Thr Leu Thr Val Asp Lys Ser Ser Thr Thr Ala Phe
   65                  70                  75                  80
   Met His Leu Asn Ser Leu Thr Ser Asp Asp Ser Ala Val Tyr Phe Cys
                   85                  90                  95
   Ala Arg Asp Val Thr Thr Ala Leu Asp Phe Trp Gly Gln Gly Thr Thr
               100                 105                 110
   Leu Thr Val Ser Ser
           115
(2) INFORMATION FOR SEQ ID NO:5:
   (i) SEQUENCE CHARACTERISTICS:
      (A) LENGTH: 7 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (iv) ANTISENSE: NO
   (v) FRAGMENT TYPE: N-terminal
   (vi) ORIGINAL SOURCE:
   (xi) SEQUENCE DESCRIPTION: SEQ ID NO:5:
   Leu Ala Ser Gln Thr Ile Asp
    1               5
(2) INFORMATION FOR SEQ ID NO:6:
   (i) SEQUENCE CHARACTERISTICS:
      (A) LENGTH: 7 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (iv) ANTISENSE: NO
   (v) FRAGMENT TYPE: N-terminal
   (vi) ORIGINAL SOURCE:
   (xi) SEQUENCE DESCRIPTION: SEQ ID NO:6:
   Ala Ala Thr Asn Leu Ala Asp
    1               5
(2) INFORMATION FOR SEQ ID NO:7:
   (i) SEQUENCE CHARACTERISTICS:
      (A) LENGTH: 9 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (iv) ANTISENSE: NO
   (v) FRAGMENT TYPE: N-terminal
   (vi) ORIGINAL SOURCE:
   (xi) SEQUENCE DESCRIPTION: SEQ ID NO:7:
   Gln Gln Val Tyr Ser Ser Pro Phe Thr
    1               5
(2) INFORMATION FOR SEQ ID NO:8:
   (i) SEQUENCE CHARACTERISTICS:
      (A) LENGTH: 6 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (iv) ANTISENSE: NO
   (v) FRAGMENT TYPE: N-terminal
   (vi) ORIGINAL SOURCE:
   (xi) SEQUENCE DESCRIPTION: SEQ ID NO:8:
   Thr Asp Tyr Asn Val Tyr
    1               5
(2) INFORMATION FOR SEQ ID NO:9:
   (i) SEQUENCE CHARACTERISTICS:
      (A) LENGTH: 17 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (iv) ANTISENSE: NO
   (v) FRAGMENT TYPE: N-terminal
   (vi) ORIGINAL SOURCE:
   (xi) SEQUENCE DESCRIPTION: SEQ ID NO:9:
   Tyr Ile Asp Pro Tyr Asn Gly Ile Thr Ile Tyr Asp Gln Asn Phe Lys
    1               5                   10                  15
   Gly
(2) INFORMATION FOR SEQ ID NO:10:
   (i) SEQUENCE CHARACTERISTICS:
      (A) LENGTH: 8 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (iv) ANTISENSE: NO
   (v) FRAGMENT TYPE: N-terminal
   (vi) ORIGINAL SOURCE:
   (xi) SEQUENCE DESCRIPTION: SEQ ID NO:10:
   Asp Val Thr Thr Ala Leu Asp Phe
    1               5
(2) INFORMATION FOR SEQ ID NO:11:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTISENSE: NO
   (v) FRAGMENT TYPE:
   (vi) ORIGINAL SOURCE:
   (xi) SEQUENCE DESCRIPTION: SEQ ID NO:11:
      CTGGCAAGTC AGACCATTGA T 21
(2) INFORMATION FOR SEQ ID NO:12:
   (i) SEQUENCE CHARACTERISTICS:
      (A) LENGTH: 21 base pairs
      (B) TYPE: nucleic acid
      (C) STRANDEDNESS: single
      (D) TOPOLOGY: linea
   (ii) MOLECULE TYPE: DNA
   (iii) HYPOTHETICAL: NO
   (iv) ANTISENSE: NO
   (v) FRAGMENT TYPE:
   (vi) ORIGINAL SOURCE:
   (xi) SEQUENCE DESCRIPTION: SEQ ID NO:12:
      GCTGCCACCA ACTTGGCAGA T 21
(2) INFORMATION FOR SEQ ID NO:13:
   (i) SEQUENCE CHARACTERISTICS:
      (A) LENGTH: 28 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTISENSE: NO
   (v) FRAGMENT TYPE:
   (vi) ORIGINAL SOURCE:
   (xi) SEQUENCE DESCRIPTION: SEQ ID NO:13:
      CAACAAGTTT ACAGTTCTCC ATTCACGT 28
(2) INFORMATION FOR SEQ ID NO:14:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTISENSE: NO
   (v) FRAGMENT TYPE:
   (vi) ORIGINAL SOURCE:
   (xi) SEQUENCE DESCRIPTION: SEQ ID NO:14:
      ACTGACTACA ACGTGTAC 18
(2) INFORMATION FOR SEQ ID NO:15:
   (i) SEQUENCE CHARACTERISTICS:
      (A) LENGTH: 51 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTISENSE: NO
   (v) FRAGMENT TYPE:
   (vi) ORIGINAL SOURCE:
   (xi) SEQUENCE DESCRIPTION: SEQ ID NO:15:
      TATATTGATC CTTACAATGG TATTACTATC TACGACCAGA ACTTCAAGGG C 51
(2) INFORMATION FOR SEQ ID NO:16:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTISENSE: NO
   (v) FRAGMENT TYPE:
   (vi) ORIGINAL SOURCE:
   (xi) SEQUENCE DESCRIPTION: SEQ ID NO:16:
      GATGTGACTA CGGCCCTTGA CTTC 24
(2) INFORMATION FOR SEQ ID NO:17:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTISENSE: NO
   (v) FRAGMENT TYPE:
   (vi) ORIGINAL SOURCE:
   (xi) SEQUENCE DESCRIPTION: SEQ ID NO:17:
      GCACCTCCAG ATGTTAACTG CTC 23
(2) INFORMATION FOR SEQ ID NO:18:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTISENSE: NO
   (v) FRAGMENT TYPE:
   (vi) ORIGINAL SOURCE:
   (xi) SEQUENCE DESCRIPTION: SEQ ID NO:18:
      GAARTAVCCC TTGACCAGGC 20
(2) INFORMATION FOR SEQ ID NO:19:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTISENSE: NO
   (v) FRAGMENT TYPE:
   (vi) ORIGINAL SOURCE:
   (xi) SEQUENCE DESCRIPTION: SEQ ID NO:19:
      GGAGGCGGCG GTTCTGACAT TGTGMTGWCM CARTC 35
(2) INFORMATION FOR SEQ ID NO:20:
   (i) SEQUENCE CHARACTERISTICS:
      (A) LENGTH: 45 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTISENSE: NO
   (v) FRAGMENT TYPE:
   (vi) ORIGINAL SOURCE:
   (xi) SEQUENCE DESCRIPTION: SEQ ID NO:20:
      ATTTCAGGCC CAGCCGGCCA TGGCCGARGT YCARCTKCAR CARYC 45
(2) INFORMATION FOR SEQ ID NO:21:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTISENSE: NO
   (v) FRAGMENT TYPE:
   (vi) ORIGINAL SOURCE:
   (xi) SEQUENCE DESCRIPTION: SEQ ID NO:21:
      CCCGGGCCAC CATGKCCCCW RCTCAGYTYC TKG 33
(2) INFORMATION FOR SEQ ID NO:22:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTISENSE: NO
   (v) FRAGMENT TYPE:
   (vi) ORIGINAL SOURCE:
   (xi) SEQUENCE DESCRIPTION: SEQ ID NO:22:
      CCCGGGCCAC CATGGRATGS AGCTGKGTMA TSCTC 35
(2) INFORMATION FOR SEQ ID NO:23:
   (i) SEQUENCE CHARACTERISTICS:
      (A) LENGTH: 52 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTISENSE: NO
   (v) FRAGMENT TYPE:
   (vi) ORIGINAL SOURCE:
   (xi) SEQUENCE DESCRIPTION: SEQ ID NO:23:
      ATATACTCGC GACAGCTACA GGTGTCCACT CCGAGATCCA GCTGCAGCAG TC 52
(2) INFORMATION FOR SEQ ID NO:24:
   (i) SEQUENCE CHARACTERISTICS:
      (A) LENGTH: 31 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTISENSE: NO
   (v) FRAGMENT TYPE:
   (vi) ORIGINAL SOURCE:
   (xi) SEQUENCE DESCRIPTION: SEQ ID NO:24:
      GACCTGAATT CTAAGGAGAC TGTGAGAGTG G 31
(2) INFORMATION FOR SEQ ID NO:25:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTISENSE: NO
   (v) FRAGMENT TYPE:
   (vi) ORIGINAL SOURCE:
   (xi) SEQUENCE DESCRIPTION: SEQ ID NO:25:
      TTAATTGATA TCCAGATGAC CCAGTCTCC 29
(2) INFORMATION FOR SEQ ID NO:26:
   (i) SEQUENCE CHARACTERISTICS:
      (A) LENGTH: 45 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTISENSE: NO
   (v) FRAGMENT TYPE:
   (vi) ORIGINAL SOURCE:
   (xi) SEQUENCE DESCRIPTION: SEQ ID NO:26:
      TAATCGTTCG AAAAGTGTAC TTACGTTTCA GCTCCAGCTT GGTCC 45

## Description

### FIELD OF THE INVENTION

The present invention features methods for making humanized immune system molecules. In one aspect, the invention provides methods for humanizing antibodies that involve optimizing sequence similarity between individual antibody framework regions rather than a larger framework or variable domain. The invention has a wide spectrum of applications including use in the production of humanized monoclonal antibodies with suitable binding affinity and minimized human immunogenicity.

### BACKGROUND OF THE INVENTION

There is general recognition that antibodies have important uses. For example, many are known to detect antigen with exquisite specificity. Polyclonal and monoclonal antibodies have been reported. See generally Molecular Biology of the Cell (B. Alberts et al. Eds. 2nd edition) (1989) Garland Publishing, Inc. New York and references cited therein.

There has been substantial interest toward understanding antibody structure and function. For instance, much is known about the structure and function of variable (V) domains. Nearly all antibody V domains include hypervariable complementarity determining regions ("CDRs") and framework regions ("FRs"). For most antibody molecules, a single CDR is separated from another CDR by an intervening FR. It is generally recognized that FRs serve as molecular scaffolding that helps position CDR loops in the proper configuration for antigen recognition and binding. See generally B. Alberts *et al., supra,* and references cited therein.

Many workers have used "framework" to describe the FRs in their entirety from a single antibody light chain variable domain (VL) or heavy chain variable domain (VH). Accordingly, one antibody V domain includes individual framework region subsets (FR1, FR2, FR3 and FR4) in which each subset is linked to its corresponding CDR (CDR1, CDR2, and CDR3). A framework consists of the framework region subsets in their entirety from a single antibody.

At least for some antibodies, the amino acid residues in FRs are thought to contibute to antigen binding. See Foote, J. and G. Winter (1992) J. Mol. Biol. 224: 487.

There have been attempts to use monoclonal antibodies as therapeutic agents. However, the approach has been challenging at least with certain antibodies. For example, some human subjects have been reported to develop undesirable immune side reactions following exposure to antibodies derived from non-human sources. These reactions, in some instances, can pose serious health problems and may be life threatening.

There have been attempts to make antibodies more immunologically acceptable to human subjects. Although there are some reports of human monoclonal antibodies, it is generally accepted that such molecules are difficult to make and use.

An alternative strategy for making antibodies more acceptable to humans has been to introduce genes that encode human antibodies into non-human animals (*e. g*. mice). Such "transgenic" animals have been reported to make antibodies with human sequence. However, making such animals has often been difficult and time consuming. Also, these transgenic mice are proprietary and possess less than optimal human antibody gene repertoires.

Another approach for making immunologically acceptable antibodies has been to use recombinant DNA technologies. One concept has been to clone and modify non-human antibodies so that resulting molecules resemble human antibodies. Collectively, such antibodies have been referred to as "humanized". See U.S Pat. Nos. 5,766,886 to Studnicka et al.*;* 5,693,762 to Queen et al.*;* 5,985,279 to Waldeman et al.*;* 5,225,539 to Winter; 5,639,641 to Pedersen, et al.; and references cited therein.

Several specific approaches have been proposed for humanizing antibodies.

One strategy involves making what has been described as a chimeric antibody molecule. Typically, an antigen is used to immunize a non-human animal such as a mouse. Monoclonal antibodies are then made from the animal using conventional techniques. Genes encoding the monoclonal antibody are generated using conventional polymerase chain reaction (PCR) amplification. Isolated sequences encoding murine antibody variable domains are genetically fused by standard recombinant DNA technology to the sequences encoding the constant domains of human antibodies. This strategy has been used to make a human-mouse chimeric antibody. See *e.g.*, S. L.Morrison and V. Oi (1989) Adv. Immunol. 44: 65.

Unfortunately, there have been reports of problems making and using some chimeric antibodies. Specifically, there has been disclosure of unacceptable immune responses after administration of the antibodies to human subjects. Reduced circulatory half-life is also believed to be a problem with at least some chimeric antibodies. See *e.g.,* Boulianne et al. in Nature 312: 643 (1984); Junghans et al. Cancer Res. 50: 1495 (1990) and Bruggemann et al. (1989) J. Exp. Med. 170: 2153.

Additional strategies for making humanized antibodies have been reported.

One approach has been described as "CDR grafting" (sometimes called "framework grafting" or "antibody reshaping"). CDR grafting has been taught to involve inserting murine CDRs into a human V domain. The murine CDRs are then substituted for the corresponding human CDRs. See E. Padlan Mol. Immunol. 28: 489(1991). Usually, all FRs in the V domain are derived from a single human antibody. Some in the field have predicted that human FRs of the framework will correctly position the murine CDRs to bind antigen. The expectation has been that such antibodies will evade recognition by the human immune system. See, *e.g.*, Jones et al., Nature 321: 522-525 (1986); Junghans *et al., supra.*

However, there have been problems using many CDR grafting techniques.

For instance, some "grafted" antibodies have unacceptable antigen binding properties. See Gorman, et al. PNAS (USA) 88: 4181 (1991). Attempts to improve antigen binding, usually by adding murine residues back to CDR grafted V domain, have not always been successful. See Queen et al., PNAS (USA) 86: 10029 (1989); and Co, et al., PNAS (USA) 88: 2869 (1991).

There have been further attempts to produce humanized antibodies using what has been described as "antibody resurfacing". One approach has been to replace exposed amino acid residues of an allogeneic antibody with those usually found in host antibodies. The hope has been to decrease immunogenicity of the antibody while preserving as much antigen binding function as possible. See Roguska, et al. PNAS (USA) 91: 969 (1994).

Unfortunately, there have been problems using many of the prior antibody reshaping techniques.

For instance, many reshaped antibodies are thought to exhibit unacceptable antigen binding properties. See E. Padlan Mol. Immunol. 28: 489(1991). Further, most reshaping techniques require detailed knowledge about the structure of the antibody to be reshaped. Substantial information about solvent accessibility and related parameters is often required. However such information is not always available for antibodies that need to be humanized.

A major drawback shared by most antibody humanization techniques is that for a desired antibody to be humanized, a relatively large framework or V domain is selected for grafting or reshaping manipulations. The concept of using such large antibody segments as the basis for manipulating non-human antibodies has produced undesirable results *e.g.*, reducing antigen binding affinity and retaining unacceptable immunogenicity. To address these deficiencies, additional efforts are employed to restore affinity. These efforts typically end in a compromise between adding enough non-human amino acid residues to boost affinity while not adding so much that immunogenicity is also increased.

More particularly, it is believed that many of the prior humanization techniques involve manipulations that use an unacceptably large antibody framework, or worse, entire V domain to humanize antibodies. According to one approach, a large non-human framework or V domain is used as a query to identify a set of human frameworks with acceptable sequence identity to the query. The identified human framework is often said to be the "best fit" if it exhibits the greatest sequence identify to the query non-human sequence with respect to the search parameters used.

It is believed that past practice of selecting best fit human frameworks based on comparisons with relatively large framework or V domains has hindered attempts to make and use many humanized immune system molecules. For instance, use of the prior antibody humanization methods is believed to have missed many potential "best fit" matches. That is, by limiting the pool of "best fit" matches to framework or V domains, the ability to humanize many antibodies has been compromised.

Additionally, many prior methods for making humanized antibodies have searched for "best fit" matches using complete antibody sequences. However this strategy poses significant disadvantages.

For example, such searching approaches cannot include FR sequences from incompletely sequenced human antibodies. This shortcoming has made it difficult to take advantage of more complete collections of human antibody sequence information.

The prior methods for making humanized antibodies are believed to suffer from other deficiencies.

For instance, there is recognition in the field that many humanized antibodies do not exhibit optimal antigen binding affinity. Steric hinderance (sometimes called interference) between amino acids of the non-human CDRs adjacent to the human FRs and the amino acid residues in key locations in the FRs (referred to as vernier zone residues) is thought to contribute to the problem. Because the prior humanization methods are believed to miss many potential "best fit" human FR sequences, the ability to address these and related interference problems has been limited.

It would be useful to have more effective methods for humanizing immune system molecules. More specifically, it would be useful to have methods for making humanized antibodies that involve optimizing sequence similarity between individual framework subsets as the basis for making humanized immune system molecules with suitable binding affinity and minimized human immunogenicity.

### SUMMARY OF THE INVENTION

The present invention features effective and useful methods for humanizing immune system molecules. In one aspect, the invention provides methods for making humanized antibodies and fragments thereof comprising a variable (V) domain that optimize sequence similarity between individual framework region subsets (FRs). Comparisons between larger antibody frameworks and/or variable domains is avoided and selected "best fit" human FRs are used to make the humanized immune system molecules. The invention has a wide variety of important applications including use in making humanized immunoglobulins with suitable antigen affinity and minimized human immunogenicity.

We have discovered a novel method for "humanizing" a broad spectrum of immune system molecules *e.g*., antibodies and fragments thereof comprising a variable domain. The method involves optimizing sequence similarity between at least one non-human FR subset and at least one corresponding human FR. Sequence similarity is optimized for all four of the FRs (*i.e*., FR1, FR2, FR3 and FR4). Such sequence similarity can be optimized by considering one FR at a time (*e.g*., consecutive FRs like FR1 and FR2) or more than one at a time (*e.g*., all four FRs together) as needed to suit intended use. One or more identified human FR subsets that have the highest sequence similarity to the corresponding non-human FR subset is selected for further manipulation. The selected human FR subset is often referred to as being the "best fit" with respect to the non-human FR subset to which it has been compared. Practice of the invention further involves substituting the "best fit" human FR subsets for the corresponding non-human FR subsets. Preferred use of the invention is thus intended to maximize sequence similarity between all of the individual human and non-human FR subsets. Optimizing sequence similarity at the level of framework sets (*i.e.,* FR1, FR2, FR3 and FR4 together) and whole variable domains is avoided. Selected best fit human FR subsets are subsequently used as components for assembling the humanized immune system molecules disclosed herein.

The present invention provides many important advantages.

For example, prior practice has often detected best fit human frameworks (*i.e*., FR1-4) based on similarity between entire framework sets or antibody variable domains. This approach is believed to have hampered efforts to make and use humanized immunoglobulins *e.g*., by causing a lack of FR selection sensitivity. Moreover, many best fit matches between individual human FRs have been missed.

More specifically, it is believed that prior humanization methods have severely limited the number of best fit possibilities. In contrast, practice of the invention is not so constrained. That is, optimal use of the invention involves identifying and maximizing sequence similarity at the level of individual FR subsets. Less sensitive sequence comparisons between much larger framework sets and/or antibody variable domains are specifically avoided. These and related advantages provided by the invention expand the number of best fit possibilities, typically by the arithmetic factorial of the number of FR subsets on the immune system molecule to be humanized. This feature of the invention substantially enhances opportunities to make and use humanized molecules, helps lower costs associated with humanization procedures, and provides a rational basis for assembling nearly any humanized immune system molecule of interest.

By way of illustration, the present invention can be used to humanize a desired non-human antibody. In this example, the pool of available human FR subsets for sequence comparison is the arithmetic factorial of each of the four framework subsets (FR1, FR2, FR3 and FR4) on each of the light and heavy chains for which sequence information is available (including, significantly, FR sequence information from incomplete variable domain sequences). A key result is an increase in pool size of at least about four fold. In some embodiments, the pool size will be even larger due to availability of best fit FRs from incomplete V domain sequences. This is a significant advantage over prior methods, since it boosts the size of the pool of human FRs subsets available for selection. Potential best fit human FR possibilities are thus enhanced. Accordingly, the chances of detecting a best fit human FR subset is higher according to the invention when compared to past approaches.

Practice of the present invention provides other significant advantages.

As discussed, it is an object of the invention to optimize and select the best fit for each non-human FR subset to be humanized. This process, unlike past approaches, is conducted essentially independently of the other FRs within the subject immune system molecule. Thus, the number of mismatched amino acids, to the extent they exist at all, will be substantially lower by using the invention method. Unlike past methods which are often limited by the pool of fully sequenced human antibodies and variable domains available, use of the invention provides new opportunities for selecting best fit human FR subsets. The invention further provides better chances of detecting best fit human FRs with no or few mismatches when compared to the corresponding non-human FR. Practice of the present methods will thus enhance opportunities for making and using a wide variety of immune system molecules. Importantly, such methods can be used to prepare humanized antibodies and fragments thereof comprising a variable domain whose antigen binding affinity is substantially preserved when compared to a parental non-human or chimeric molecule from which it was obtained.

Accordingly, and in one aspect, the invention provides a method for producing a humanized antibody variable (V) domain. In most embodiments, the method includes at least one and preferably all of the following steps.

Typically, at least one amino acid sequence of a desired non-human antibody FR subset, preferably less than about four of such FRs, more preferably about one FR such as FR1 or FR2, is compared to a pool of corresponding human amino acid antibody or variable domain sequences or fragments thereof. In most instances, a collection of corresponding human antibody framework amino acid sequences is used for the comparison although other collections may be used for some applications including collections of partial framework sequences. A corresponding human FR subset is selected from the pool that has the best fit with respect to the corresponding non-human FR subset. Generally, a suitable best fit human FR subset will exhibit at least about 75% sequence identity, preferably at least about 80% sequence identity, more preferably at least about 90% up to about 100% sequence identity when compared with the corresponding non-human FR subset.

Subsequently, the non-human FR subset is mutagenized to encode essentially the best fit human FR subset identified above. One or a combination of standard mutagenesis procedures can be used as discussed below. Preferably, mutagenesis of the non-human FR subset is conducted to produce a humanized FR subset (sometimes called "huFR") that is substantially identical to the selected human FR subset *i.e.*, at least about 75% identical, preferably at least about 80% identical, more preferably at least about 90% identical up to about 100% identical to the selected subset.

The prior humanization steps of the invention can be repeated, as needed, to humanize one or more desired FR subsets of the non-human V domain. More specifically, the steps can be repeated one, two, three, or even more times, and under conditions that produce a plurality of DNA sequences in which each DNA sequence encodes a corresponding huFR subset. In most instances (*e.g.*, as when a fully humanized immune system molecule is required), it will be useful to repeat the steps about three to four times to humanize each of the FR subsets (FR1, FR2, FR3, and FR4). Thus in embodiments in which a non-human antibody is of interest, each of the FR subsets on each of the immunoglobulin light and heavy chains will be subjected to the aforementioned humanization steps.

Successful practice of the invention does not depend on any particular order of humanizing the non-human FRs so long as intended results are achieved.

One or a combination of the DNA sequences encoding the huFR subsets is then substituted into a suitable (first) vector using standard recombinant approaches. Preferably, the first vector encodes at least the V domain of the non-human antibody to be humanized. However in some embodiments, the first vector further encodes an immunoglobulin light or heavy chain constant domain or a fragment thereof covalently linked to the V domain.

Preferred substitution steps are generally conducted under conditions in which at least one and preferably all of the huFR DNA sequences is used as a replacement for one or more corresponding non-human FR subsets encoded by the vector. The order of FR substitution is usually not important provided the intended humanized molecule is produced. Thus for example, a non-human FR1 can be humanized first followed by non-human FR2. Alternatively, the non-human FR2 can be humanized before the non-human FR1. Preferred substitution steps are conducted according to conventional manipulations and result in each of the huFR subsets being operatively linked to one or more corresponding complimentarity determining regions (CDR).

The first vector can be employed to express one or more encoded humanized immune system molecules in a wide spectrum of suitable host cells as discussed below. Generally preferred methods are conducive to expressing the humanized antibody V domain in the host cells. Additionally preferred methods are fully compatible with approaches intended to purify the humanized molecule from cell constituents which accompany it.

The invention is fully compatible with a wide range of recombinant approaches that convert a non-human amino acid at each mismatched position on a given non-human FR subset to a desired human amino acid. An example of an acceptable recombinant approach is site-directed mutagenesis or related PCR-based method. Because the pool of potential best fit human FR subsets is greater according to the invention, there is a much higher probability of identifying a human FR subset with fewer mismatches. This feature of the invention helps reduce the number of FR mismatches that need to be considered when a particular immune system molecule is to be humanized. These and other invention advantages help reduce cost and time expenditures that are typical of many prior humanization methods.

As discussed, most prior humanization approaches have been hindered by unacceptable antigen binding of the resulting molecules. More specifically, most prior humanized antibodies exhibit too low an affinity for cognate antigen when compared to the parental molecule. Many parental chimeric antibodies have better antigen binding characteristics than the humanized version of the antibody. Without wishing to be bound to theory, it is believed that such unacceptable antigen binding can be attributed, in large part, to hindrance stemming from one or more amino acids in the FR subsets proximal to the CDRs (the vernier zone residues). There are reports that at least for some antibodies, the vernier zone residues may contribute to antigen binding by the antibody V domain.

Past attempts to minimize vernier zone hindrance problems have been largely unsuccessful or require sophisticated computer algorithms to correct and these rely heavily on computer-based molecular modeling and structural knowledge.

For example, in one approach, computer-assisted modeling software has been used to help reduce the hindrance. Such software is often complicated and difficult to use. The present invention addresses the problem by optimizing the detection and selection of best fit huFR subsets without assistance from complicated software. That is, the invention provides more best fit candidates, thereby decreasing chances for mismatches in the vernier zone and elsewhere in the immune system molecule to be humanized. Accordingly, use of the invention can significantly maintain antigen binding affinity of the parental immune system molecule. This invention advantage is especially important when a non-human antibody of interest has an unusually high affinity (Kd >10⁹M or greater) for antigen. In such situations, high affinity often places demands on maintaining the CDR in the appropriate configuration. The invention provides for substantial compliance with these demands by minimizing or eliminating potential for vernier zone incompatibilities. Past practice has used X-ray crystallographic or computer based antibody information to help address the vernier zone incompatibilities. Significantly, the invention reduces and in many instances avoids user reliance on this information.

Other aspects of the invention are discussed *infra.*

### BRIEF DESCRIPTION OF THE DRAWINGS

Figs. 1A and 1B shows the nucleic acid (SEQ ID NOS 1 and 3) and amino acid (SEQ ID NOS 2 and 4) sequences of light chain and heavy chain variable domains of H36.D2.B7, the murine anti-tissue factor antibody, with hypervariable regions (CDRs or Complementarity Determining Regions) underlined (single underline for nucleic acid sequences and double underline for amino acid sequences).
Fig. 2 is a drawing showing a plasmid map of humanized anti-TF IgG1 antibody expression vector (pSUN-34).
Figs. 3A-D are sequences of partially and fully humanized light chain (LC) variable domains of the anti-TF antibody (SEQ ID NOS 72-82). Fig. 3A shows the sequence named "LC-09" which is representative of a fully humanized LC framework (SEQ ID NO: 79). Light chain CDR sequences of cH36 and LC-09 are shown in Figs. 3B-D (SEQ ID NOS 103,6 and 7, respectively).
Figs. 4A-D are drawings showing the sequences of partially and fully humanized heavy chain (HC) variable domains of the anti-TF antibody (SEQ ID NOS 83-96). Fig. 4A shows the sequence named "HC-08" which is representative of a fully humanized HC framework (SEQ ID NO: 91). Heavy chain CDR sequences for cH36 and HC-08 are shown in Figs. 4B-D (SEQ ID NOS 104, 104, 9, 101, 10 and 10, respectively).
Figs. 5A-B are sequences showing human constant domains in the IgG1 anti-tissue factor antibody (hOAT), with Fig. 5A showing the human kappa light chain constant domain (SEQ ID NO: 97) and Fig. 5B showing the human IgG1 heavy chain constant domain (SEQ ID NO: 98). The figures show hOAT (IgG1) constant domain amino acid sequences.
Figs. 6A-B are sequences showing human constant domains in the IgG4 anti-tissue factor antibody (hFAT) with Fig. 6A showing the human kappa light chain constant domain (SEQ ID NO: 99) and Fig. 6B showing the human IgG4 heavy chain constant domain (SEQ ID NO: 100).
Figs. 7A and 7B shows the nucleic acid (SEQ ID NOS 105 and 107) and amino acid (SEQ ID NOS 106 and 108) sequences of light chain and heavy chain variable domains of A110, the chimeric anti-LTA antibody, with hypervariable regions (CDRs or Complementarity Determining Regions) underlined (single underline for nucleic acid sequences and double underline for amino acid sequences).
Fig. 8 is a drawing showing a plasmid map of an expression vector encoding humanized anti-LTA IgG1 (pJRS 391).
Figs. 9A-H are drawings showing sequences of partially and fully humanized variable domains of the anti-LTA antibody. Fig. 9A shows the sequence of the humanized light chain (LC) variable domain framework regions (SEQ ID NOS 109-114, respectively). Figs. 9B-D show light chain CDRs1-3 (SEQ ID NOS 115-117, respectively). Fig. 9E shows the sequences of partially and fully humanized heavy chain (HC) variable domain framework regions (SEQ ID NOS 118-123, respectively). Figs. 9F-K show heavy chain CDRs1-3 (SEQ ID NOS 124-126, respectively).
Fig. 10 is a table showing plasmid constructs producing humanized A110 antibody for evaluation.
Figs. 11 is a graph showing determination of antibody expression by humanized anti-LTA by different plasmids.

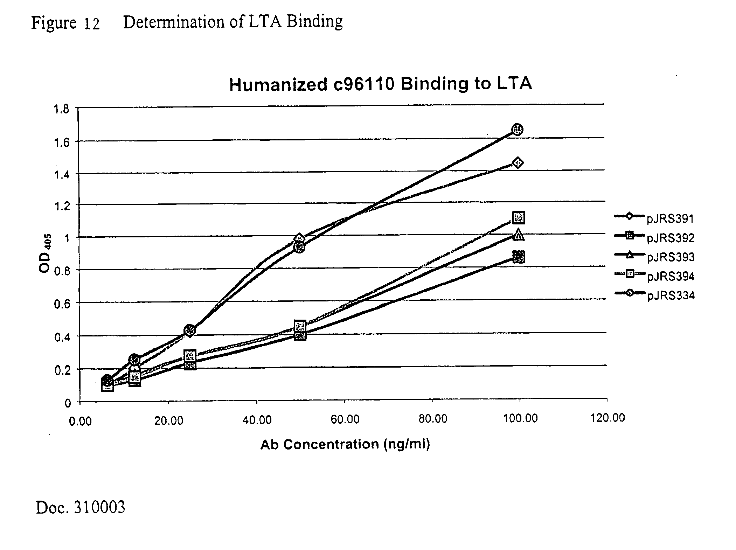

Fig. 12 is a graph showing determination of LTA binding.

### DETAILED DESCRIPTION OF THE INVENTION

As discussed, the invention features methods for making humanized immune system molecules. Examples of such molecules include humanized antibody variable (V) domains, humanized antibodies (chimeric and monoclonal) as well as antigen-binding fragments thereof. For instance, the invention provides new methods for humanizing antibodies that involves optimizing sequence similarity between at least one non-human framework (FR) subset, typically two, three or four of such subsets, and one or more corresponding human FR subsets. At least one selected humanized FR subset (huFR) is substituted for the corresponding non-human FR subset to humanize the molecule. Preferred humanized immune system molecules of the invention feature good antigen binding affinity and minimal immunogenicity in human subjects.

The advantages of using the present invention to humanize immune system molecules is demonstrated throughout the disclosure including Examples 6-9, below. As discussed, a key difference between past humanization attempts and the present invention is that the invention methods searches for FRs with the best fit. In contrast, the prior attempts have used entire frameworks (usually derived from a single antibody variable domain) or worse, entire variable domains to determine best fits. Thus with the present methods identified FRS can be derived from as many as four different antibodies for each antibody chain, or a total of eight FRs per antibody. This feature of the invention greatly expands the available pool of detectable best fit FRs. This flexibility in searching for the FRs with the best fit results in fewer constraints being placed on the search so that more FRs can be considered. These benefits help provide for better fits requiring fewer amino acid substitutions, fewer changes to vernier residues and better homology or identity scores. In searching for an entire framework that has the best fit, the result is a compromise best fit over that entire framework. In some examples, the entire frameworks for both chains are taken from a single antibody, or in a less constrained search, the frameworks for each chain can come from different antibodies.

More particularly, Example 6, shown below, provides an *in silico* comparison between a past attempt to humanize the anti-TAC antibody and the present invention methods (sometimes referred to herein as "FR Best Fit Humanization"). As shown in this Example, use of the present invention provides a superior humanization result. That is, virtual humanization of the anti-TAC antibody in accord with the present invention produces a better antibody.

Further advantages of using the invention are apparent from the *in silico* results shown in Example 7 (Mc3 antibody); Example 8 (anti-TF antibody); and Example 9 (anti-LTA antibody). In these Examples including Example 6, virtual humanization of described antibodies produces a superior humanized antibody when contrasted with prior humanization approaches. Thus, the invention is one of general application that can be used to make and use a wide range of humanized immune system molecules.

As discussed, the invention can be used to humanize a wide spectrum of immune system molecules such as antibodies and fragments thereof comprising a variable domain. Particular invention methods can be used to produce a humanized antibody variable (V) domain. In one embodiment, the method includes at least one and preferably all of the following steps:
a) comparing the amino acid sequence of a framework region (FR) subset of a non-human antibody variable (V) domain to a collection of human antibody frameworks or variable domain amino acid sequences or sequences of fragments thereof,
b) selecting a human FR subset from the collection having the greatest amino acid sequence identity to the non-human FR subset,
c) mutagenizing DNA of the non-human FR subset to encode a humanized FR subset having an amino acid sequence substantially identical to the selected human FR subset from step b),
d) repeating steps a) thru c) for each of the FR subsets in the non-human V domain to produce a plurality of DNA sequences in which each DNA sequence encodes a humanized FR subset (huFR); and
e) substituting into a first vector encoding at least the V domain of the non-human antibody, each of the huFR DNA sequences from step d) for the corresponding non-human FR subsets encoded by the vector; wherein the substitution operatively links each of the huFRs to its corresponding complimentarity determining region (CDR); and expressing the first vector in host cells and under conditions conducive to making the humanized antibody V domain or full antibody.

The V domain to be humanized will include at least one murine complimentarity determining region (CDR). As will be appreciated, immunoglobulin light and heavy chain share certain structural similarities e.g., each includes a framework of four framework region subsets (FR1-4) whose sequences are relatively conserved. Each of FR1-4 (FR1, FR2, FR3, FR4) are covalently connected by three CDRs *i.e*., CDR1, CDR2, CDR3. There is general recognition that the four FRs largely adopt a beta-sheet configuration and the interconnected CDRs form connecting loops, and in some instances, forming part of the beta-sheet structure. Most CDRs are held close to adjoining FRs, and with a corresponding CDR from the opposite light or heavy chain, help form the antigen binding site. A wide range of CDRs and FRs have been disclosed. See *e.g*., Kabat et al. in Sequences of Proteins of Immunological Interest Fifth Edition, U.S. Dept. of Health and Human Services, U.S. Government Printing Office (1991) NIH Publication No. 91-3242.

See also EP-A-0239400 and U.S. Pat. No. 5,985,279 (describing methods of making altered antibodies in which CDRs are derived from different species than the FR).

By the phrase "antigen binding fragment" is meant at least a part of an antibody that specifically binds antigen. An example of such a fragment includes an antibody V domain and its V domain binding partner. Further suitable fragments further include parts of the V domain having a combined molecular mass for the V domain and it V domain binding partner of between from about 15 kilodaltons to about 40 kilodaltons, preferably between from about 20 kilodaltons to about 30 kilodaltons, more preferably about 25 kilodaltons as determined by a variety of standard methods including SDS polyacrylamide gel electrophoresis or size exclusion chromatography using appropriately sized marker fragments, mass spectroscopy or amino acid sequence analysis.

Additionally suitable antigen binding fragments include at least part of an antigen binding V domains alone or in combination with a cognate constant (C) domain or fragment thereof ("cognate" is used to denote relationship between two components of the same immunoglobulin heavy (H) or light (L) chain). Typical C domain fragments have a molecular mass of between from about 5 kilodaltons to about 50 kilodaltons, more preferably between from about 10 kilodaltons to about 40 kilodaltons, as determined by a variety of standard methods including SDS polyacrylamide gel electrophoresis or size exclusion chromatography using appropriately sized marker fragments, mass spectroscopy or amino acid sequence analysis. Additionally suitable antigen binding fragments are disclosed below.

By the term, "specific binding" or a similar term is meant a molecule disclosed herein which binds another molecule, thereby forming a specific binding pair. However, the molecule does not recognize or bind to other molecules as determined by, *e.g*., Western blotting ELISA, RIA, mobility shift assay, enzyme-immunoassay, competitive assays, saturation assays or other protein binding assays know in the art. See generally, Sambrook et al. in Molecular Cloning: A Laboratory Manual (2d ed. 1989); and Ausubel et al., Current Protocols in Molecular Biology, John Wiley & Sons, New York, 1989. See Harlow and Lane in, Antibodies: A Laboratory Manual (1988) Cold Spring Harbor, New York for examples of methods for detecting specific binding between molecules.

By the phrase "humanized" is meant an immunoglobulin that includes at least one human FR subset, preferably at least two or three of same, more preferably four human FR subsets, and one or more CDRs from a non-human source, usually rodent such as a rat or mouse immunoglobulin. Typically preferred humanized immunoglobulins of the invention will include two or more preferably three CDRs. Constant domains need not be present but are often useful in assisting function of humanized antibodies intended for *in vivo* use. Preferred constant domains, if present, are substantially identical to human immunoglobulin constant domains *i.e.*, at least about 90% identical with regard to the amino acid sequence, preferably at least about 95% identical or greater. Accordingly, nearly all parts of the humanized immunoglobulin, with the possible exception of the CDRs, are preferably substantially identical to corresponding parts of naturally occurring human immunoglobulin sequences.

Methods for determining amino acid sequence identity are standard in the field and include visual inspection as well as computer-assisted approaches using BLAST and FASTA (available from the National Library of Medicine (USA) website). Preferred matching programs for most embodiments are available from website for the international ImMunoGeneTics (IMGT) database and a more preferred matching program for this embodiment is the program called Match which is available in the Kabat database. See Johnson G, Wu T. "Kabat database and its application: Future directions." Nucleic Acids Res. (2001) 29:205-206.

By the phrase "humanized antibody" is meant an antibody that includes a humanized light chain and a humanized heavy chain immunoglobulin. See S.L. Morrison, *supra*; Oi *et al.*, *supra;* Teng *et al.*, *supra*; Kozbor *et al.*, *supra*; Olsson *et al.*, *supra*; and other references cited previously. Accordingly, a "humanized antibody fragment" means a part of that antibody, preferably a part that binds antigen specifically.

As discussed, the invention includes one or more method steps intended to compare and optimize the amino acid sequence of each individual non-human FR to a collection of human amino acid sequences, preferably a collection of sequences which includes human antibody framework amino acid sequences. In practice, the FR in the human framework with the highest sequence identity score has been an FR corresponding to the non-human antibody FR, but this is not necessarily required by the search parameters. By "corresponding" is meant relationship between two FRs from the same or similar position on the antibody V domain. For instance, a rodent FR1 from a given antibody light chain corresponds to a human FR1 from that light chain. Correspondence is often denoted by FR number *i.e.*, a rodent FR1 corresponds with human FR1, a rodent FR2 corresponds with human FR2, *etc.*

In one embodiment of the method, the described V domain is from a non-human antibody light chain. As discussed the precise order of FR subset humanization is typically not important. Thus in one example of the invention, the light FR subset of step a) in the method will be the first variable domain framework region (FR1). However in other embodiments, other FR subsets will be humanized before FR1 *e.g*., FR2, FR3 or FR4.

As discussed, step b) of the method involves selecting a human FR subset from a plurality of human amino acid sequences having the greatest amino acid sequence identity to the non-human FR subset. In embodiments in which the non-human framework to be humanized is FR1, the sequence identity between the FR1 of the non-human antibody light chain and the selected human FR subset is preferably at least about 70%, more preferably at least about 80%, even more preferably at least about 95%.

As also discussed, particular invention methods involve iterative (typically sequential) humanization of each non-human framework region. Thus in embodiments in which FR1 is manipulated first, the method subsequently includes manipulation of FR2, FR3 and FR4. As mentioned, the precise order of humanizing the FRs is not important but in the interest of convenience, it may be helpful to humanize the light and heavy frameworks in numerical order *i.e.*, FR1, FR2, FR3 and FR4.

Thus in one invention embodiment, step d) of the method will further include comparing the second non-human framework region (FR2) of the non-human light chain (or heavy chain) V domain to the collection and selecting a human FR subset having at least about 70% sequence identity, preferably at least about 80%, more preferably at least about 95% sequence identity to its human FR subset (in practice, this is typically a human FR2). The step d) of the method will further include comparing a third framework region (FR3) of the non-human light chain (or heavy chain) V domain to the collection and selecting a human framework region having at least about 70% sequence identity, preferably at least about 80%, more preferably at least about 95% sequence identity to a human FR subset (in practice, this is typically a human FR3). Typically, the step d) will further include comparing a fourth framework region (FR4) of the non-human light chain (or heavy chain) V domain to the collection and selecting a human framework region having at least about 70% sequence identity, preferably at least about 80%, more preferably at least about 95% sequence identity to its corresponding human framework subset (in practice, this is typically a human FR4).

As will be apparent from the present disclosure, the invention can be employed to humanize a wide variety of immune system molecules including light chain V domains, heavy chain V domains of any heterodimeric immunoglobulin-like molecules, including but not limited to T-cell receptors, major histocompatibility complexes, and antibodies. In one embodiment, the humanized light chain (or heavy chain) includes the following components covalently linked in sequence: huFRl- CDR1-huFR2-CDR2-huFR3-CDR3-huFR4..

As mentioned, it is an object of the invention to produce humanized antibodies in which potential vernier zone hindrance problems on at least one of the V domains is minimized and preferably eliminated. This invention feature helps to maximize specific binding between the immune system molecule and its cognate antigen. Thus in one embodiment of the method, vernier zone amino acid residues in each FR subset on at least one of the light and heavy chains of the variable domain is identical when the non-human FR subset is compared with the corresponding human FR subset of the antibody V domain. For example, the vernier zone amino acid residues of non-human FR1 on the light or heavy chain should be identical to corresponding residues in the human FR1 subset.

The first vector used in the method typically includes sequence information needed for suitable expression of the encoded immune system molecule in a desired host. In embodiments in which the immune system molecule is a humanized light chain V domain, it will often be useful if the first vector further includes a human light chain constant domain or fragment thereof. Typically, but not exclusively, the human light chain constant domain or fragment will be covalently linked to the humanized light chain.

In preferred embodiments, the human light chain constant domain is Cκ, C_{λ} or a fragment thereof. Often, the humanized light chain fragment will have an amino acid length of between from about 80 to about 250 amino acids, preferably between from about 95 to about 235 amino acids, more preferably between from about 104 to about 225 amino acids. The size of the humanized light chain fragment can be determined by a variety of standard methods including SDS polyacrylamide gel electrophoresis or size exclusion chromatography using appropriately sized marker fragments, mass spectroscopy or amino acid sequence analysis.

In embodiments in which the humanized immune system molecule is a heavy chain V domain, the humanized heavy chain fragment will have an amino acid length of between about 80 to about 650 amino acids, preferably between from about 95 to about 540 amino acids, more preferably about 102 to about 527 amino acids as determined e.g., by standard SDS polyacrylamide gel electrophoresis or size exclusion chromatography using appropriately sized marker fragments, mass spectroscopy or amino acid sequence analysis.

As discussed, it is an object of the invention to provide novel humanization methods in which each framework region from a non-human immune system molecule is independently compared to a collection of human framework subsets. So for example, to humanize a chimeric antibody, the sequence of the first framework region (FR1) in the heavy chain (HC) variable domain is compared to all known sequences for FR subsets in the heavy chain variable domains of human antibodies. Candidates with the highest degree of identity or homology (fewest number of mismatches in the amino acid sequence) are identified. The process is then preferably repeated for each of FR2, FR3 and FR4 for the HC. A similar process is performed for the FR in the variable domain of the light chain. Best fit human FR subsets may be taken from the same or different antibodies as needed to suit an intended use of the invention. This is a significant departure from other humanization methods in which the best fit selected is a single framework in its entirety from a single human antibody sequence.

In a particular embodiment of the method, the first vector further includes a human heavy chain constant domain or fragment thereof covalently linked to the humanized heavy chain. Preferably, the human constant domain is one of an IgG1, IgG2, IgG3 or IgG4 isotype or a fragment thereof.

By the phrase "chimeric antibody" or related phrase including plural forms is meant antibodies whose light and heavy chain genes have been constructed, typically by genetic engineering, from immunoglobulin gene segments belonging to different species. For example, the variable (V) domains of the genes from a mouse monoclonal antibody may be joined to human constant (C) domains, such as γ₁, γ₂, γ₃, or γ₄. A typical therapeutic chimeric antibody is thus a hybrid protein consisting of the V or antigen-binding domain from a mouse antibody and the C or effector domain from a human antibody, although other mammalian species may be used. Specific chimeric antibodies are the anti-tissue factor antibody, cH36 and the anti-lipotechoic acid antibody, c96-110 (sometimes referred to as A110) disclosed below.

As discussed, the invention method compares the amino acid sequence of a non-human antibody variable (V) domain framework region (FR) to a plurality of human amino acid sequences, preferably a collection of human antibody framework amino acid sequences or sequences of fragments thereof. An example of such a collection is one from a database that includes a list of fully sequenced human antibodies. Unlike prior humanization methods, the collection may further include one or more amino acid sequences of partially sequenced human antibodies. Alternatively, the collection may consist essentially of only the partially sequenced human antibodies. Examples of such collections include, but are not limited to, the following databases: GenBank, IMGT, Swiss-Prot, and Kabat, *supra.*

In a more particular example of the invention, there is provided a method for making a humanized antibody or a humanized antibody fragment comprising heavy and light chain variable (V) domains. In one embodiment, the method includes the following steps:
a) comparing the amino acid sequence of a non-human antibody light chain variable (V) domain framework region (1-FR) to a collection of human antibody light chain framework amino acid sequences,
b) selecting a human FR subset from the collection having the greatest amino acid sequence identity to the 1-FR,
c) mutagenizing DNA of the 1-FR to encode a light chain humanized FR subset (L-huFR) having an amino acid sequence at least 75% identical to the selected human FR subset from step b),
d) repeating steps a) thru c) for each of the FR subsets in the light chain V region to produce a plurality of DNA sequences in which each DNA sequence encodes an L-huFR,
e) substituting into a first vector encoding at least the light chain V domain of the non-human antibody, each of the L-huFR DNA sequences from step d) for the corresponding 1-FRs encoded by the vector; wherein the substitution operatively links each of the L-huFRs to a corresponding complimentarity determining region (CDR),
f) comparing the amino acid sequence of a non-human antibody heavy chain variable (V) domain framework region (h-FR) to a collection of human antibody heavy chain amino acid sequences,
g) selecting a human FR subset from the collection having the greatest amino acid sequence identity to the h-FR,
h) mutagenizing DNA of the h-FR to encode a humanized heavy chain FR subset (H-huFR) having an amino acid sequence at least 75% identical to the selected human FR from step g),
i) repeating steps f) thru h) for each of the h-FRs in the non-human heavy chain V domain to produce a plurality of DNA sequences in which each DNA sequence encodes a H-huFR,
j) substituting into said first vector or a second vector encoding at least the heavy chain V domain of the non-human antibody, each of the H-huFR DNA sequences from step i) for the corresponding h-FRs of the antibody; wherein the substitution operatively links each of the H-huFRs to a corresponding heavy chain CDR; and
k) expressing the vector or vectors in the same host cell and under conditions conducive to producing humanized light and heavy chains and making the humanized antibody or the humanized antibody fragment comprising heavy and light chain V domains.

In one embodiment of the foregoing method, the first and second vectors are co-expressed in the same host cell. In another embodiment, DNA molecules encoding the humanized light and heavy chains or fragments thereof are contained on a single vector and co-expressed in the same host.

Suitable first vectors for use with the foregoing antibody humanization method will include sequence information needed for suitable expression of the encoded immune system molecule. For example, acceptable first vectors will include a human light chain constant domain or fragment thereof covalently linked to the humanized light chain V domain. Preferably, the constant domain is Cκ, C_{λ} or a fragment thereof.

Preferred second vectors in accord with the method typically further include a human heavy chain constant domain or fragment thereof covalently linked to the humanized heavy chain V domain. For instance, the human constant domain can be one of an IgGI, IgG2, IgG3 or IgG4 isotype including fragments thereof, or other isotypes (IgA, IgD, IgE or IgM).

The present invention is also compatible with additional steps intended to purify the humanized immune system molecules from cell components that naturally accompany it. Thus in one embodiment, the forgoing methods will further include one or more steps that include purifying the humanized antibody or humanized antibody fragment comprising heavy and light chain V domains from the host cells to produce a substantially pure preparation of the antibody or fragment. Preferably, the substantially purified humanized antibody specifically binds antigen with an affinity not less than about 10-fold lower than the parental non-human antibody.

As will be apparent, the present invention can be used to make a broad spectrum of humanized immune system molecules. For example, a humanized antibody includes: 1) light and heavy chain framework regions (FRs) that are each individually at least about 90% identical in amino acid sequence to a human FR subset, preferably at least 95% identical to same, more preferably at least about 98% up to 100% identical to the human FR subset, 2) at least one CDR from a rodent such as a mouse, preferably all the CDRs from the mouse, 3) and an immunoglobulin constant domain that is at least about 90% identical, preferably at least 95% up to about 100% identical to a corresponding human immunoglobulin constant domain. It will be appreciated that an antibody has been "humanized" by the process of "humanization" because the resultant humanized antibody is expected to bind to the same antigen as the antibody that provides the CDRs.

It will be further appreciated that particular humanized immune system molecules disclosed herein, usually humanized antibodies, may have one or more additional conservative amino acid substitutions which can be contiguous or non-contiguous as needed. For example, such substitutions will typically have substantially little or no effect on antigen binding or other immunoglobulin functions. By the phrase "conservative substitution" including plural forms is meant combinations of: gly↔ala; val↔ile↔leu; asp↔glu; asn↔gln; ser↔thr, lys↔arg; and phe↔tyr.

Antibodies are preferably substantially pure when used in the disclosed methods and assays. References to an antibody being "substantially pure" mean an antibody or protein that has been separated from components which naturally accompany it. For example, by using standard immunoaffinity or protein A affinity purification techniques, an antibody of the invention can be purified from hybridoma or cell culture medium by using native TF as an antigen or protein A resin. Similarly, native TF can be obtained in substantially pure form by using an antibody of the invention with standard immunoaffinity purification techniques. Particularly, an antibody or protein is substantially pure when at least 50% of the total protein (weight % of total protein in a given sample) is an antibody or protein of the invention. Preferably the antibody or protein is at least 60 weight % of the total protein, more preferably at least 75 weight %, even more preferably at least 90 weight %, and most preferably at least 98 weight % of the total material. Purity can be readily assayed by known methods such as SDS polyacrylamide gel electrophoresis (PAGE), column chromatography (*e.g*., affinity chromatography, size exclusion chromatography), mass spectroscopy or HPLC analysis.

Such substantially purified and humanized antibodies can be used to specifically bind a wide range of antigens. For instance, in one embodiment, the antibodies produced by the present methods can be used to specifically recognize and bind lipotechoic acid or a related fatty acid. Other humanized antibodies and fragments are produced that specifically recognize and bind human tissue factor.

Humanized immune system molecules provide a broad spectrum of important uses. For instance, the humanized antibodies can be used to prevent or treat diseases in humans or animals. Other contemplated uses include use as a diagnostic product.

Antibodies for humanization in accordance with the method of the invention can be readily obtained from a variety of sources. Alternatively, they can be made *de novo.* In one approach, such molecules can be prepared by immunizing a mammal with a purified sample of native human TF, or an immunogenic peptide as discussed above, alone or complexed with a carrier or as a mixture with an adjuvant. Suitable mammals include typical laboratory animals such as sheep, goats, rabbits, guinea pigs, rats and mice. Rats and mice, especially mice, are preferred for obtaining monoclonal antibodies. The antigen can be administered to the mammal by any of a number of suitable routes such as subcutaneous, intraperitoneal, intravenous, intramuscular or intracutaneous injection. The optimal immunizing interval, immunizing dose, *etc.* can vary within relatively wide ranges and can be determined empirically based on this disclosure. Typical procedures involve injection of the antigen several times over a number of months. Antibodies are collected from serum of the immunized animal by standard techniques and screened to find antibodies specific for desired antigen. Monoclonal antibodies can be produced in cells which produce antibodies and those cells used to generate monoclonal antibodies by using standard fusion techniques for forming hybridoma cells. See G. Kohler, et al., Nature 256: 456 (1975). Typically this involves fusing an antibody-producing cell with an immortal cell line such as a myeloma cell to produce the hybrid cell. Alternatively, monoclonal antibodies can be produced from cells by the method of Huse, et al., Science, 256: 1275 (1989). Such an antibody can be sequenced by conventional methodologies if desired.

For some applications, it may be desirable to use, as the antibody to be humanized, a chimeric antibody, e.g. antibody molecules that combine a non-human animal variable domain and a human constant domain. A variety of types of such chimeric antibodies can be prepared, including e.g. by producing human variable domain chimeras, in which parts of the variable domains, especially conserved regions of the antigen-binding domain, are of human origin and only the hypervariable regions are of non-human origin. See also discussions of humanized chimeric antibodies and methods of producing same in S.L. Morrison, Science, 229: 1202-1207 (1985); Oi et al., BioTechniques 4: 214 (1986); Teng et al., Proc. Natl. Acad. Sci. U.S.A., 80:7308-7312 (1983); Kozobor et al. Immunology Today 4: 72-79 (1983); Olsson et al. Meth. Enzymol. 92: 3-16 (1983).

Additionally, transgenic mice can be employed to make particular human monoclonal antibodies. For example, transgenic mice carrying human antibody repertoires have been created which can be immunized with an antigen of interest. Splenocytes from such immunized transgenic mice can then be used to create hybridomas that secrete human monoclonal antibodies that specifically react with the antigen. See N. Lonberg et al., Nature, 368:856-859 (1994); L.L. Green et al., Nature Genet., 7:13-21 (1994); S.L. Morrison, et al., Proc. Natl. Acad. Sci. U.S.A., 81:6851-6855 (1984).

Nucleic acids which code for the antibodies also can be prepared by polymerase chain reaction (see primers disclosed in Example 1 which follows). See generally, Sambrook et al., Molecular Cloning (2nd ed. 1989). Such nucleic acids also can be synthesized by known methods, *e.g.* the phosphate triester method (see Oligonucleotide Synthesis, IRL Press (M. J. Gait, ed., 1984)), or by using a commercially available automated oligonucleotide synthesizer. Such a prepared nucleic acid can be employed to express an antibody by known techniques.
For example, a nucleic acid coding for an antibody can be incorporated into a suitable vector by known methods such as by use of restriction enzymes to make cuts in the vector for insertion of the construct followed by ligation. The vector containing the inserted nucleic acid sequence, suitably operably linked to a promoter sequence, is then introduced into host cells for expression. See, generally, Sambrook *et al., supra.* Selection of suitable vectors can be made empirically based on factors relating to the cloning protocol. For example, the vector should be compatible with, and have the proper replicon for the host cell that is employed. Further, the vector must be able to accommodate the inserted nucleic acid sequence. Suitable host cells will include a wide variety of prokaryotic or eukaryotic cells, such as *E. coli, Bacillus subtilis, Streptomyces lividans or* other bacterial hosts, *Saccharomyces cerevisiae* or other yeast, *Aspergillus niger* or other fungi, or other microbial hosts, CHO, BK or NSO mammalian cells, avian or plant cells and the like.

According to a particular invention embodiment, the method described herein will include the step of introducing one or more desired vector types into plant cells under conditions suited to expressing the vector(s) in those cells. A particular plant cell of interest is *Arabidopsis.* See U.S Pat No. 6,417,429 and references cited therein.

For instance, in the foregoing method for making a V domain, step e) will include introducing the first vector into plant cells, preferably *Arabidopsis,* and expressing the first vector therein to produce the antibody V domain. The method is readily adapted to express the full (entire) antibody.

With respect to the previously described method for making a humanized antibody, step k) will include introducing the first and second vectors into plant cells, preferably *Arabidopsis*, and expressing the vectors therein to produce the desired molecules. In some invention embodiments, what is referred to below as a "mega" vector can be used instead of first and second vectors.

The molecular weight of the antibodies will vary depending on several factors such as the intended use and whether the antibody includes a conjugated or recombinantly fused toxin, pharmaceutical, radioisotope or detectable label or the like. Also the molecular weight will vary depending on nature and extent of post-translational modifications if any (such as glycosylation) to the antibody. The modifications are a function of the host used for expression with *E*. *coli* producing non-glycosylated antibodies and eucaryotic hosts, such as mammalian cells, producing glycosylated antibodies. In general, an antibody will have a molecular weight of between approximately 20 to 150kDa. Such molecular weights can be readily determined by molecular sizing methods such as SDS-PAGE followed by protein staining or Western blot analysis.

"Antibody" or other similar term refers to whole immunoglobulin as well as immunologically active fragments which bind a desired antigen. The immunoglobulins and immunologically active (antigen-binding) fragments thereof include an epitope-binding site (*i.e.*, a site or epitope capable of being specifically bound by an antibody recognizing antigen). Exemplary antibody fragments include, for example, Fab, F(v), Fab', F(ab')₂ fragments, "half molecules" derived by reducing the disulfide bonds of immunoglobulins, single chain immunoglobulins, or other suitable antigen binding fragments (see *e.g.,* Bird et al., Science, 242: 423-426 (1988); Huston et al., PNAS, (USA), 85:5879 (1988); Webber et al., Mol. Immunol., 32:249 (1995)). The antibody or immunologically active fragment thereof may be of animal (*e.g.*, a rodent such as a mouse or a rat), or chimeric form (see Morrison et al., PNAS, 81:6851 (1984); Jones et al., Nature, 321: 522 (1986)). Single chain and humanized antibodies of the invention may be useful for some applications of the invention.

Thus according to another embodiment, the forgoing methods further include additional steps intended to make a humanized single-chain antibody (sc-Fv) from the humanized V regions. Additionally, fragments of humanized antibodies can be made by conventional methods, particularly F(v), F(ab')₂, Fab' or Fab; as well as antigen-binding fragments thereof.

Similarly, a "nucleic acid" refers to a nucleotide sequence which can be expressed to provide an antibody as such term is specified to mean immediately above.

In some instances, it may be desirable to modify the antibody to impart a desirable biological, chemical or physical property thereto. Thus the present invention method is compatible with additional conventional steps intended to conjugate (*i.e.* covalently link) one or more humanized immune system molecules to a pharmaceutical agent. Such linkage can be accomplished by several methods including use of a linking molecule such as a heterobifunctional protein cross-linking agent, *e.g.*
SPDP, carbodimide, or the like, or by recombinant methods. Particular conjugation strategies compatible with use of the humanized antibodies have been disclosed in PCT Application WO 99/21572 to Rhode, P. *et al.* See also Ausubel et al., Current Protocols in Molecular Biology, John Wiley & Sons, New York, (1989); Harlow and Lane in Antibodies: A Laboratory Manual, CSH Publications, NY (1988).

Particular humanized antibodies of the present invention can be polyclonal or monoclonal, as needed, and may have, without limitation, an IgG1, IgG2, IgG3 or IgG4 isotype or IgA, IgD, IgE, IgM.

The humanized antibodies disclosed herein can be produced by one or a combination of strategies including those described below in Examples 1-9.

In an approach described in Examples 1 and 4, four general steps were employed to humanize an antibody. First, the amino acid sequences of the mouse antibody light and heavy chains were obtained from the cH36 mouse-human chimeric antibody. Second, the cH36 antibody was humanized by determining which human antibody framework region gave the "best fit" *i.e.,* most closely resembled the mouse framework region amino acid sequence. Third, relevant light and heavy chain FR sequences were humanized, and fourth, transfection and expression of isolated nucleic acid(s) that encode the humanized light or heavy chain (or humanized light and heavy chain *e.g.*, see the mega vectors described below).

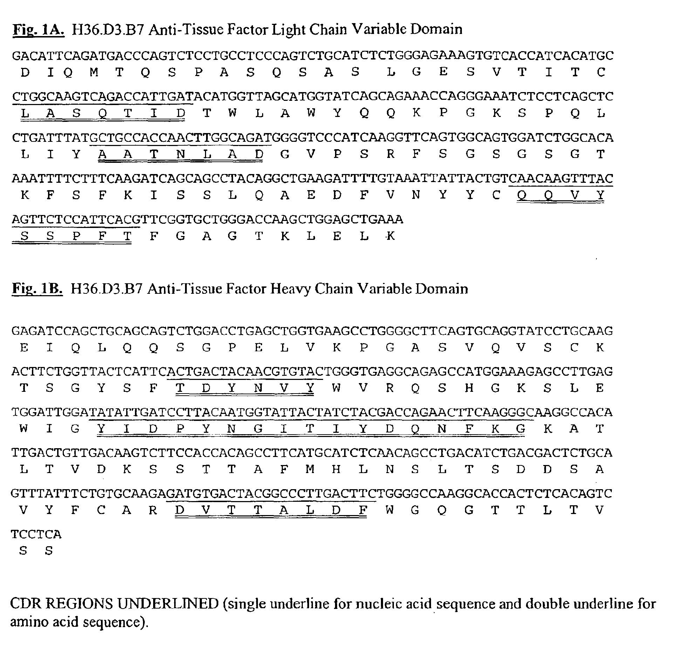

More particularly, the "FR best fit" approach was applied to humanizing the chimeric anti-tissue factor antibody cH36. In this approach, the murine light and heavy chain variable domain sequences shown in Figs. 1A and 1B (SEQ ID NOS 2 and 4) were used to search ("compare") all available protein databases for those human antibody variable domain sequences that are most homologous to the murine variable domain. See *e.g.,* Kabat *et al., supra.* A number of readily available computer programs can be used to perform this step such as BLAST, FASTA and related programs. Framework regions 1, 2, 3, and 4 of the light and heavy chain were of special interest since these sites are almost universally understood to hold the CDRs in proper orientation for antigen binding. Output stemming from the search was typically a list of sequences most homologous to the query mouse sequences, the percent homology to each sequence, and an alignment of each human sequence to the corresponding murine sequence. The analysis was generally performed on the light and heavy chains independently.

According to the "FR best fit" approach, the number of mismatched amino acids was minimized between the query mouse FR subset and the human FR subset. In most cases, suitable human framework region subsets were selected based on the following identity criteria. On the light chain, the amino acid sequence of the murine FR1 was at least about 80% identical to the human FR subset; the murine FR2 was at least about 90% identical to the human FR subset, the murine FR3 was at least about 90% identical to the human FR subset; and the murine FR4 was at least about 75% identical to the human FR subset. And on the heavy chain, the amino acid sequence of the murine FR1 was chosen to be at least about 80% identical to the human FR subset; the murine FR2 was at least about 85% identical to the human FR subset; the murine FR3 was chosen to be at least about 70% identical to the human FR subset; and the murine FR4 was at least about 90% identical to the human FR subset. Typically, conservative amino acid substitutions were favored when evaluating similar candidate human framework region sequences. It was found that when such factors were considered the resulting human framework regions served as a good reference point for humanization of the chimeric cH36 antibody.

Once a decision on a desired human framework region was made, recombinant polymerase chain reaction (PCR) techniques were used to make desired amino acid substitutions in both the light and heavy chains. Typically, oligonucleotides were made and used to mutagenize mouse variable domain frameworks to contain desired residues. Oligonucleotides having a variety of lengths were employed. See WO 92/07075 for general disclosure relating to recombinant PCR and related methods.

In general, regular PCR was used for cloning, to introduce cloning or diagnostic restriction endonuclease sites, and to change amino acid residues located at the ends of the variable domains. PCR-based mutagenesis was used to change multiple amino acid residues at a time, especially when these residues were in the middle of the variable domains. Site-directed mutagenesis was used to introduce one or two amino acid substitutions at a time. After each step, the partially humanized clones were sequenced and some of these variable domains were later cloned into expression vectors. More specific methods for performing these manipulations are described in the Examples section.

After performing the foregoing "FR best fit" approach to humanizing each non-human FR, mutagenized nucleic acids encoding humanized framework region (huFR) and/or CDR were linked to an appropriate DNA encoding a light or heavy chain constant domains. Such constructs were then cloned into an expression vector, and transfected into host cells, preferably mammalian cells. These steps were achieved by using recombinant and cell culture techniques known in the field.

In one approach, a humanized antibody can be prepared by the following general method:
(a) preparing a first expression vector including a replicon appropriate for the expression host and a suitable promoter operably linked to a DNA sequence which encodes at least a variable domain of an Ig heavy or light chain, the variable domain comprising individually humanized framework regions (FR1-4) and murine CDRs 1-3 from a desired antibody,
(b) preparing a second replicable expression vector including a suitable promoter operably linked to a DNA sequence which encodes at least the variable domain of a complementary Ig light or heavy chain respectively, that variable domain comprising corresponding and individually humanized framework regions (FR1-4) and murine CDRs 1-3 from the antibody;
(c) transfecting a cell line with the first or both prepared vectors; and
(d) culturing said transfected cell line to produce said humanized antibody.

Preferably the DNA sequence in steps (a) and (b) encode suitable constant domains from the human antibody chain. Suitable isotypes include, without limitation, IgG1 and IgG4, for example.

Alternatively, a suitable humanized antibody can be prepared by making a single replicable "mega" vector that includes an appropriate promoter operably linked to a DNA sequence which encodes at least a variable domain of an Ig heavy or light chain, the variable domain comprising each of the individually humanized framework regions (FR1-4) and murine CDRs 1-3 from a subject antibody.

In one embodiment, the mega vector will further include a suitable promoter operably linked to a DNA sequence which encodes at least the variable domain of a complementary Ig light or heavy chain respectively, that variable domain comprising corresponding and individually humanized framework regions (FR1-4) and murine CDRs 1-3 from the cH36 antibody or other suitable CDRs. Use of the mega vector will often be appropriate where humanized antibody expression from a single vector is needed.

It will be apparent that the foregoing method for making the humanized cH36 antibody can be readily adapted to make other humanized antibodies and antigen binding fragments according to the invention.

Particular examples of antibodies well suited for the humanization methods of this invention have been disclosed in WO03/037911 as well as the Examples that follow.

By the words "assembling" or "assembled" is meant use of standard recombinant techniques to introduce subject DNA sequences encoding the humanized frameworks or framework regions into the vectors. Such assembly can be performed by one or combination of approaches including, but not limited to, introducing iterative changes to a single framework or framework region sequence, cutting and pasting fragments together (via use of restriction endonucleases and ligase), or by synthetic DNA synthesis techniques. See generally Harlow and Lane *supra* and Ausubel *et al. supra.*

The foregoing methods for making humanized antibodies can be practiced with nearly any acceptable mutagenesis technique. In particular, relevant method steps can employ site-directed mutagenesis and/or standard PCR methods to replace desired rodent amino acids in the framework with appropriate human amino acids. It can also be accomplished by DNA synthesis of modified fragments or entire coding regions, or by the *in vitro* recombination using standard recombinant DNA or genetic engineering techniques or any combination of these. Typically, the sequence of the modified (humanized) framework or framework region corresponds to the selected human framework or framework region sequence from the database.

Suitable nucleic acids encode at least one of the heavy or light chains of the humanized antibodies or fragments thereof disclosed herein. Typically, the nucleic acid is a recombinant DNA vector that includes the isolated nucleic acid. The DNA vector will typically further include an expression control polynucleotide sequence operably linked to the humanized immunoglobulin coding sequences, including naturally associated or heterologous promoter regions. Preferably, the expression control sequences will be eukaryotic promoter systems in vectors capable of transforming or transfecting eukaryotic host cells, but control sequences for prokaryotic hosts may also be used. Once the vector has been incorporated into the appropriate host, the host is maintained under conditions suitable for high-level expression of the nucleotide sequences, and, as desired, the collection and purification of the light chains, heavy chains, light/heavy chain dimers or intact antibodies, binding fragments or other immunoglobulin forms may follow.

The nucleic acid sequences capable of ultimately expressing the desired humanized antibodies can be formed from a variety of different polynucleotides (genomic or cDNA, RNA, synthetic oligonucleotides, *etc.*) and components (*e g*., V, J, D, and C regions), as well as by a variety of different techniques.
Joining appropriate genomic and synthetic sequences is presently the most common method of production, but cDNA sequences may also be utilized. See *e.g.*, S. L. Morrison, *supra*; Oi *et al.*, *supra*; Teng *et al.*, *supra*; Kozbor *et al.*, *supra*; Olsson *et al.*, *supra*; European Patent Publication No. 0239400 and Riechmann, L. et al., Nature, 332: 323-327 (1988); and references cited therein.

Use of the invention is compatible with a wide range of suitable hosts *e.g.*, mammalian, plant or microbial host cells. In one embodiment, suitable DNA expression vectors include one or more selection markers, *e.g.*, tetracycline, ampicillin, geneticin, hygromycin, puromycin, or neomycin (or the like), to permit detection of those cells transformed with the desired DNA sequences (see, *e.g.,* U.S. Pat. No. 4,704,362). *E. coli* is one prokaryotic host useful particularly for cloning the polynucleotides of the present invention. Other microbial hosts suitable for use include but are not limited to bacilli, such as *Bacillus subtilus*, and other *Enterobacteriacea*, such as *Salmonella*, *Serratia*, various *Pseudomonas* species and other microbes such as actinomycetes (*e.g.*, *Streptomyces* species), yeast (*e.g.*, *Saccharomyces* species) or fungi (*e.g.*, *Aspergillus* species). In these prokaryotic hosts, one can also make expression vectors, which will typically contain expression control sequences compatible with the host cell (*e.g.*, promoters and an origin of replication). In addition, any number of a variety of well-known promoters will be present, such as the lactose promoter system, a tryptophan (*trp*) promoter system, a beta-lactamase promoter system, or a promoter system from phage lambda. The promoters will typically control expression, optionally with an operator sequence, and have ribosome binding site sequences and the like, for initiating and completing transcription and translation. Other microbes, such as yeast, may also be used for expression. *Saccharomyces* is a preferred host, with suitable vectors having expression control sequences, such as promoters, including 3-phosphoglycerate kinase or other glycolytic enzymes, and an origin of replication, termination sequences and the like as desired.

In addition to forgoing microorganism-based systems, eukaryotic hosts may also be used to express and produce the polypeptides of the present invention (see, Winnacker, "From Genes to Clones", VCH Publishers, N.Y., N.Y. (1987) ). In many embodiments, eukaryotic hosts will be generally preferred, typically mammalian cell lines without limitation, including CHO cell lines, various COS cell lines, NS0 cells, BK cells, HeLa cells, preferably myeloma cell lines, *etc.,* or transformed B-cells of hybridomas. Expression vectors for these cells can include expression control sequences, such as an origin of replication, a promoter, and enhancer (Queen et al., Immunol. Rev. 89: 46-68 (1986)), and necessary processing information sites, such as ribosome binding sites, RNA splice sites, polyadenylation sites, and transcriptional terminator sequences. Preferred expression control sequences are promoters derived from immunoglobulin genes, SV40, Adenovirus, Bovine Papilloma Virus, cytomegalovirus and the like. In other embodiments, eukaryotic hosts will be generally preferred where the eukaryotic host is a plant or plant cells without limitation, including *e.g.*, *Arabidopsis*, *Nicotinia*, *etc.* and plant cell culture may also be used to express and produce the antibodies of the present invention. In other embodiments, eukaryotic hosts will be generally preferred where the eukaryotic host is an insect cell, avian species or a transgenic animal.

Preferred DNA vectors for practicing the invention include the following operatively linked sequences: an antibiotic resistance marker *e.g.*, ampicillin resistance, F1 origin, and heavy chain (HC) or light chain (LC) variable domain. That variable domain can be inserted into an appropriate HC expression vector that includes operatively linked in sequence: the HC variable domain, human IgG1 or IgG4 constant domain, first polyA site, SV40 promoter, antibiotic resistance marker such as neomycin resistance, second polyA site, cytomegelovirus (CMV) promoter/enhancer, and suitable leader sequence.

Additionally preferred DNA vectors include the LC variable domain operatively linked to a rodent kappa intron (*e.g.*, mouse) which intron is operatively linked to a suitable human kappa constant domain; and antibiotic resistance marker such a neomycin resistance.

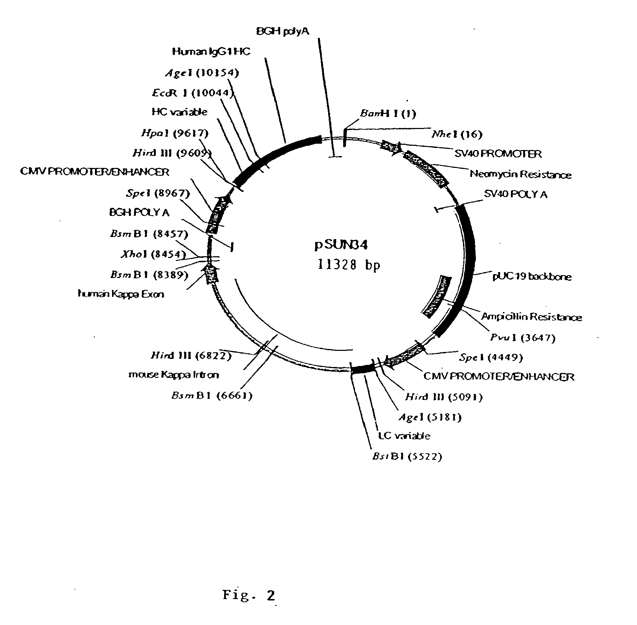

As discussed, it will often be highly useful to express humanized antibodies of the present invention from a single nucleic acid. A preferred DNA vector is sometime referred to herein as a "mega" vector and includes operatively linked in sequence the following components: SV40 promoter, antibiotic resistance marker such as neomycin, first polyA site, first CMV promoter/enhancer, LC variable domain, rodent kappa intron (*e.g*., mouse} human kappa exon, second polyA site, second CMV promoter/enhancer, HC variable domain, and human IgG1 or IgG4 heavy chain constant domain. A specific example of such a mega vector is the humanized anti-TF IgG1 antibody expression vector described below in Examples 1-3. See also Fig. 2

### A. Preparation Of Humanized Anti-Tissue Factor Binding Antibody

Preparation and use of a humanized anti-tissue factor binding antibody has already been described in WO03/037911. See also Examples 1-3 below.

Briefly, preferred antibodies bind human tissue factor to form a binding complex. The tissue factor may be naturally occurring or recombinant human (rhTF). Preferably, factor X or factor IX binding to the complex is inhibited. In a preferred invention embodiment, the humanized antibody has an apparent affinity constant (K_{A}, M⁻¹) for the hTF of less than about 1 nM, preferably less than about 0.5 nM, more preferably between from about 0.01 nM to about 0.4 nM. See Examples 1-3, below for more information about determining affinity constants for the humanized antibodies. By "specific binding" is meant that the humanized antibodies form a detectable binding complex with the TF (or rhTF) and no other antigen as determined by standard immunological techniques such as RIA, Western blot or ELISA.

More preferred humanized anti-TF binding antibodies made in accord with this invention exhibit an apparent affinity constant (K_{A}, M⁻¹) for native human TF of at least about 1 x 10⁸ M⁻¹ as determined by surface plasmon analysis (particularly, BIACore analysis in accordance with the procedures of Example 3 which follows), more preferably at least about 5 x 10⁸ M⁻¹ as determined by surface plasmon analysis, still more preferably an apparent affinity constant (K_{A}, M⁻¹) for native human TF of at least about 3 x 10⁹M⁻¹ as determined by surface plasmon resonance analysis. Such substantial binding affinity of antibodies of the invention contrast sharply from much lower binding affinities of previously reported antibodies.

In this regard, a quite low effective concentration of the humanized tissue factor binding antibody can be employed, *e.g.* a relatively low concentration of antibody can be employed to inhibit TF function as desired (*e.g.* at least about 95, 98 or 99 percent inhibition) in an *in vitro* assay such as described in Example 3 which follows.

The nucleic acid (SEQ ID NOS 1 and 3) and amino acid (SEQ ID NOS 2 and 4) sequences of a particular tissue factor binding antibody that has been humanized by the present methods *i.e.* H36.D2.B7. See Figures 1A and 1B of the drawings. SEQ ID NOS 1 and 2 are the nucleic acid and amino acid respectively of the light chain variable domain, and SEQ ID NOS 3 and 4 are the nucleic acid and amino acid respectively of the heavy chain variable domain, with hypervariable regions (CDRs or Complementarity Determining Regions) underlined in all of those sequences.

Additional tissue factor binding humanized antibodies will have substantial amino acid sequence identity to either one or both of the light chain or heavy sequences shown in Figures 1A and 1B. More particularly, such antibodies include those that have at least about 70 percent homology (amino acid sequence identity) to SEQ ID NOS 2 and/or 4, more preferably about 80 percent or more homology to SEQ ID NOS 2 and/or 4, still more preferably about 85, 90 or 95 percent or more homology to SEQ ID NOS 2 and/or 4.

More particular tissue factor binding humanized antibodies will have high amino acid sequence identity to hypervariable regions (shown with double underlining in Figures 1A and 1B) of SEQ ID NOS 2 and 4). Specific antibodies will have one, two or three hypervariable regions of a light chain variable domain that has high sequence identity (at least 90% or 95% amino acid sequence identity) to or be the same as one, two or three of the corresponding hypervariable regions of the light chain variable domain of H36.D2.B7 (those hypervariable regions shown with underlining in Figure 1A and are the following:
1) LASQTID (SEQ ID NO: 5);
2) AATNLAD (SEQ ID NO: 6); and
3) QQVYSSPFT (SEQ ID NO: 7).

Additionally specific antibodies that have been humanized by the methods described herein and bind tissue factor will have one, two or three hypervariable regions of a heavy chain variable domain that have high sequence identity (at least 90% or 95% amino acid sequence identity) to or be the same as one, two or three of the corresponding hypervariable regions of the heavy chain variable domain of H36.D2.B7 (those hypervariable regions shown with underlining in Figure 1B and are the following:
1) TDYNVY (SEQ ID NO: 8);
2) YIDPYNGITIYDQNFKG (SEQ ID NO: 9); and
3) DVTTALDF (SEQ ID NO: 10).

Certain nucleic acids preferably are of a length sufficient (preferably at least about 100, 200 or 250 base pairs) to bind to the sequence of SEQ ID NO: 1 and/or SEQ ID NO: 3 under the following moderately stringent conditions (referred to herein as "normal stringency" conditions): use of a hybridization buffer comprising 20% formamide in 0.9M saline/0.12M sodium citrate (6xSSC) buffer at a temperature of 37°C and remaining bound when subject to washing once with that 2xSSC buffer at 37°C.

More specifically, certain nucleic acids (preferably at least about 100, 200 or 250 base pairs) will bind to the sequence of SEQ ID NO: 1 and/or SEQ ID NO: 3 under the-following highly stringent conditions (referred to herein as "high stringency" conditions): use of a hybridization buffer comprising 20% formamide in 0.9M saline/0.12M sodium citrate (6xSSC) buffer at a temperature of 42°C and remaining bound when subject to washing twice with that 1xSSC buffer at 42°C.

Nucleic acids preferably comprise at least 20 base pairs, more preferably at least about 50 base pairs, and still more preferably a nucleic acid of the invention comprises at least about 100,200, 250 or 300 base pairs.

Generally preferred nucleic acids will express an antibody of the invention that exhibits the preferred binding affinities and other properties as disclosed herein.

Other nucleic acids also will have substantial sequence identity to either one or both of the light chain or heavy sequences shown in Figures 1A and 1B. More particularly, preferred nucleic acids will comprise a sequence that has at least about 70 percent homology (nucleotide sequence identity) to SEQ ID NOS 1 and/or 3, more preferably about 80 percent or more homology to SEQ ID NOS 1 and/or 3, still more preferably about 85, 90 or 95 percent or more homology to SEQ ID NOS 1 and/or 3.

Additionally specific nucleic acid sequences will have high sequence identity to hypervariable regions (shown with underlining in Figures 1A and 1B) of SEQ ID NOS 1 and 3). Such nucleic acids include those that code for an antibody light chain variable domain and have one, two or three sequences that code for hypervariable regions and have high sequence identity (at least 90% or 95% nucleotide sequence identity) to or be the same as one, two or three of the sequences coding for corresponding hypervariable regions of H36.D2.B7 (those hypervariable regions shown with underlining in Figure 1A and are the following:
1) CTGGCAAGTCAGACCATTGAT (SEQ ID NO: 11);
2) GCTGCCACCAACTTGGCAGAT (SEQ ID NO: 12); and
3) CAACAAGTTTACAGTTCTCCATTCACGT (SEQ ID NO: 13).

More specific nucleic acids also code for an antibody heavy chain variable domain and have one, two or three sequences that code for hypervariable regions and have high sequence identity (at least 90% or 95% sequence identity) to or be the same as one, two or three of the sequences coding for corresponding hypervariable regions of H36.D2.B7 (those hypervariable regions shown with underlining in Figure 1B and are the following:
1) ACTGACTACAACGTGTAC (SEQ ID NO: 14);
2) and
3) GATGTGACTACGGCCCTTGACTTC (SEQ ID NO: 16).

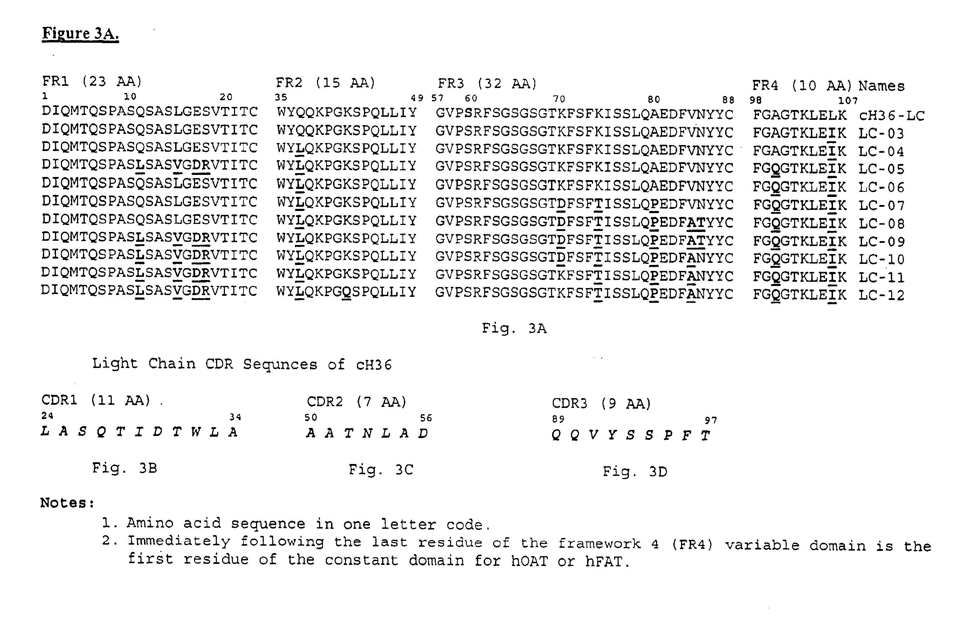

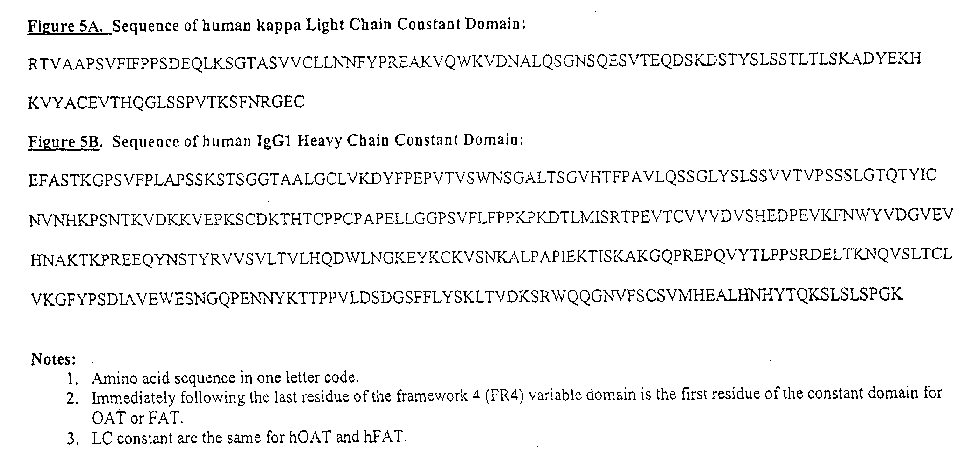

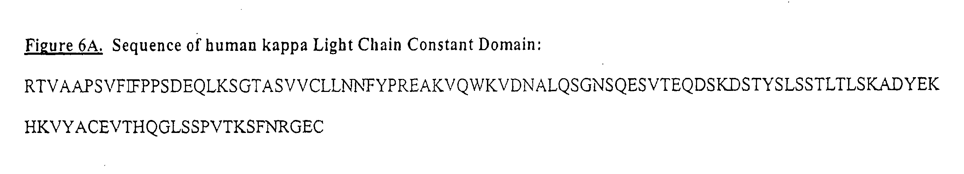

More specific humanized antibodies that bind TF are those in which each of framework regions (FRs) 1, 2, 3 and 4 has at least about 90% amino acid sequence identity, preferably at least about 95% or greater identity to the light chain FR sequences shown in Figure 3A (SEQ ID NOS 72-82), preferably, the sequence shown as "LC-09" in Figure 3A. Additionally specific humanized antibodies include a light chain constant domain having at least about 90% amino acid sequence identity, preferably at least about 95% sequence identity or greater to the sequence shown in Figure 5A (SEQ ID NO: 97) or Figure 6A (SEQ ID NO: 99).

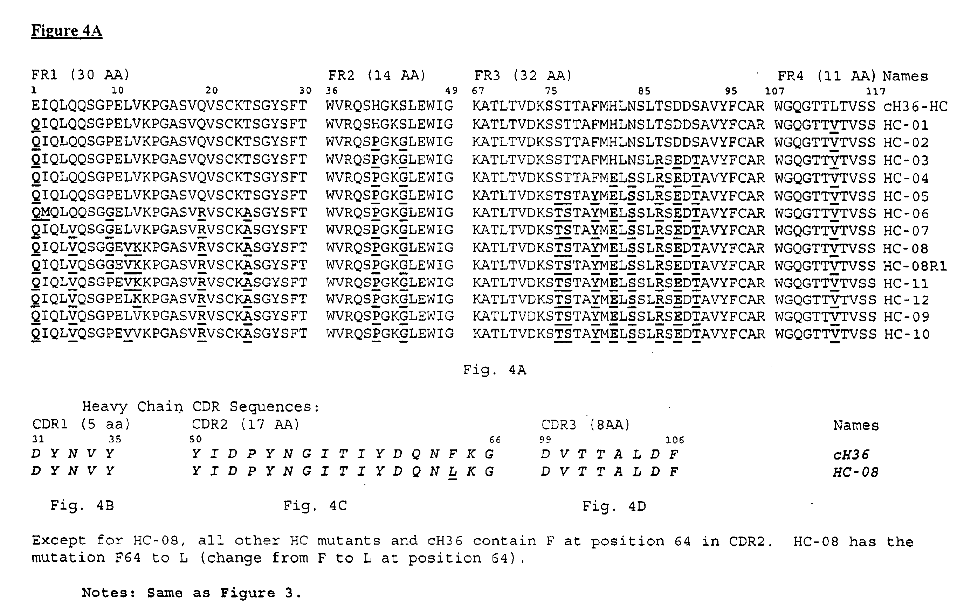

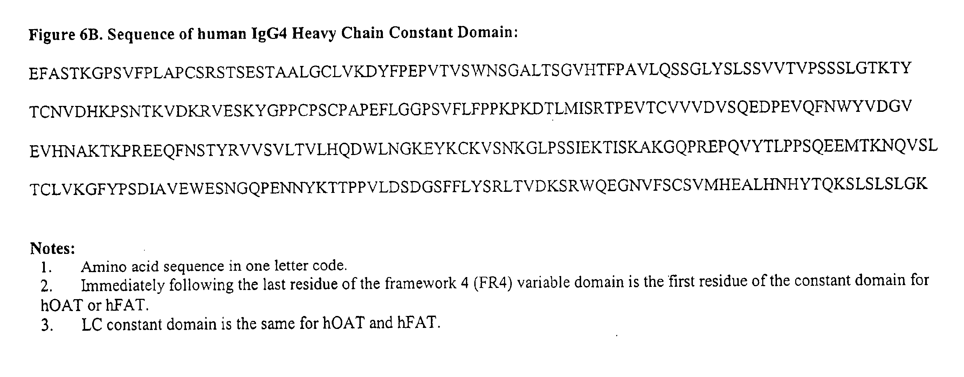

Further specific humanized antibodies are those in which each of framework regions (FRs) 1, 2, 3 and 4 has at least about 90% amino acid sequence identity, preferably about 95% identity or greater to the heavy chain sequences shown in Figure 4A (SEQ ID NOS 83-96), preferably, the sequence shown as "HC-08" in Figure 4A. Additional humanized antibodies have a heavy chain constant domain with at least about 90% amino acid sequence identity, preferably at least about 95% identity or greater, to sequence shown in Figure 5B (SEQ ID NO: 98) or Figure 6B (SEQ ID NO: 100).

In certain cases, the humanized antibody will have an IgG1 (hOAT) or IgG4 (hFAT) isotype as disclosed in WO03/037911.

Functional fragments of the humanized antibodies are also disclosed herein. Examples of such fragments include, but are not limited to, those that bind TF with an affinity constant (Kd) of less than about 1 nM, preferably less than about 0.5 nM, more preferably between from about 0.01 nM to about 0.4 nM. Specifically preferred are antigen binding Fab, Fab', and F(ab)₂ fragments.

As discussed, humanized antibodies made by the method of the present invention may include at least one murine complementarity determining region (CDR), *e.g.*, CDR1, CDR2, CDR3. For example, the antibodies may bind specifically to human tissue factor (TF) to form a complex. Typically, the factor X or factor IX binding to TF or TF:FVIIa and activation by TF:FVIIa thereto is inhibited. As mentioned above, preferred CDRs (light and heavy chain) are from a rodent source, typically the mouse.

The humanized antibodies made by the method of the invention may further include at least one human framework region (FR) subset. Preferably, all the FRs (light and heavy chains) are human.

Suitably, the first CDR (CDR1) of the heavy chain hypervariable region that binds human TF is at least 90% identical to the CDR1 amino acid sequence shown in Figure 4B (SEQ ID NO: 104), preferably at least about 95% identical or greater to that sequence. Typically, the second CDR (CDR2) of the heavy chain hypervariable region is at least 90% identical to the CDR2 amino acid sequence shown in Figure 4C (SEQ ID NOS 9 and 101), preferably at least about 95% identical or greater. Preferably also, the third CDR (CDR3) of the heavy chain hypervariable region is at least 90% identical to the CDR3 sequence shown in Figure 4D (SEQ ID NO: 10), more preferably about 95% identical or greater to that sequence.

Suitably, the first CDR (CDRs1) of the light chain hypervariable region that binds human TF is at least 90% identical to the CDR1 amino acid sequence shown in Figure 3B (SEQ ID NO: 103), preferably at least about 95% identical or greater. Typically, the second CDR (CDR2) of the light chain hypervariable region is at least 90% identical to the CDR2 amino acid sequence shown in Figure 3C (SEQ ID NO: 6), preferably about 95% identical or greater. Preferably, the third CDR (CDR3) of the light chain hypervariable region is at least 90% identical to the CDR3 amino acid sequence shown in Figure 3D (SEQ ID NO: 7), more preferably about 95% identical or greater to that sequence.

Additional humanized antibodies made by the method of the invention include a first framework region (FR1) of the heavy chain hypervariable region that binds human TF which FR1 is at least 90% identical to the amino acid sequence shown in Figure 4A (SEQ ID NO: 91) as "FR1 HC-08", preferably about 95% identical or greater to that sequence. Suitably, the FR1 comprises at least one of the following amino acid changes: E1 to Q; Q5 to V; P9 to G; L11 to V; V12 to K; Q 19 to R; and T24 to A. Preferably, the FR1 includes two, three, four, five, or six of those changes with all of those amino acid changes being preferred for many applications.

Further humanized antibodies that suitably bind human TF include a second framework region (FR2) of the heavy chain hypervariable region which FR2 is at least 90% identical to the sequence shown in Figure 4A (SEQ ID NO: 91) as "FR2 HC-08", preferably about 95% identical or greater to that sequence. Suitably, the FR2 at least one of the following amino acid changes: H41 to P; and S44 to G. A preferred FR2 includes both of those amino acid changes.

The method of the invention may also produce humanized antibodies that bind human TF in which a third framework region (FR3) of the heavy chain hypervariable region is at least 90% identical to the sequence shown in Figure 4A (SEQ ID NO: 91) as "FR3 HC-08", preferably about 95% identical or greater to that sequence. Suitably, the FR3 includes at least one of the following amino acid changes: S76 to T; T77 to S; F80 to Y; H82 to E; N84 to S; T87 to R; D89 to E; and S91 to T. A preferred FR3 includes two, three, four, five or six of those amino acid changes with all seven of those amino acid changes being generally preferred.

The method of the present invention may also produce humanized antibodies that suitably bind human TF in which the fourth framework region (FR4) of the heavy chain hypervariable region is at least 90% identical to the amino acid sequence shown in Figure 4A (SEQ ID No: 91) as "FR4 HC-08", preferably at least about 95% identical or greater to that sequence. Preferably, the FR4 includes the following amino acid change: L113 to V.

Additional humanized antibodies that may be made in accord with the method of the invention and that bind human TF feature a first framework region (FR1) of the light chain hypervariable region which is at least about 90% identical to the amino acid sequence shown in Figure 3A (SEQ ID NO: 79) as "FR1 LC-09", preferably at least about 95% identical or greater to that sequence. Suitably, the FR1 comprises at least one of the following amino acid changes: Q11 to L; L 15 to V; E 17 to D; and S 18 to R. A preferred FR1 includes two or three of such amino acid changes with all four amino acid changes being generally preferred.

The method of the present invention may also produce humanized antibodies that bind human TF in which a second framework region (FR2) of the light chain hypervariable region is at least about 90% identical to the amino acid sequence shown in Figure 3A (SEQ ID NO: 79) as "FR2 LC-09", preferably at least about 95% identical or greater to that sequence. A preferred FR2 has the following amino acid change: Q37 to L.

The method of the present invention may also produce humanized antibodies that bind human TF in which a third framework region (FR3) of the light chain hypervariable region is at least about 90% identical to the amino acid sequence shown in Figure 3A (SEQ ID NO: 79) as "FR3 LC-09", preferably at least about 95% identical or greater to that sequence. Suitably, the FR3 has at least one of the following amino acid changes: K70 to D, K74 to T, A80 to P, V84 to A, and N85 to T. Preferably, the FR3 has two, three, or four of such amino acid changes with all five of the changes being generally preferred.

Additional humanized antibodies that may be made by the method of the invention and that bind TF include a fourth framework region (FR4) of the light chain hypervariable region which FR4 is at least about 90% identical to the sequence shown in Figure 3A (SEQ ID NO: 79) as "FR4 LC-09", preferably at least about 95% identical or greater to that sequence. Suitably, the FR4 includes at least one and preferably all of the following amino acid changes: A100 to Q; and L106 to I.

The method of the invention may also produce a human TF binding fragment of the foregoing humanized antibodies. Examples of such fragments include Fab, Fab', and F(ab)₂.

WO03/037911 describes additional preferred humanized anti-TF antibodies that may be made by the method of the invention. As disclosed therein, the following three nucleic acid vectors pSUN36 (humanized anti-TF antibody Ig G1-HC expression vector), pSUN37 (humanized anti-TF antibody Ig G4-HC expression vector), and pSUN38 (humanized anti-TF antibody LC expression vector) have been deposited pursuant to the Budapest Treaty with the American Type Culture Collection (ATCC) at 10801 University Boulevard, Manassas VA 20110-2209. The vectors were assigned the following Accession Numbers: PTA-3727 (pSUN36); PTA-3728 (pSUN37); and PTA-3729 (pSUN38).

Suitable expression and purification strategies for making and using the humanized anti-TF antibodies of this invention have been disclosed in WO03/037911.

### B. Preparation Of Humanized Anti-LTA Binding Antibody

The original anti-LTA chimeric antibody is the subject of the following patent applications: "Opsonic and Protective Monoclonal and Chimeric Antibodies specific of Lipotechoic Acid of Gram Positive Bacteria" Pending U.S.S.N. 09/097,055 (Published as WO 98/57994)

Preparation and use of a humanized anti-LTA (lipotechoic acid) binding antibody is described below in Examples 4-5. Preferred antibodies generally bind LTA to form a specific binding complex. Particular chimeric anti-LTA antibodies bind antigen with an apparent affinity constant (K_{A}, M⁻¹) of less than about 1µM, preferably less than about 100 nM, more preferably between from about 20 nM to about 2 nM. See Example 5, below for further information about characterizing anti-LTA antibodies.

Lipotechoic acid is a cell component found in some Gram-positive bacteria including *Staphylococcus* species, some *Streptococcus* species and *Entercoccus.* It is incorporated into the cell wall as part of a mixed macromolecular polymer that is heterodisperse in molecular weight, and as such the LTA component can be found in cell walls and fragments thereof that can have an extremely broad molecular mass range.

More preferred humanized anti-LTA binding antibodies an association constant (K_{A}, M⁻¹) for LTA of at least about 5x10⁶ M⁻¹ as determined by surface plasmon analysis (particularly, BIACore analysis in accordance with the procedures of Example 3 which follows), more preferably at least about 1x10⁷ M⁻¹ as determined by surface plasmon analysis, still more preferably a Kₐ for LTA of at least about 1x10⁸ M⁻¹ as determined by surface plasmon resonance analysis.

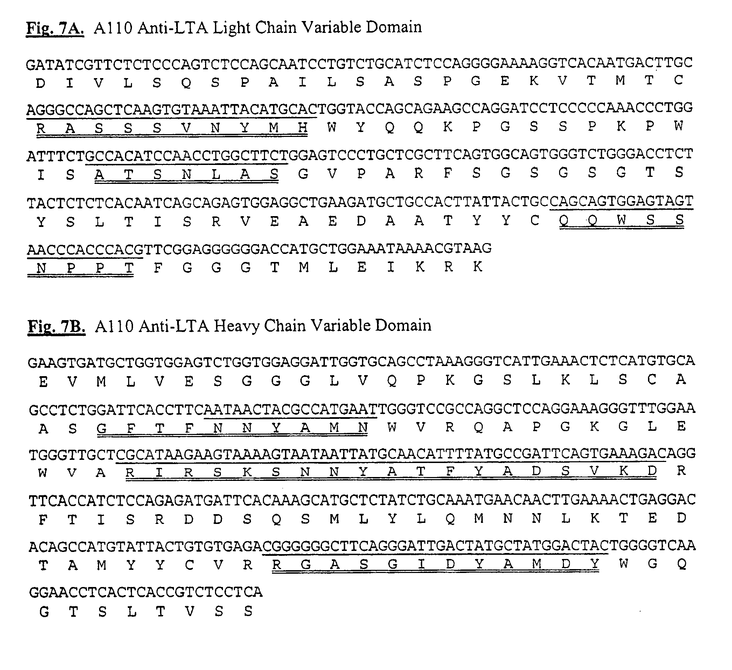

Suitably, the first CDR (CDR1) of the light chain hypervariable region that binds the LTA antigen is at least about 90% identical to the CDR1 amino acid sequence shown in Figure 7A (underlined) preferably at least about 95% or greater identity. Typically, the second CDR (CDR2) of the same light chain hypervariable region is at least about 90% identical to the CDR2 amino acid sequence shown in Figure 7A (underlined), preferably at least about 95% or greater identity. Preferably also, the third CDR (CDR3) of the light chain hypervariable region is at least about 90% identical to the CDR3 amino acid sequence shown in Figure 7A (underlined), more preferably at least about 95% or greater sequence identity.

With respect to the heavy chain hypervariable region, additionally preferred anti-LTA antibodies will exhibit at least about 90% identity to the CDR1 amino acid sequence shown in Figure 7B (underlined), preferably at least about 95% or greater identity. Typically, the second CDR (CDR2) of the same light chain hypervariable region is at least about 90% identical to the CDR2 amino acid sequence shown in Figure 7B (underlined), preferably at least about 95% or greater identity. Preferably also, the third CDR (CDR3) of the light chain hypervariable region is at least about 90% identical to the CDR3 amino acid sequence shown in Figure 7B (underlined), more preferably at least about 95% or greater sequence identity.

Additionally specific anti-LTA antibodies include those in which each of light chain framework region subsets (FRs) 1, 2, 3 and 4 has at least about 90% amino acid sequence identity, preferably at least about 95%, 98% up to 100% identity to each of the light chain FR sequences shown in Table 6 of Example 5. In one embodiment, the framework includes at least one and preferably all of the following amino acid changes: D1 to Q; S5 to T; L11 to M; E17 to D; M21 to I; S41 to Q; R76 to A; V77 to M; and M102 to K. An especially preferred anti-LTA antibody has at least one of and preferably all of each of the L chain FR subsets shown for the A110-LC in Table 6 of Example 5 *i.e.,* LC-FR1 (residues 1 to 23 of SEQ ID NO: 109); LC-FR2 (residues 24 to 38 of SEQ ID NO: 109); LC-FR3 (residues 39 to 70 of SEQ ID NO: 109) and LC-FR4 (residues 71 to 80 of SEQ ID NO: 109).

Further specific humanized antibodies that specifically bind the LTA antigen include those in which each of framework regions (FRs) 1, 2, 3 and 4 has at least about 90% amino acid sequence identity, preferably about 95%, 98% up to about 100% identity to the heavy chain sequences shown in Table 7 of Example 5. In one embodiment, the framework includes at least one and preferably all of the following amino acid changes: M3 to Q; K15 to G; Q78 to K; S79 to N; M80 to S; N87 to S; M95 to V, V99 to A and L119 to V. An especially preferred anti-LTA antibody has at least one of and preferably all of each of the H chain FR subsets shown for the A110-HC in Table 7 of Example 5. That is, HC-FR1 (residues 1 to 25 of SEQ ID NO: 118); HC-FR2 (residues 26 to 39 of SEQ ID NO: 118); HC-FR3 (residues 40 to 71 of SEQ ID NO: 118); and HC-FR4 (residues 72 to 82 of SEQ ID NO: 118).

Suitably, the humanized anti-LTA antibody will have an IgG1 heavy chain constant domain (similar to hOAT) or an IgG4 heavy chain constant domain (similar to hFAT) isotype. See WO03/037911.

Additionally preferred anti-LTA antibodies will have each of the light and heavy chain FRs shown in Tables 6 and 7 of Example 5.

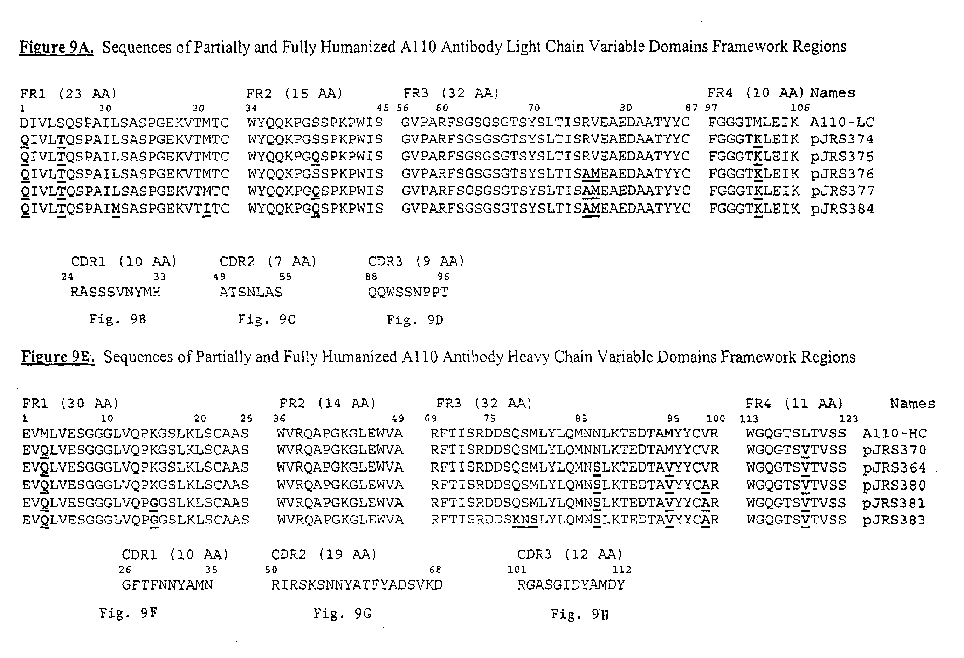

Further preferred humanized anti-LTR antibodies will have an amino acid light chain variable domain with at least 95% sequence identity, preferably at least about 98% or greater identity to the amino acid sequence shown in Figure 9A (SEQ ID NO: 109). More preferably, such an antibody will have a light chain variable domain that is the same as the sequence shown in Figure 9A (SEQ ID NO: 109). Additionally preferred antibodies will have an amino acid heavy chain variable domain with at least 95% sequence identity, preferably at least about 98% or greater identity to the amino acid sequence shown in Figure 9E (SEQ ID NO: 118). More preferably, such an antibody will have a heavy chain variable domain that is the same as the sequence shown in Figure 9E (SEQ ID NO: 118).

In a particular example, there is provided a humanized anti-LTA antibody that specifically binds LTA and includes at least one rodent CDR, usually from a mouse. Preferably, the LTA antigen binds specifically to the antibody to form a complex. Also preferably, such a humanized anti-LTA antibody includes, on the heavy chain, at least one of and preferably all of the following components:
a) a first CDR (CDR1) which is at least 95% identical to CDR1 amino acid sequence shown in Figure 9F (SEQ ID NO: 124),
b) a second CDR (CDR2) which is at least 95% identical to the CDR2 amino acid sequence shown in Figure 9G (SEQ ID NO: 125),
c) a third CDR (CDR3) which is at least 95% identical to the CDR3 amino acid sequence shown in Figure 9H (SEQ ID NO: 126),
d) a first framework subset (FR1) which is at least 95% identical to the amino acid sequence shown in Table 6A (residues 1 to 23 of SEQ ID NO: 109) as "LC-FR1",
e) a second framework subset (FR2) which is at least 95% identical to the amino acid sequence shown in Table 6A (residues 24 to 38 of SEQ ID NO: 109) as "LC-FR2",
f) a third framework subset (FR3) which is at least 95% identical to the amino acid sequence shown in Table 6B (residues 39 to 70 of SEQ ID NO: 109) as "LC-FR3", and
g) a fourth framework subset (FR4) which is at least 95% identical to the amino acid sequence shown in Table 6B (residues 71 to 80 of SEQ ID NO: 109) as "LC-FR4".

Suitably, the humanized anti-LTA antibody also includes, on the light chain, at least one of and preferably all of the following components:
h) a first CDR (CDR1) which is at least 95% identical to CDR1 amino acid sequence shown in Figure 9B (SEQ ID NO: 115),
i) a second CDR (CDR2) which is at least 95% identical to the CDR2 amino acid sequence shown in Figure 9C (SEQ ID NO: 116),
j) a third CDR (CDR3) which is at least 95% identical to the CDR3 amino acid sequence shown in Figure 9D (SEQ ID NO: 117),
k) a first framework subset (FR1) which is at least 95% identical to the amino acid sequence shown in Table 7A (residues 1 to 25 of SEQ ID NO: 118) as "HC-FR1 ",
l) a second framework subset (FR2) which is at least 95% identical to the amino acid sequence shown in Table 7A (residues 26 to 39 of SEQ ID NO: 118) as "HC-FR2",
m) a third framework subset (FR3) which is at least 95% identical to the amino acid sequence shown in Table 7B (residues 40 to 71 of SEQ ID NO: 118) as "HC-FR3", and
n) a fourth framework subset (FR4) which is at least 95% identical to the amino acid sequence shown in Table 7B (residues 72 to 82 of SEQ ID NO: 118) as "HC-FR4. Preferably, the humanized antibody further includes the light chain constant sequence of Figure 6A (hFAT (IgG4) SEQ ID NO: 99). Also preferably, the antibody includes the heavy chain constant region of Figure 6B (IgG4 SEQ ID NO: 100).

Suitably, nucleic acid molecules encode one or more of the amino acid anti-LTA light chain variable domain and anti-LTA heavy chain variable domain shown in Figure 9A (SEQ ID NOS 109-114) and 9E (SEQ ID NOS 118-123), respectively. More specific nucleic acids express at least part of an anti-LTA binding antibody that exhibits the preferred binding affinities and other properties disclosed herein. The molecular weight of such nucleic acids will generally be less than about 1000 basepairs (bp), preferably between from about 200bp to about 750bp, as determined by conventional gel electrophoresis methods.

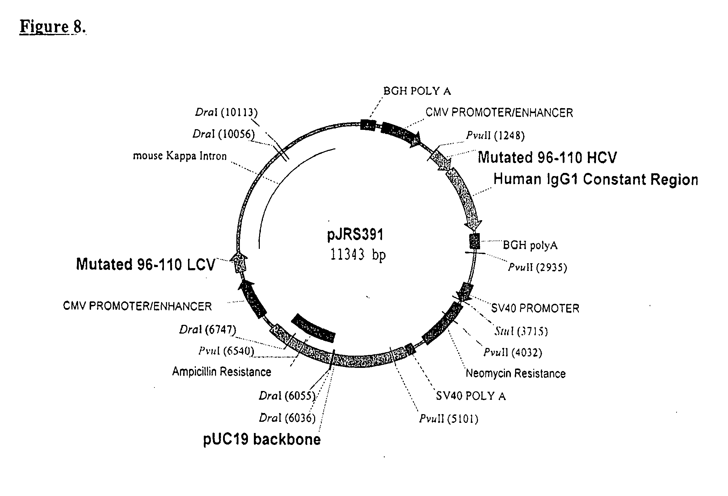

A specifically preferred nucleic acid is the plasmid represented by Figure 8 (pJRS 391).

Also provided are functional fragments of the humanized anti-LTA antibodies disclosed herein. Examples of such fragments include, but are not limited to, those that bind LTA with an apparent affinity constant (K_{A}, M⁻¹) of less than about 100 nM, preferably less than about 25 nM, more preferably between from about 1 nM to about 5 nM. Specifically preferred are antigen binding Fab, Fab', and F(ab)₂ fragments, single chain Fv and full length antibodies.

In general, nucleic acids are isolated, usually constitutes at least about 0.5%, preferably at least about 2%, and more preferably at least about 5% by weight of total nucleic acid present in a given fraction. A partially pure nucleic acid constitutes at least about 10%, preferably at least about 30%, and more preferably at least about 60% by weight of total nucleic acid present in a given fraction. A pure nucleic acid constitutes at least about 80%, preferably at least about 90%, and more preferably at least about 95% by weight of total nucleic acid present in a given fraction.

The following non-limiting examples are illustrative of the invention. In the following examples and elsewhere the method of the invention is applied to the humanization of the murine anti-tissue factor antibody H36 and the anti-lipotechoic acid antibody A110. H36 is also referred to as H36.D2 and as H36.D2.B7, but H36 is the antibody produced by the mother clone, and H36.D2 is obtained from the primary clone, whereas H36.D2.B7 is obtained from the secondary clone. No differences have been observed between the antibody produced by those three clones with respect to the antibody's ability to inhibit TF or other physical properties. In general usage, H36 is often used to indicate anti-TF antibody produced by any of these clones or related cell lines producing the antibody. The mouse-human chimeric version of H36 is referred to cH36 (and also as Sunol-cH36). The anti-lipotechoic acid antibody A110 is also referred to as 96-110, c96-110 and as BSYX-A110 and is a mouse-human chimeric antibody.

See WO03/03791 for further disclosure relating to making and using the H36 antibody. See also U.S Patent No. 5,986,065.

### EXAMPLE 1 - Humanization of Anti-Tissue Factor Antibody

The description of how to make and use a particular murine antibody called H36.D2 (sometimes also called H36 as discussed above) is described in US Pat. No. 5,986,065. The present example shows how to make and use a humanized version of that antibody. A humanized H36 antibody has a variety of uses including helping to minimize potential for human anti-mouse antibody (HAMA) immunological responses. These and other undesired responses pose problems for use of the H36 antibody in human therapeutic applications.

### A. Preparation of chimeric anti-tissue factor antibody (cH36)

The H36 antibody described previously is an IgG2a murine antibody. H36 was first converted to a mouse-human chimeric antibody for clinical development. To do this, the heavy and light chain genes for H36 were cloned (see US Patent No. 5,986,065). The heavy chain variable domain was fused to a human IgG4 constant (Fc) domain and the light chain variable domain was fused to a human kappa light chain constant domain. The resulting IgG4κ chimeric antibody was designated cH36 (and is also referred to as Sunol-cH36). For multiple uses of H36 or cH36 in patients with chronic diseases, a fully humanized cH36 is preferred so that it will decease or eliminate any human anti-chimeric antibody (HACA) immunological response. The humanization of cH36 is described below.

### B. Humanization Strategy for cH36 Antibody

Humanization of the chimeric anti-tissue factor antibody cH36 was achieved by using a "FR best-fit" method of the invention. This method takes full advantage of the fact that a great number of human IgGs with known amino acid sequences or sequences of human IgG fragments are available in the public database. The sequences of the individual framework regions of the mouse heavy and light variable domains in cH36 are compared with the sequences respective heavy or light chain variable domains or human frameworks (or fragments thereof) in the Kabat database (see http://immuno.bme.nwu.edu). The following criteria were used to select the desired human IgG framework region subsets for humanization: (1) The number of mismatched amino acids was kept as low as possible. (2) Amino acids inside the "vernier" zone (amino acids in this zone may adjust CDR structure and fine-tune the fit to antigen, see Foote, J. and Winter, G., J. of Mol. Bio. 224(2): 487-499 [1992]) were left unchanged. (3) Conservative amino acid substitutions were favored when evaluating similar candidates. The matching program used for this comparison can be found in Kabat database. See Johnson G, Wu T. "Kabat database and its application: Future directions." Nucleic Acids Res. (2001) 29:205-206. The program finds and aligns regions of homologies between the mouse sequences and human sequences in the Kabat's database. By using this unique FR best-fit method, it is anticipated that the humanized LC or HC variable domains of the target IgG may have all the four FRs derived from as few as one human IgG molecule or to as many as four different human IgG molecules.

### B(i). Selection of Human IgG Kappa Light Chain Variable Domain Framework Regions

The amino acid sequence in each of the framework regions of cH36 LC was compared with the amino acid sequence in the FRs in human IgG kappa light chain variable domain in Kabat Database. The best-fit FR was selected based on the three criteria described above.

The amino acid sequence of human IgG kappa light chain variable domain with a Kabat Database ID No. 005191 was selected for humanization of cH36 LC FR1. The amino acid sequence of human IgG kappa light chain variable domain with a Kabat Database ID No. 019308 was selected for humanization of cH36 LC FR2. The following mutations were made in cH36 LC FR1 to match the amino acid sequence of a human IgG kappa light chain variable domain with a Kabat Database ID No. 005191: Q11 → L, L15 → V, E17 → D, S18 → R. One mutation Q37 → L was made cH36 LC FR2 to match the amino acid sequence of a human IgG kappa light chain variable domain with a Kabat Database ID No. 019308 (see Table 1A for sequence information).

The amino acid sequence of a human IgG kappa light chain variable domain with a Kabat Database ID No. 038233 was selected for humanization of cH36 LC FR3. The amino acid sequence of a human IgG kappa light chain variable domain with a Kabat Database ID No. 004733 was selected for humanization of cH36 LC FR4. The following mutations were made in cH36 LC FR3 to match the amino acid sequence of a human IgG kappa light chain variable region with a Kabat Database ID No. 038233: K70 → D, K74 → T, A80 → P, V84 → A, N85 → T. Two mutations A100 → Q and L106 → I were made cH36 LC FR4 to match the amino acid sequence of a human IgG kappa light chain variable domain with a Kabat Database ID No. 004733 (see Table 1B for sequence information).

### B(ii). Selection of Human IgG Heavy Chain Variable Domain Framework Regions

The amino acid sequence in each of the framework regions of cH36 HC was compared with the amino acid sequence in the FRs in human IgG heavy chain variable domain in Kabat Database. The best-fit FR was selected based on the three criteria described above.

The amino acid sequence of a human IgG heavy chain variable domain with a Kabat Database ID No. 000042 was selected for humanization of cH36 HC FR1. The amino acid sequence of a human IgG heavy chain variable domain with a Kabat Database ID No. 023960 was selected for humanization of cH36 HC FR2. The following mutations were made in cH36 HC FR1 to match the amino acid sequence of a human IgG heavy chain variable domain with a Kabat Database ID No. 000042: E1 → Q, QS → V, P9 → G, L11 → V, V12 → K, Q19 → R, T24 → A. Two mutations H41 → P and S44 → G were made cH36 HC FR2 to match the amino acid sequence of a human IgG heavy chain variable domain with a Kabat Database ID No. 023960 (see Table 2A for sequence information).

The amino acid sequence of a human IgG heavy chain variable domain with a Kabat Database ID No. 037010 was selected for humanization of cH36 HC FR3. The amino acid sequence of a human IgG heavy chain variable domain with a Kabat Database ID No. 000049 was selected for humanization of cH36 HC FR4. The following mutations were made in cH36 HC FR3 to match the amino acid sequence of a human IgG heavy chain variable domain with a Kabat Database ID No. 037010: S76 → T, T77 → S, F80 → Y, H82 → E, N84 → S, T87 → R, D89 → E, S91 → T. One mutations L113 → V was made cH36 HC FR2 to match the amino acid sequence of a human IgG heavy chain variable domain with a Kabat Database ID No. 000049 (see Table 2B for sequence information).

**Table 1. Comparison of cH36 (SEQ ID NO: 72) and Human Light Chain (LC) (SEQ ID NO: 79) FR Sequences**

| | Table 1A | | |
|---|---|---|---|
| Names | LC-FR1 (23 aa) | | |
| cH36-LC | | | |
| Human-LC | | | |

| | Table 1B | | |
|---|---|---|---|
| Names | LC-FR3 (32 aa) | | LC-FR4 (10 aa) |
| cH36-LC | | | |
| Human-LC | | | |

**Table 2. Comparison of cH36 (SEQ ID NO: 83) and Human Heavy Chain (HC) (SEQ ID NO: 91) FR Sequences**

| | Table 2A | | |
|---|---|---|---|
| Names | HC-FR1 (30 aa) | | |
| cH36-HC | | | |
| Human-HC | | | |

| | Table 2B | | |
|---|---|---|---|
| Names | HC-FR3 (32 aa) | | HC-FR4 (11 aa) |
| cH36-HC | | | |
| Human-HC | | | |

Once the decisions on the desired human framework regions were made, the following three techniques were used to achieve the desired amino acid substitutions in both the light and heavy chains: (1) Regular PCR was used for cloning, to introduce cloning or diagnostic restriction endonuclease sites, and to change amino acid residues located at the ends of the variable domains. (2) PCR-based mutagenesis was used to change multiple amino acid residues at a time, especially when these residues were in the middle of the variable domains. (3) Site-directed mutagenesis was used to introduce one or two amino acid substitutions at a time. Site-directed mutagenesis was done following the protocol described in Stratagene's "QuickChange Site-Directed Mutagenesis Kit" (Catalog #200518).

After each step, the partially humanized clones were sequenced and some of these variable domains were later cloned into expression vectors. The plasmid tKMC180 was used to express LC mutants, and pJRS 355 or pLAM 356 vector was used to express HC mutants as IgG1 or IgG4, respectively. Some of these clones were then combined and expressed transiently in COS cells to determine the expression levels by ELISA.

The final fully humanized forms of the anti-TF heavy and light variable domains were cloned into what is sometimes referred to herein as a "mega vector" and transfected into CHO and NSO cells for IgG expression. Stable cell lines were then used to produce amounts of humanized anti-TF sufficient for analysis. The resulting humanized versions are 100% human in origin (when the CDR sequences are not considered). The humanized IgG4 kappa version is designated hFAT (humanized IgG Four Anti-Tissue Factor antibody) and the IgG1 kappa version is designated hOAT (humanized IgG One Anti-Tissue Factor antibody). These fully humanized versions of cH36 are intended for treating chronic indications, such as thrombosis, cancer and inflammatory diseases.

### C. Generation of Humanized Anti-TF Antibody Heavy Chain

1. PCR amplification and cloning into pGem T-easy of anti-TF mAb cH36 heavy chain (HC) variable domain were performed using plasmid pJAIgG4TF.A8 (an expression vector for chimeric H36) as template and primers TFHC1s2 and TFHC1as2. Primer TFHC1s2 introduced a *BsiW*1 site upstream of the initiation codon and also an amino acid change **E1** to Q in framework (FR) 1. Primer TFHC1as introduced an amino acid change **L113** to V in FR4. This step resulted in the construct **HC01.**
2. PCR-based mutagenesis using the previous construct (HC01) and the following four primers generated construct **HC02**. Upstream PCR used primers TFHC1s2 and TFHC7as. Downstream PCR used primers TFHC7s and TFHC1as2. PCR using upstream and downstream PCR products as templates and with primers TFHC1s2 and TFHC1as2 yielded **HC02**. The use of primers TFHC7s and TFHC7as introduced two amino acid changes in FR2: **H41** to P and **S44** to G.
3. PCR-based mutagenesis using HC02 as template and the following four primers generated construct **HC03**. Upstream PCR used primers TFHC1s2 and TFHC5as2. Downstream PCR used primers TFHC5s and TFHC1as2. PCR using upstream and downstream PCR products as templates and with primers TFHC1s2 and TFHC1as2 yielded **HC03**. The use of primers TFHC5s and TFHC5as2 introduced three amino acid changes in FR3: **T87** to R, **D89** to E, and **S91** to T. A *Bgl* II site was also introduced at position. 87.
4. PCR amplification was performed using primers TFHC2s and TFHC3as and **HC03** in pGem as template. TFHC2s sits upstream of the cloning site in pGem. TFHC3as sits in framework 3 and introduces two amino acid changes in FR3: **H82** to E and **N84** to S. The resulting PCR band was cloned into pGem and then the proper size insert was digested with *BsiW*1 and *Bgl* II. Cloning of this fragment into **HC03** yields **HC04.**
5. PCR-based mutagenesis using **HC04** as template and the following primers resulted in **HC05.** Upstream PCR used primers TFHC1s2 and TFHC6as. Downstream PCR used primers TFHC6s and TFHC1as2. Mutagenic PCR using upstream and downstream PCR products as templates and with primers TFHC1s2 and TFHC1as2 yielded **HC05**. This step introduced the following amino acid changes in FR3: **S76** to T, **T77** to S, and **F80** toY.
6. PCR-based mutagenesis using **HC05** as template and the following four primers generated **HC06.** Upstream PCR used primers TFHC2s and TFHC2as2. Downstream PCR used primers TFHC3s2 and TFHC1as2. Amplification using TFHC2as2 introduced an amino acid change in FR1: **P9** to G. Primer TFHC3s2 changes **Q19** to R and **T24** to A. PCR using upstream and downstream PCR products as template and with primers TFHC1s2 and TFHC1as2 yielded **HC06**.
7. A point mutation from I to M in position 2 of FR1 was spontaneously introduced during construction of **HC06.** PCR amplification using **HC06** as template and TFHC1s3 and TFHC1as2 as primers, corrected this erroneous substitution and also introduced an amino acid. change in FR1: **Q5** to V. The resulting construct was **HC07.**
8. Construct **HC08** was made by PCR-based mutagenesis using **HC07** as template and the following primers. TFHC2s and TFHC2as3 were used for the upstream product. The downstream product was previously amplified using TFHC1s3 and TFHC1as2 (see step 7). The use of primer TFHC2as3 introduced two amino acid changes in FR1: **L11** to V and **V12** to K. A spontaneous point mutation resulted in a phenylalanine to leucine (F → L) change at position 64 in CDR2. Further screening and sequencing yielded construct **HC08R1,** which has the correct sequence of F at position 64 in CDR2.
9. Two constructs, **HC11** and **HC12,** were generated by site-directed mutagenesis from **HC07.** Two complementary primers TFHC8sP and TFHC8asP were used along with **HC07** as template to produce **HC11** which contains three amino acid changes in FR1: G9 to P, L11 to V, and V12 to K. Then, **HC11** was methylated and column purified for the next round of site directed mutagenesis. PCR using **HC11** as a template and the complementary primers TFHC9sL and TFHC0asL generated **HC12** which has a mutation from **V11** to L in FR1.
10. Construct **HC09** was derived from **HC12** by performing PCR using **HC12** as a template and the complementary primers TFHC10sK and TFHC10asK. **HC09** contains an amino acid change: **K12** to V in FR1.
11. Construct **HC10** was made from **HC09.** PCR using **HC09** as a template and the complementary primers LV-1 and LV-2 resulted in the generation of **HC10,** which contains a mutation from **L11** to V in FR1.

After each mutation step, the partially humanized or fully humanized clones were sequenced and some of these variable domains were later cloned into expression vectors. pJRS 355 or pLAM 356 vector was used to express HC mutants fused to Fc of human IgG1 or IgG4.

Figures 3A-D summarize steps 1-11 and shows incremental amino acid changes introduced into FR1-4. Except HC08, all other heavy chain mutants and cH36 contain F at position 64 in CDR2. HC08 has a mutation from F to L at position 64. Figures 4B-D show the heavy chain CDR sequences.

### Primers Used for Heavy Chain Humanization

TFHC1s2
   5' TTTCGTACGTCTTGTCCCAGATCCAGCTGCAGCAGTC 3' (SEQ ID NO: 31)
TFHC1as2
   5' AGCGAATTCTGAGGAGACTGTGACAGTGGTGCCTTGGCCCCAG 3' (SEQ ID NO: 32)
TFHC7s
   5' GTGAGGCAGAGCCCTGGAAAGGGCCTTGAGTGGATTGG 3' (SEQ ID NO: 33)
TFHC7as
   5' CCAATCCACTCAAGGCCCTTTCCAGGGCTCTGCCTCAC 3' (SEQ ID NO: 34)
TFHC5s
   5'GCATCTCAACAGCCTGAGATCTGAAGACACTGCAGTTTATTTCTGTG 3' (SEQ ID NO: 35)
TFHC5as2
   5' CTGCAGTGTCTTCAGATCTCAGGCTGTTGAGATGCATGAAGGC 3' (SEQ ID NO: 36)
TFHC3as
   5' GTCTTCAGATCTCAGGCTGCTGAGCTCCATGAAGGCTGTGGTG 3' (SEQ ID NO: 37)
TFHC2s
   5' TACGACTCACTATAGGGCGAATTGG 3' (SEQ ID NO: 38)
TFHC6s
   5' CTGTTGACAAGTCTACCAGCACAGCCTACATGGAGCTCAGCAG 3' (SEQ ID NO: 39)
TFHC6as
   5' CTGCTGAGCTCCATGTAGGCTGTGCTGGTAGACTTGTCAACAG 3' (SEQ ID NO: 40)
TFHC2as2
   5' GCACTGAAGCCCCAGGCTTCACCAGCTCACCTCCAGACTGCTGCAGC 3' (SEQ ID NO: 41)
TFHC3s2 5'CTGGGGCTTCAGTGCGGGTATCCTGCAAGGCTTCTGGTTACTCATTCAC 3' (SEQ ID NO: 42)
TFHC1s3
   5' TCGTACGTCTTGTCCCAGATCCAGCTGGTGCAGTCTGGAGGTGAGC 3' (SEQ ID NO: 43)
TFHC2as3
   5' GCACTGAAGCCCCAGGCTTCTTCACCTCACCTCCAGACTGCACC 3' (SEQ ID NO: 44)
TFHC9sL
   5' GCAGTCTGGACCTGAGCTGAAGAAGCCTGGGG 3' (SEQ ID NO: 45)
TFHC9asL
   5' CCCCAGGCTTCTTCAGCTCAGGTCCAGACTGC 3' (SEQ ID NO: 46)
TFHC8sP
   5' GCTGGTGCAGTCTGGACCTGAGGTGAAGAAGCC 3' (SEQ ID NO: 47)
TFHC8asP
   5' GGCTTCTTCACCTCAGGTCCAGACTGCACCAGC3' (SEQ ID NO: 48)
TFHC10sK
   5' GCAGTCTGGACCTGAGCTGGTGAAGCCTGGGGCTTC 3' (SEQ ID NO: 49)
TFHC10asK
   5' GAAGCCCCAGGCTTCACCAGCTCAGGTCCAGACTGC 3' (SEQ ID NO: 50)
LV-1
   5' CAGTCTGGACCTGAGGTGGTGAAGCCTGGG 3' (SEQ ID NO: 51)
LV-2
   5' CCCAGGCTTCACCACCTCAGGTCCAGACTG 3' (SEQ ID NO: 52)

### D. Generation of Humanized Anti-TF Antibody Light Chain

1. PCR amplification was performed using plasmid pJAIgG4TF.A8 (an expression vector for chimeric H36) as template and primers TFLC1s2.1 and TFLC1as2. This step introduced a cloning site, *Age*I, upstream of the coding region. It also introduced the **L106I** mutation in FR4. This step yielded the construct **LC03.**
2. Site-directed mutagenesis was performed using complementary primers TFLC5s and TFLC5as and LC03 as template. This step introduced the mutation **Q37L** in FR2 and added a *Pst*I site for diagnostic purposes. This new construct is named **LC04.**
3. PCR amplification was performed using **LC04** as template and primers TFHC2s and TFLC2as1. This step generated **Fragment A** that will be used in step 6. This step introduced **Q11L** and **L15V** mutations in FR1.
4. PCR amplification was performed using **LC04** as template and primers TFLC1s2.1 and TFLC1asR. This introduced the *Kpn*I site at the end of LC variable domain. Cloning of this PCR fragment into pGEM yields **pGEM04K** that will be used in step 6.
5. PCR amplification was performed using **LC04** as template and primers TFLC2s and TFLC4as. This step generated **Fragment C** that will be used in step 6. Three mutations **E17D, S18R** in FR1 and **A100Q** in FR4 were introduced in this step.
6. PCR-based mutagenesis using **Fragment A** and **Fragment C** as templates and primers TFHC2s and TFLC4as yielded **Fragment D.** Cloning of **Fragment D** into pGEM04K yielded the construct **LC05.**
7. PCR amplification was performed using pGEM04K as template and primers TFLC1s2.1 and TFLC4as. This step generated **Fragment H,** which is then cloned into pGEM04K. This introduced the **A100Q** mutation in FR4 and the construct is named **LC06.**
8. PCR amplification was performed using **LC06** as template and primers TFLC1s2.1 and TFLC3as. This step generated **Fragment I** that will be used in step 10. This introduced the **K70D** and the **K74T** mutations in FR3.
9. PCR amplification was performed using **LC06** as template and primers TFLC3s2 and TFLC4as. This step generated **Fragment F** that will be used in step 10. This introduced the **A80P** mutation in FR3.
10. PCR using **Fragment I** and **Fragment F** as templates and primers TFLC1s2.1 and TFLC4as yielded **Fragment J.** Cloning of **Fragment J** into pGEM yielded the construct **LC07.**
11. Site-directed mutagenesis was conduced using complementary primers TFLC08sds and TFLC08sdsa and **LC07** as template. This step introduced the mutations **V84A** and **N85T** in FR3. This construct is named **LC08.**
12. The *Age*I to *Eco*O109I fragment from **LC05** containing the mutations **Q11L, L15V, E17D, S18R** and **Q37L** is cloned into **LC08.** This yielded the construct **LC09.**
13. Site-directed mutagenesis was conduced using **LC09** as template and the complementary primers L105 and LC103. This step introduced the **T85N** mutation in FR3 and yielded the construct **LC10.**
14. Site-directed mutagenesis was conducted using **LC10** as template and the complementary primers LC 115 and LC 113. This step introduced the **D70K** mutation in FR3. This yielded the construct **LC11.**
15. Site-directed mutagenesis was conducted using **LC11** as template and the complementary primers LC125a and LC123a. This step introduced the **K42Q** mutation in FR2. This yielded the construct **LC12.**

After each mutation step, the partially humanized or fully humanized LC clones were sequenced and some of these variable domains were later cloned into expression vector tKMC180.

### Oligonucleotide Primers Used for Light Chain Humanization

TFLC1as2:
   5' TTCGAAAAGTGTACTTACGTTTGATCTCCAGCTTGGTCCCAG 3' (SEQ ID NO: 53)
TFLC1s2.1:
   5' ACCGGTGATATCCAGATGACCCAGTCTCC 3' (SEQ ID NO: 54)
TFLC5s:
   5' GGTTAGCATGGTATCTGCAGAAACCAGGG 3' (SEQ ID NO: 55)
TFLC5as:
   5' CCCTGGTTTCTGCAGATACCATGCTAACC 3' (SEQ ID NO: 56)
TFHC2s:
   5' TACGACTCACTATAGGGCGAATTGG 3' (SEQ ID NO: 57)
TFLC2as1:
   5' CCACAGATGCAGACAGGGAGGCAGGAGACTG 3' (SEQ ID NO: 58)
TFLC1asR:
   5' TTCGAAAAGTGTACTTACGTTTGATCTCCAGCTTGGTACCAGCACCGAACG 3' (SEQ ID NO: 59)
TFLC2s:
   5'CCTGTCTGCATCTGTGGGAGATAGGGTCACCATCACATGC 3' (SEQ ID NO: 60)
TFLC4as:
   5' GATCTCCAGCTTGGTACCCTGACCGAACGTGAATGG 3' (SEQ ID NO: 61)
TFLC3as:
   5' GTAGGCTGCTGATCGTGAAAGAAAAGTCTGTGCCAGATCC 3' (SEQ ID NO: 62)
TFLC3s2:
   5' CACGATCAGCAGCCTACAGCCTGAAGATTTTGTAAATTATTACTGTC 3' (SEQ ID NO: 63)
TFLC08sds:
   5' GCAGCCTACAGCCTGAAGATTTTGCAACTTATTACTGTCAACAAG 3' (SEQ ID NO: 64)
TFLC08sdsa:
   5' CTTGTTGACAGTAATAAGTTGCAAAATCTTCAGGCTGTAGGCTGC 3' (SEQ ID NO: 65)
LC105:
   5' CAGCAGCCTACAGCCTGAAGATTTTGCAAATTATTACTGTCAAC 3' (SEQ ID NO: 66)
LC103:
   5' GTTGACAGTAATAATTTGCAAAATCTTCAGGCTGTAGGCTGCTG 3' (SEQ ID NO: 67)
LC115:
   5' CAGTGGATCTGGCACAAAGTTTTCTTTCACGATCAGCAGC 3' (SEQ ID NO: 68)
LC113:
   5' GCTGCTGATCGTGAAAGAAAACTTTGTGCCAGATCCACTG 3' (SEQ ID NO: 69)
LC125a:
   5' CTGCAGAAACCAGGGCAATCTCCTCAGCTCCTG 3' (SEQ ID NO: 70)
LC123a:
   5' CAGGAGCTGAGGAGATTGCCCTGGTTTCTGCAG 3' (SEQ ID NO: 71)

Figure 5A shows the sequence of the human kappa light chain constant domain (SEQ ID NO: 97). Figure 5B shows the human IgG1 heavy chain constant domain (SEQ ID NO: 98). Figure 6A shows the hFAT (IgG4) constant domain sequence (SEQ ID NO: 99). Figure 6B provides the human IgG4 heavy chain constant domain (SEQ ID NO: 100). See also the WO03/03791 for additional disclosure relating to the foregoing immunoglobulin constant domain sequences.

### EXAMPLE 2 - Expression and Purification of Humanized anti-TF Antibodies

The partially humanized or fully humanized LC and HC clones were cloned into expression vectors. The plasmid tKMC18 was used to express LC mutants fused to human kappa chain, and pJRS 355 or pLAM 356 vector was used to express HC mutants fused to Fc of human IgG1 or IgG4. Some combinations of the HC and LC clones were then co-transfected into COS cells. The transiently expressed IgGs in COS cells were assayed for the whole IgG production and binding to TF by ELISA. For disclosure relating to these particular vectors see WO03/037911.

The final fully humanized forms of the anti-TF heavy and light variable domains (combination of HC08 and LC09) were cloned into what is referred to as a Mega expression vector (pSUN34, see Figure 2) and transfected into CHO and NSO cells for IgG expression. Stably transfected cell lines producing the IgG4κ or IgGlκ humanized anti-TF antibody were cloned. The selected stable cell lines were then used to produce amounts of humanized anti-TF sufficient for analysis. The resulting humanized versions are approximately 100% human in origin (when the CDR sequences are not considered). The humanized IgG4 kappa version (produced by pSUN35) is designated hFAT (humanized IgG Four Anti-Tissue Factor antibody) and the IgG1 kappa version (produced by pSUN34) is designated hOAT (humanized IgG One Anti-Tissue Factor antibody). These fully humanized versions of cH36 are intended for treating chronic indications, such as cancer and inflammatory diseases.

One of the NSO cell lines (OAT-NSO-P10A7) that expresses hOAT (combination of HC08 and LC09) was thawed and extended in 10 mL of IMDM medium supplemented with 10% FBS in a 15 mL tube and centrifuged. The cell pellet was resuspended in 10 mL of fresh media and passed to a T25 flask and incubated at 37°C in 5% CO₂. In order to prepare a sufficient number of cells to inoculate a hollow fiber bioreactor, the cells were expanded to obtain a total of 6x10^{8 -}cells. A bioreactor was set up as per manufacturer's instruction manual. The harvested cells were pelleted and resuspended in 60 mL of IMDM containing 35% FBS and injected into the extracapillary space of the bioreactor. Concentrations of glucose and lactate were monitored daily and the harvest material was centrifuged and pooled. The harvested material was tested for anti-TF antibody concentrations by ELISA assay. The pooled sample containing anti-TF antibody (hOAT) were then purified and analyzed as described below.

### A. rProtein A Sepharose Fast Flow Chromatography

Recombinant humanized anti-TF monoclonal antibody consists of two light and two heavy chains. Heavy chain is a fusion of mouse variable domain (unaltered or humanized as described above) and human IgG1 or IgG4 Fc domain, while light chain contains mouse variable domain (unaltered or humanized as described above) and human κ domain. It is well established that human IgG Fc region has high affinity for Protein A or recombinant Protein A (rProtein A).

Harvest pools containing humanized anti-TF antibody (hOAT) were adjusted to pH 8.0 ± 0.1 by adding 0.08 ml of 1 M Tris-HCl, pH 8.0 per ml of sample. Then the sample is filtered through low protein-binding 0.22 micron filters (e.g., Nalgene sterile disposable tissue culture filter units with polyethersulfone membrane from Nalge Nunc International, Cat. No. 167-0020). Following sample application, rProtein A column (from Pharmacia) is washed with 5 bed volumes of 20 mM Tris-HCl, pH 8.0 to remove unbound materials such as media proteins. Since the harvest medium contains high content of bovine serum, a stepwise pH gradient wash was used to remove bovine IgG from the column. The stepwise pH gradient was achieved by increasing the relative percentage of Buffer B (100 mM acetic acid) in Buffer A (100 mM sodium acetate). A typical pH stepwise wash employed 20%, 40%, and 60% Buffer B. Elute the column with 100% Buffer B and collect fractions based on A₂₈₀. The pooled fractions were adjusted to pH 8.5 with addition of 1 M Tris base.

### B. Q Sepharose Fast Flow Chromatography

Anion ion exchange chromatography is very effective in separating proteins according to their charges. The eluted and pH-adjusted sample from rProtein A column was diluted with two volumes of water, and the pH is checked and adjusted to 8.5. The sample was then loaded to a 5 ml (1.6 x 2.5 cm) Q Sepharose Fast Flow equilibrated with 20 mM Tris-HCl, pH 8.5 and the column washed with (1) 5 bed volumes of 20 mM Tris-HCl, pH 8.5; and (2) 4 bed volumes of 20 mM Tris-HCl, pH 8.5 containing 100 mM NaCl. The IgG protein was then eluted with bed volumes of 20 mM Tris-HCl, pH 8.5 containing 500 mM NaCl. The protein peaks were pooled and buffer-exchanged into PBS using ultrafiltration device.

Using the same transfection, cell culture, and purification methods, hFAT was also produced and purified.

### EXAMPLE 3 - Properties of Humanized Anti-TF Antibodies

### A. Inhibition of TF Function by Humanized Anti-TF Antibody

One of the key properties of anti-TF antibodies is its ability to inhibit tissue factor-initiated blood coagulation. The purified hOAT and hFAT were measured for their ability to inhibit TF activity in a standard PT assay. PT assay is widely used to measure tissue factor-dependent blood clotting times. The principal of this assay is that tissue factor (TF) forms complex with factor VIIa in plasma. This complex then activates factor X to FXa; FXa then converts prothrombin to thrombin in the presence of factor Va and phospholipids. Thrombin eventually leads to formation of a blood clot. In standard PT assays, lipidated TF is added to plasma to initiate blood coagulation and the clotting is recorded by an Organon Teknika Coag-A-Mate Coagulation Analyzer or equivalent.

The anti-TF antibody, H36, inhibits human TF activity by a unique mechanism. It binds to TF (free or in complex with factor VIIa) in such a way that factor X and IX binding to TF:FVIIa complex is prohibited, thus FX and FIX activation by TF:FVIIa is blocked (see US Patent No. 5,986,065). In PT tests, the prolongation of clotting times anti-TF antibody added into human plasma is a clear indication that this TF-dependent coagulation is inhibited. The clotting time is related to the amount of TF activity. A TF standard curve is generated by measuring PT clotting times of serially diluted TF. From the data of TF standard curve, the inhibition of TF activity by anti-TF antibody is determined.

Reagents: Innovin (Cat No 68100-392) and Ci-Trol Coagulation Control, Level I (Cat No 68100-336) are obtained from VWR. Lipidated recombinant human TF was produced as described in Example 3 in US Pat. No. 5,986,065.

Method: PT test is performed at 37°C using a Coagulation Analyzer. PT reaction is initiated by adding 0.2 ml of lipidated recombinant human tissue factor (*e.g*., Innovin) into 0.1 ml of human plasma (Ci-Trol Control Level I) containing 0.01 ml buffer (50 mM Tris-HCl, pH 7.5, 0.1% BSA) or anti-TF antibody.
1. Add purified water to a vial of Innovin according to manufacturer's instruction. Warm the reagent to 37°C. The reagent is stable for a few days if stored at 4-8°C.
2. Add 1 ml purified water to each vial of Ci-Trol. Mix to solubilize. If more one vials are used, combine them into one container (e.g., a 10 ml test tube). 1 ml Ci-Trol can run 5 assays (each assay uses 2 x 0.1 ml = 0.2 ml). Ci-Trol can be stored on ice and last for a few hours.
3. From anti-TF antibody stock, make a series of anti-TF antibody solutions (200 nM to 1600 nM) with 50 mM Tris-HCl, pH 7.5, 0.1 % BSA
4. Add 10 µl of 50 mM Tris-HCl, pH 7.5, 0.1% BSA or 10 µl of diluted anti-TF to each well of the twin-well cuvette that contains 0.1 ml of Ci-Trol. Use a pipette with 0.1 ml tip to mix each well. Make sure no air bubbles are in the well. Following mixing anti-TF (or buffer) with plasma (Ci-Trol), measure clotting times within 10 min by adding 0.2 ml of Innovin to the plasma.
5. For TF standard curve, first dilute Innovin (100% TF) to 20%, 10%, 5%and 2.5% with 50 mM Tris-HCl, pH 7.5, 0.1 % BSA. Then PT assays were performed as in Step 4 but using diluted Innovin samples.

Table 3 is the summary of the effect of cH36, hOAT, and hFAT on PT clotting times. Compared to the data in Table 4, cH36, hFAT, and hOAT showed very potent inhibition of TF function. At a protein concentration of above 12.9 nM, all antibodies achieved about 95% inhibition. The results in Table 3 also indicate that humanization of anti-TF, cH36, by the method described above did not have any significant effect on cH36 inhibitory activity since both hFAT and hOAT showed very similar ability to inhibit TF-dependent blood coagulation as seen for cH36.

**Table 3. Effect on Prothrombin Times by Chimeric (cH36) and Humanized) Anti-TF Antibodies (hFAT and hOAT)^{#}**

| Anti-TF Antibody Concentrations (nM) in PT Assays | PT Time (in seconds) | | |
|---|---|---|---|
| | cH36 | hOAT | hFAT |
| 0 | 12.2 | 12.2 | 12.2 |
| 6.45 | 14.9 | nd | nd |
| 9.7 | 17.8 | 16.5 | nd |
| 12.9 | 19.8 | 18.9 | 20.5 |
| 25.8 | 40 | 33.7 | 41.7 |
| 51.6 | 101.3 | 82.1 | 94.8 |

| | | | |
|---|---|---|---|
| ^{#}All assays used the same 100% TF activity (concentration) sample as in Table 4. | | | |

**Table 4. Clotting Times and Relative Tissue Factor Activities (Concentrations)**

| Relative TF Activities (Concentrations) | PT Clotting Times (Seconds) |
|---|---|
| 100% (neat) | 11.90 |
| 20% | 13.225 |
| 10% | 14.675 |
| 5% | 16.700 |
| 2.5% | 20.000 |

### B. Determination of Affinity Constants

The affinity of humanized anti-TF antibody for TF was determined by surface plasmon resonance (BIAcore from Pharmacia Biosensor) with recombinant human tissue factor covalently immobilized on a CM5 sensor chip. The affinity constants were the average data calculated from four anti-TF monoclonal antibody concentrations (0.125 nM, 0.25 nM, 0.5 nM, and 1 nM) by the BIAcore computer software. The results in Table 5 indicate that humanization of anti-TF, cH36, by the method described above did not have any significant effect on cH36 affinity for TF since both cH36 and hFAT have similar affinity for TF.

**Table 5. Apparent Affinity and Dissociation Constants of Anti-TF Antibodies**

| Anti-TF Antibody | Apparent K_{A} (M⁻¹) | Apparent K_{d} (M) |
|---|---|---|
| H36 | 1.56 x 10¹⁰ | 6.4 x 10⁻¹¹ |
| cH36 | 7.94 x 10⁹ | 1.26 x 10⁻¹⁰ |
| hFAT | 2.99 x 10⁹ | 3.35 x 10⁻¹⁰ |

### EXAMPLE 4 - Humanization of anti-LTA Antibody

### A. Chimeric anti-LTA Antibody

### B. Humanization Strategy for anti-LTA antibody

Humanization of the chimeric anti-LTA (lipoteichoic acid) antibody, A110, was achieved by using an "FR best-fit" method. This method takes full advantage of the fact that a great number of human IgG variable domains with known amino acid sequences are available in the public database. The individual frameworks of the mouse heavy and light variable domains in A110 are compared with their corresponding human frameworks in the Kabat database (see http://immuno.bme.nwu.edu). The following criteria were used to select the desired human IgG frameworks for humanization. (1) The number of mismatched amino acids was kept as low as possible. (2) Amino acids inside the "vernier" zone (amino acids in this zone may adjust CDR structure and fine-tune the fit to antigen, see Foote, J. and Winter, G., J. Mol. Bio. **224;** (2) 487-499 [1992]) were left unchanged. (3) Conservative amino acid substitutions were favored when evaluating similar candidates. The matching program used for this comparison can be found in Kabat's internet home page. The program finds and aligns regions of homologies between the mouse sequences and human sequences in the database. By using this unique best-fit method, it is anticipated that the humanized LC or HC variable domain of the target IgG may have all the four FRs derived from as few as one human IgG molecule to as many as four human IgG molecules.

### B (i). Selection of Human IgG Kappa Light Chain Variable Domain Frameworks

The amino acid sequence in each of the frameworks of A110 LC was compared with the amino acid sequence in the corresponding FR in human IgG kappa light chain variable domain in Kabat Database. The best-fit FR was selected based on the three criteria described above.

The amino acid sequence of human IgG kappa light chain variable domain with a Kabat Database ID No. 036047 was selected for humanization of A110 LC FR1 and FR3. The amino acid sequence of human IgG kappa light chain variable domain with a Kabat Database ID No. 037658 was selected for humanization of A110 LC FR2 and No. 004763 was selected for humanization of FR4. The following mutations were made in A110 LC to match the amino acid sequence of a human IgG kappa light chain variable domain with selected FRs: D1 → Q, S5 → T, L11 → M, M21 → I, S42 → Q, R76 → A, V77 → M, and, M102 → K. (see Table 6 for sequence information).

### B (ii). Selection of Human IgG Heavy Chain Variable Domain Frameworks

The amino acid sequence in each of the frameworks of A110 HC was compared with the amino acid sequence in the corresponding FR in human IgG heavy chain variable domain in Kabat Database. The best-fit FR was selected based on the three criteria described above.

The amino acid sequence of a human IgG heavy chain variable domain with a Kabat Database ID No. 000468 is selected for humanization of A110 HC FR1. The amino acid sequence of a human IgG heavy chain variable domain with a Kabat Database ID No. 000565 is selected for humanization of A110 HC FR2. The amino acid sequence of a human IgG heavy chain variable domain with a Kabat Database ID No. 000628 is selected for humanization of A110 HC FR3. The amino acid sequence of a human IgG heavy chain variable domain with a Kabat Database ID No. 031571 is selected for humanization of A110 HC FR4. The following mutations were made in A110 HC FR1 to match the amino acid sequence of a human IgG heavy chain variable domain with a Kabat Database ID No. 000468: M3 → Q, and, K15 → G. No changes were necessary for A110 HC FR2 in order to match the Kabat Database ID No. 000565 FR2 sequence. Six mutations were made A110 HC FR3 to match the amino acid sequence of a human IgG heavy chain variable domain with a Kabat Database ID No. 000628: Q78 → K, S79 → N, M80 → S, N87 → S, M95 → V, and, V99 → A. One mutation, L119 → V was made A110 HC FR4 to match the amino acid sequence of a human IgG heavy chain variable domain with a Kabat Database ID No. 031571 (see Table 7 for sequence information).

**Table 6. Comparison of A110 (SEQ ID NO: 109) and Human Light Chain (LC) (SEQ ID NO: 127) FR Sequences**

| | Table 6A | |
|---|---|---|
| Names | | LC-FR1 (23 aa) |
| A110-LC | | |
| Human-LC | | |

| | Table 6B | |
|---|---|---|
| Names | | LC-FR3 (32 aa) |
| A110-LC | | |
| Human-LC | | |

**Table 7. Comparison of A110 (SEQ ID NO: 118) and Human Heavy Chain (HC) (SEQ ID NO: 123) FR Sequences**

| | Table 7A | |
|---|---|---|
| Names | | HC-FR1 (25 aa) |
| A110-HC | | |
| Human-HC | | |

| | Table 7B | |
|---|---|---|
| Names | | HC-FR3 (32 aa) |
| A110-HC | | |
| Human-HC | | |

Once the decision on the desired human frameworks were made, the following three techniques were used to achieve the desired amino acid substitutions in both the light and heavy chains. (1) Regular PCR was used for cloning, to introduce cloning or diagnostic endonuclease sites, and to change amino acid residues located at the ends of the variable domains. (2) Mutagenic PCR was used to change multiple amino acid residues at a time, especially when these residues were in the middle of the variable domains. (3) Site directed mutagenesis was used to introduce one or two amino acid substitutions at a time. Site directed mutagenesis was done following the protocol described in Stratagene's "QuickChange Site-Directed Mutagenesis Kit" (Catalog #200518).

After each step, the partially humanized clones were sequenced and some of these variable domains were later cloned into expression vectors. The plasmid tKMC180 was used to express LC mutants, and pJRS355 was used to express HC mutants as IgG1. Some of these clones were then combined and expressed transiently in COS cells.

The final fully humanized forms of the anti-LTA heavy and light variable domains were cloned into what is sometimes referred to herein as a "mega vector" and transfected into COS cells for IgG expression and LTA binding analysis.

### C. Generation of Humanized Anti-LTA Antibody Heavy Chain

1. PCR amplification and cloning into pGEM T-easy (*Promega*) of anti-LTA mAb A110 heavy chain (HC) variable domain were performed using plasmid pJRS334 (an expression vector for A110) as template and primers HChuF1 and HChuR2. Primer HChuF1 introduced a *BsiW*1 site upstream of the first codon of the variable domain and also an amino acid change **M3** to **Q** in framework (FR) 1. Primer HChuR2 introduced an amino acid change **L119** to **V** in FR4 and a C-terminal *EcoR*I restriction site for cloning purposes. This step resulted in the construct **pJRS362.** This fragment was then sub-cloned into the expression vector pJRS355 as a *BsiW*I to *EcoR*I restriction fragment resulting in pJRS370.
2. PCR amplification of anti-LTA mAb A110 heavy chain (HC) variable domain fragments was performed using plasmid pJRS334 (an expression vector for A110) as template and primers HChuF1 and HChuR1 for the N-terminal fragment and primers HChuF2 and HChuR2 for the C-terminal fragment. The PCR resulted in a variable domain fragment containing two additional mutations, **N87** to **S** and **M95** to **V** into FR3. The cloning of this fragment into pGEM T-Easy resulted in the construct **pJRS364.**
3. Site directed mutagenesis using the QuikChange system (*Stratagene*) was then used to introduce another mutation into the heavy chain variable domain. A pair of complementary primers JSS80 and JSS81 were combined with plasmid pJRS364 and amplfied with *Pfu*Turbo DNA polymerase. Following digestion of the products with *Dpn*I, the DNA was used to transform *E. coli*, XL1B cells. This manipulation resulted in the **V99 to A** mutation in FR3 and the construct was designated **pJRS373.** The cloning of this fragment into the expression vector pJRS355 as a *BsiW*I to *EcoR*I restriction fragment resulted in plasmid **pJRS380.**
4. Site directed mutagenesis using the QuikChange system (*Stratagene*) was then used to introduce another mutation into the heavy chain variable domain. A pair of complementary primers JSS82 and JSS83 were combined with plasmid pJRS373 and amplfied with *Pfu*Turbo DNA polymerase. Following digestion of the products, the DNA was used to transform *E. coli,* XL1B cells. This manipulation resulted in the **K15 to G** mutation in FR1 and the construct was designated **pJRS378.** The cloning of this fragment into the expression vector pJRS355 as a *BsiW*I to *EcoR*I restriction fragment resulted in plasmid **pJRS381.**
5. PCR amplification of anti-LTA mAb A110 heavy chain (HC) variable domain fragments was performed using plasmid pJRS381 (an expression vector for a mutated HCV of A110) as template. Primers MV-HC Leader and JSS87, for the N-terminal fragment, and primers JSS86 and HCV Back, for the C-terminal fragment. The PCR resulted in a variable domain fragment containing three additional mutations, **Q78** to **K, S79** to **N, and, M80 to S** into FR3. The cloning of this fragment into pJRS355 as a *BsiW*I to *EcoR*I restriction fragment resulted in the construct **pJRS383.**

### Primers Used for Heavy Chain Humanization

### D. Generation of Humanized Anti-LTA Antibody Light Chain

1. PCR amplification and cloning into pGEM T-easy (*Promega*) of anti-LTA mAb A110 heavy chain (LC) variable domain were performed using plasmid pJRS334 (an expression vector for A110) as template and primers LChuF1 and LChuR3. Primer LChuF1 introduced a *Age*I site upstream of the first codon of the variable domain and also amino acid changes, **D1** to **Q** and **S5** to **T,** in framework 1. Primer LChuR3 introduced an amino acid change **M102 to L** in FR4 and a C-terminal *BstB*I restriction site for cloning purposes. This step resulted in the construct **pJRS363.**
2. PCR amplification of anti-LTA mAb A110 light chain (LC) variable domain fragments was performed using plasmid pJRS334 (an expression vector for A110) as template and primers LChuF1 and LChuR1 for one N-terminal fragment (LCN1) and primers LChuF1 and LChuR2 for a second N-terminal fragment (LCN2). Two C-terminal fragments were generated using the primers LChuF2 and LChuR3 (LCC1) for one, and LChuF3 and LChuR3 for the second (LCC2). An internal fragment for PCR was also generated using the primers LChuF2 and LChuR2 (LCI).
3. PCR reactions using fragments LCN1 and LCC1 as templates and LChuF1 and LChuR3 as primers were performed and the resulting products were cloned into pGEM T-easy to produce the plasmid **pJRS365.** This introduced another mutation, **S41** to **Q** in FR2.
4. PCR reactions using fragments LCN2 and LCC2 as templates and LChuF1 and LChuR3 as primers were performed and the resulting products were cloned into pGEM T-easy to produce the plasmid **pJRS366.** This introduced two other mutations, **R76** to **A** and **V77** to **M** in FR3.
5. PCR reactions using fragments LCI and LCC2 as templates and LChuF1 and LChuR3 as primers were performed. A second PCR was then done using this new fragment and LCN1 as template and LChuF1 and LChuR3 as primers. The resulting product was cloned into pGEM T-easy to produce the plasmid **pJRS367.** This combined all of the light chain mutations into one light chain variable domain fragment.
6. The **L102** in all of the LC variable domain clones was then converted to a **K** by PCR. Using plasmids pJRS363, 365, 366 and 367 as templates, the variable domains were re-amplified using primers LChuF1 and LChuR4. The resulting products were again cloned into pGEM T-easy to generate plasmids **pJRS363K, 365K, 366K,** and **367K.**
7. All four versions of humanized variable domain were subsequently cloned into a light chain expression vector, tKMC180, as an *Age*I to *BstB*I restriction fragment. The final constructs for mammalian cell expression of the various mutant light chains were designated **pJRS374, 375, 376,** and, **377.**
8. PCR amplification of anti-LTA mAb A110 light chain (LC) variable domain fragments was performed using plasmid pJRS376 (an expression vector for humanized A110 light chain containing 6 mutations) as template and primers MV-LC leader and JSS89 for the N-terminal fragment (LCN1) and primers JSS90 and LC reverse for the C-terminal fragments. PCR reactions using these fragments as template and primers MV-LC leader and LC reverse resulted in products containing the mutations **L11** to **M** and **M21** to **I.** These products were then cloned into tKMC180, the LC expression vector, as an *Age*I to *BstB*I restriction fragment to generate the plasmid **pJRS384.**

All of the above expression vector constructs were tested positive for the ability to express antibodies in co-transfection experiments using COS cells. All of the combinations of humanized heavy and light chain A110 variable domains bound to LTA when tested using ELISA. Subsequently, combination expression vectors, that is, single plasmids with both heavy and light chain coding regions, were constructed. The final expression plasmid containing all 16 mutations (8 heavy and 8 light) made from the variable domains cut from pJRS383 and pJRS384 was designated **pJRS394.** When this plasmid was used to transfect COS cells, the resulting antibody produced was able to bind to LTA as shown by ELISA experiments.

### Primers used for cloning:

| **Name** | **Oligo Sequence** |
|---|---|
| HC-MV Leader | GGAGACCCAAGCTTGTTAAC (SEQ ID NO: 128) |
| HCV back | CCCAGAGGTGCTCTTGGAG (SEQ ID NO: 129) |
| HChuF1 | TGTTTTCGTACGTCTTGTCCGAAGTGCAGCTGGTGGAGTCTG (SEQ ID NO: 130) |
| HChuF2 | GAACAGCTTGAAAACTGAGGACACAGCCGTGTATTACTGTGTGAGAC (SEQ ID NO: 131) |
| HChuR1 | AATACACGGCTGTGTCCTCAGTTTTCAAGCTGTTCATTTGCAGATAGAGCATG (SEQ ID NO: 132) |
| HChuR2 | AATTCTGAATTCTGAGGAGACGGTCACTGAGGTTCCTTGACCCC (SEQ ID NO: 133) |
| JSS80 | CAGCCGTGTATTACTGTGCGAGACGGGGGGCTTC (SEQ ID NO: 134) |
| JSS81 | GAAGCCCCCCGTCTCGCACAGTAATACACGGCTG (SEQ ID NO: 135) |
| JSS82 | GGATTGGTGCAGCCTGGCGGGTCATTGAAACTCTC (SEQ ID NO: 136) |
| JSS83 | GAGAGTTTCAATGACCCGCCAGGCTGCACCAATCC (SEQ ID NO: 137) |
| JSS84 | GAGGCTCTGCACAACCGCTTCACGCAGAAGAGCCTCTCC (SEQ ID NO: 138) |
| JSS85 | GGAGAGGCTCTTCTGCGTGAAGCGGTTGTGCAGAGCCTC (SEQ ID NO: 139) |
| JSS86 | GATTCAAAAAACAGCCTCTATCTGCAAATGAACAACTTG (SEQ ID NO: 140) |
| JSS87 | CAGATAGAGGCTGTTTTTTGAATCATCTCTGGAGATGG (SEQ ID NO: 141) |
| LC-MV Leader | GAGACCCAAGCTTGGTACC (SEQ ID NO: 142) |
| LC reverse | CTGACTTTAACTCCTAACATG (SEQ ID NO: 143) |
| JSS88 | AATCATGTCTGCATCTCCAGGGGAAAAGGTCACAATCACTTGCAGGGCCAGCTC (SEQ ID NO: 144) |
| JSS90 | CAAGTGATTGTGACCTTTTCCCCTGGAGATGCAGACATGATTGCTGGAGACTGGGA G (SEQ ID NO: 145) |
| LChuF1 | ACTTATACCGGTCAGATCGTTCTCACCCAGTCTCCAGCAATC (SEQ ID NO: 146) |
| LChuF2 | ACCAGCAGAAGCCAGGATCCCAGCCCAAACCCTGGATTTCTG (SEQ ID NO: 147) |
| LChuF3 | CAGCGCAATGGAGGCTGAAGATGCTGCC (SEQ ID NO: 148) |
| LChuR1 | TTGGGCTGGGATCCTGGCTTCTGCTGG (SEQ ID NO: 149) |
| LChuR2 | CTTCAGCCTCCATTGCGCTGATTGTGAGAGAGTAAG (SEQ ID NO: 150) |
| LChuR3 | ATTACCTTCGAAAAGTGTACTTACGTTTTATTTCCAGGTTGGTCCCCCCTCCGAAC (SEQ ID NO: 151) |
| LChuR4 | ATTACCTTCGAAAAGTGTACTTACGTTTTATTTCCAGCTTGGTCCCCCCTCCGAAC (SEQ ID NO: 152) |

### EXAMPLE 5 - Characterization of Humanized anti-LTA Antibodies

### A. Transient production of recombinant humanized c96-110 antibody variants

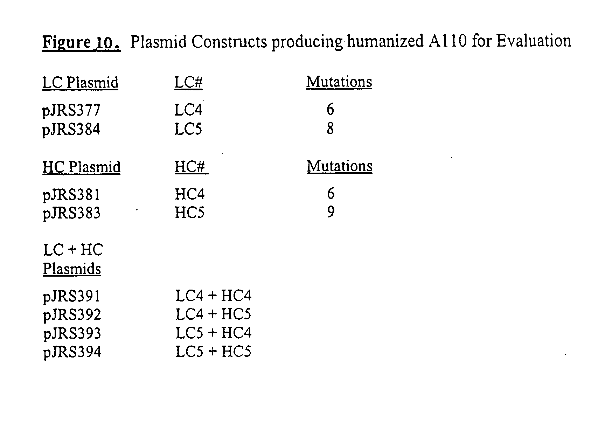

To characterize the humanized versions of the anti-LTA antibody, the plasmids pJRS334, 391, 392, 393, and 394 (see Figure 10) were transected into COS cells using Superfect (Qiagen) in 6 well tissue culture wells as described by the manufacturer. The plasmid pJRS334 encodes the c96-110 antibody and the plasmids pJRS391-4 encode humanized variants of c96-110. After two days the supernatant was assayed for the production of chimeric antibody. These antibodies were then assayed for the capability for the expressed antibody to bind to *S*. *aureus* LTA antigen.

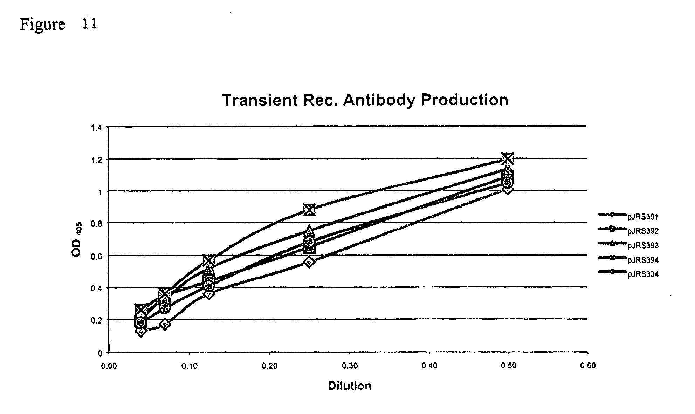

Antibody production assays were preformed in 8-well strips from 96-well microtiter plates (Maxisorp F8; Nunc, Inc.) coated at a 1:500 dilution with a goat antihuman Fc (Pierce). The plates are covered with pressure sensitive film and incubated overnight at 4°C. Plates were then washed once with Wash solution (Imidazole/NaCl/0.4%Tween-20). One hundred microliters of culture supernatant dilutions of the transiently transfected COS cells were then applied to duplicate wells and allowed to incubate for 60 minutes on plate rotator at room temperature. The plates were washed seven times with Wash solution. A Goat anti-Human IgG H+L-HRP (Zymed) conjugate was diluted 1:4000 in the sample/conjugate diluent and one hundred microliters of the dilution was added to each of the samples, and then incubated on a plate rotator for 60 minutes at room temperature. The samples were washed as above and then incubated with 100 µL/well of ABTS developing substrate (BioFx) for 1 minute at room temperature. The reaction was stopped with 100 µL/well of Quench buffer (BioFx) and the absorbance value at 405 nm was determined using an automated microtiter plate ELISA reader (see results in Table 8 and Figure 11). This assay demonstrates that the transfections of COS cells with these plasmid constructs results in the cells producing molecules containing both human IgG and Kappa domains. Approximation of antibody concentration in each of the cellular supernatants was determined by comparisons to a standard curve dilution series using human monoclonal IgG1 at 0.5 µg/mL to 0.04 µg/mL.

**Table 8. Antibody Production in COS Cells (O.D.₄₀₅ₙₘ)**

| Dilution | pJRS391 | pJRS392 | pJRS393 | pJRS394 | pJRS334 |
|---|---|---|---|---|---|
| 1:1 | 1.01 | 1.09 | 1.14 | 1.2 | 1.05 |
| 1:2 | 0.56 | 0.65 | 0.75 | 0.88 | 0.68 |
| 1:4 | 0.36 | 0.44 | 0.52 | 0.57 | 0.41 |
| 1:8 | 0.17 | 0.34 | 0.33 | 0.36 | 0.27 |
| 1:16 | 0.13 | 0.19 | 0.23 | 0.26 | 0.18 |

### B. Characterization of the Antibody Binding to LTA by the humanized variants.

The antibody containing culture supernatants from the transiently transfected COS cells were then assayed for the ability of the expressed antibodies to bind to *S*. *aureus* LTA. The activity assays were preformed in 8-well strips from 96-well microtiter plates (Maxisorp F8; Nunc, Inc.) coated at 1 µg/mL with *S*. *aureus* LTA (Sigma) using PBS. The plates were covered and incubated overnight at 4°C. Plates are then washed once with PBS. One hundred microliters of culture supernatant dilutions were then applied to duplicate wells and allowed to incubate for 60 minutes on a plate rotator at room temperature. The plates were washed seven times with Wash solution. The goat anti-Human IgG H+L-HRP (Zymed) was diluted 1:4000 in the sample/conjugate diluent and one hundred microliters of the dilution was added to each of the samples, and then incubated on a plate rotator for 60 minutes at room temperature. The samples were washed as above and then incubated with 100 µL/well of ABTS developing substrate (BioFx) for 10-15 minutes on a plate rotator at room temperature. The reaction was stopped with 100 µL/well of Quench buffer (BioFx) and the absorbance value at 405 nm was determined using an automated microtiter plate ELISA reader (see results in Table 9 and Figure 1). This assay demonstrates that the transfection of cells with these plasmid constructs result in the cells producing comparable levels of humanized A110 antibodies.

**Table 9. Antibody Binding Assay (O.D.₄₀₅ₙₘ)**

| equivalent [Ab] in ng/mL | pJRS391 | pJRS392 | pJRS393 | pJRS394 | pJRS334 |
|---|---|---|---|---|---|
| 100.00 | 1.45 | 0.86 | 1 | 1.1 | 1.65 |
| 50.00 | 0.98 | 0.4 | 0.45 | 0.45 | 0.93 |
| 25.00 | 0.42 | 0.23 | 0.27 | 0.27 | 0.43 |
| 12.50 | 0.2 | 0.13 | 0.16 | 0.15 | 0.25 |
| 6.25 | 0.1 | 0.1 | 0.1 | 0.1 | 0.13 |

### C. Affinity constant determinations of c96-110 using Surface Plasmon Resonance (SPR) analysis.

These antibodies were subsequently examined using surface plasmon resonance to evaluate antibody binding to the *S*. *aureus* LTA antigen. It is apparent from these results in Table 10 that the humanized A110 antibody produced by pJRS391 is comparable in its ability to bind LTA to the parent antibody A110 (or c96-110). Therefore the humanized version must have a similar apparent binding affinity to LTA as its parent.

### C-1. Vesicle preparation and immobilization.

Lipoteichoic acid (LTA) containing vesicles were prepared according to the method of Kalb et al., Biochemica et Biophysica Acta (1992) 1103, 307-316. Briefly, phosphatidyl-ethanolamine linoleoyl-palmitoyl (PE-L-P, SIGMA, St. Louis, MO) solution in chloroform is evaporated to dryness under vacuum. BIAcore eluent (HBS) and LTA from *Staphylococcus aureus* (SIGMA, St. Louis MO) was added to make a 0.2 mM PE-L-P solution in HBS and 1% LTA in PE-L-P. After vigorous vortexing the solution is passed 8 times through a polycarbonate filter (0.1µm pores, Nucleopore Corning). This solution is immediately injected onto the BIAcore HPA chip (BIAcore Inc. Piscataway, NJ) until a plateau of approx. 1400 RU is obtained. The vesicles spontaneously fuse to the surface of a HPA chip.

### C-2. SPR analysis

The binding kinetics were determined on a BIAcore instrument (BIAcore Inc., Piscataway, NJ) fitted with a HPA chip coated with PE-L-P/1% LTA. Different concentrations of c96-110 were injected over the surface. Since the chip surface could not be regenerated, only 1 injection per surface was performed. The association and dissociation rates were determined with the BIAevaluation Software 2.0 (BIAcore Inc., Piscataway, NJ) using the one to one binding model.

**Table 10. Apparent Affinity constants for c96-110 for different concentrations of LTA**

| **LTA conc.** | **kₐ [M⁻¹ s⁻¹]** | **k_{d} [s⁻¹]** | **K_{A} [M⁻¹]** |
|---|---|---|---|
| 0.1% | 1.68 x 10⁶ | 1.68 x 10⁻² | 1 x 10⁸ |
| 1% | 1.33 x 10⁶ | 1.15 x 10⁻² | 1.16 x 10⁸ |
| 10% | 2.07 x 10⁵ | 3.51 x 10⁻⁴ | 5.89 x 10⁸ |

### Example 6. Comparison in silico of Humanization of anti-TAC Antibody with the FR Best Fit Approach of the Invention.

To compare approaches, specific antibodies for which humanization has been performed and described in publications are compared to the results of humanization by the FR best fit approach. When the original humanization work was performed in the past, the Kabat database was not as extensive as it was when this *in silico* comparison was performed. The expansion of the database provides an increased number of frameworks that could be considered for best fit. To correct for the bias introduced as a result of the expanded database, a revised best fit search was performed and the results presented.

In this Example, the comparison was performed for the anti-TAC antibody. The *in silico* humanization is presented in Tables 11 and 12. In the first line of each table, the starting murine antibody sequence is reported and the second line is the sequence of the original humanized antibody (Queen, et al. PNAS 86: 10029-10033 (1989)) where the framework showing the best fit was 035921 for the light chain variable domain and 035918 for the heavy chain variable domain. The third line in each table shows the preferred best fit framework as determined at the time of this comparison. The last line shows the sequence that would be preferred using the FR best fit approach.

In performing this comparison, the benefits of the FR best fit approach can be seen by reviewing the overall number of amino acid substitutions required, the number of vernier residues that required changing, and the overall homology score. By minimizing the number of amino acid substitutions, the time, cost and labor involved in the actual humanization are reduced. By identifying FRs in which the vernier residues are maintained as the preferred amino acid, deleterious effects on the confirmation of the CDRs are minimized which should lead to minimal effects on antibody binding affinity. A better overall homology score (*i.e*. % homology) for the light and heavy chain frameworks is seen for the FR best fit approach compared to the other framework based approaches.

For the anti-TAC antibody, the original humanization was based on the selection of 035921 and 035918 as the frameworks with the best fit for the light chain and the heavy chain respectively. This humanization required substitution of 28 (five of which are vernier residues) of the 86 amino acids in the light chain framework and 29 (five of which are vernier residues) of 87 amino acids in the heavy chain framework. Using the expanded Kabat database, the CEA4-8A (004752) framework had the best fit for the light chain and the A10 (045903) antibody had the best fit for the heavy chain (using relaxed criteria which assumes that the light and heavy chain variable domain frameworks do not have to come from the same antibody). This resulted in a better fit compared to the original humanized anti-TAC, and would require fewer total substitutions (30 vs. 57) and fewer changes in vernier zone residues (4 vs. 10) and better homology. But the FR best fit gives the superior result, requiring only 23 amino acid substitutions, of which only 3 are vernier residues. The overall homology between the murine anti-TAC and the original humanized anti-TAC was 74.3%. The overall homology improves to 86.5% with the expanded database but improves to 89.6% when the FR best fit approach is applied.

**Comparison of Humanization Approaches for Anti-TAC Light Chain**

| | aa Changes | Vernier | %Homology |
|---|---|---|---|
| huAnti-TAC | 28/106 | 5 | 73.6% |
| CEA4-8A (004752) | 24/106 | 3 | 77.4% |
| FR Best Fit | 18/106 | 2 | 83% |

**Comparison of Humanization Approaches for Anti-TAC Heavy chain**

| | aa Changes | Vernier | %Homology |
|---|---|---|---|
| huAnti-TAC | 29/116 | 5 | 75% |
| A10(045903) | 6/116 | 1 | 94.8% |
| FR Best Fit | S/116 | 1 | 95.7% |

**Overall Comparison of Humanization Approaches for Anti-TAC Variable Domains**

| | aa Changes | Vernier | %Homology |
|---|---|---|---|
| huAnti-TAC | 57/222 | 10 | 74.3% |
| A10 (045903) | 30/222 | 4 | 86.5% |
| FR Best Fit | 23/222 | 3 | 89.6% |

**Table 11. Comparison of humanized anti-TAC Light Chain (huAnti-TAC) (SEQ ID NO: 154), muAnti-TAC (SEQ ID NO: 153) and CEA4-8A (SEQ ID NO: 155) by approaches including the FR Best Fit Approach (SEQ ID NO: 156)**

| | Table 11A | | |
|---|---|---|---|
| Names | | LC-FR1 (23 aa) | |
| muAnti-TAC | | | |
| huAnti-TAC | | | |
| CEA4-8A | | | |
| FR Best Fit | | | |

| | Table 11B | | |
|---|---|---|---|
| Names | | LC-FR3 (32 aa) | LC-FR4 (10 aa) |
| muAnti-TAC | | | |
| huAnti-TAC | | | |
| CEA4-8A | | | |
| FR Best Fit | | | |

**Table 12. Comparison of humanized anti-TAC Heavy Chain (huAnti-TAC) (SEQ ID NO: 158), muAnti-TAC (SEQ ID NO: 157) and A10 (045903) (SEQ ID NO: 159) by approaches including the FR Best Fit Approach (SEQ ID NO: 160)**

| | Table 12A | | |
|---|---|---|---|
| Names | | HC-FR1 (30 aa) | |
| muAnti-TAC | | | |
| huAnti-TAC | | | |
| A10 (045903) | | | |
| FR Best Fit | | | |

| | Table 12B | | |
|---|---|---|---|
| Names | | HC-FR3 (32 aa) | HC-FR4 (11 aa) |
| muAnti-TAC | | | |
| huAnti-TAC | | | |
| A10 (045903) | | | |
| FR Best Fit | | | |

### Example 7. Comparison in silico of Humanization of the Mc3 antibody with the FR Best Fit Approach of the Invention.

As in the previous example, a second humanized antibody is re-examined *in silico* in this example to compare the humanization approach used for this antibody to humanization by the FR best fit approach. A revised best fit search was performed and the results presented in order to correct for the bias introduced as a result of the expanded database.

In this Example, the comparison was performed for the Mc3 antibody. The *in silico* humanization is presented in Tables 13 and 14. In the first line of each table, the starting murine antibody sequence is reported and the second line is the sequence of the original humanized antibody (US Pat. No. 5,639,641). The third line in each table shows the preferred best fit framework as determined at the time of this comparison. The last line shows the sequence that would be preferred using the FR best fit approach.

For the Mc3 antibody, the original humanization was disclosed in US Pat. No. 5,639,641. This humanization required substitution of 16 residues (none of which were vernier residues) of the 107 amino acids in the light chain framework and 14 residues (none of which were vernier residues) of 117 amino acids in the heavy chain framework. Using the expanded Kabat database, the VL Clone 47 (024300) framework had the best fit for the light chain variable domain and the A10 (045903) antibody had the best fit for the heavy chain variable domain (using relaxed criteria which assumes that the light and heavy chain variable domain frameworks do not have to come from the same antibody). Even using these frameworks, the resulting in silico "humanized' antibody is not as good as the originally humanized antibody. This is due to the fact that additional changes were recommended by the method originally for the original humanized Mc3. The best fit framework from the *in silico* humanization would require 35 amino acid substitutions, compared to the 30 amino acid changes that were required by the original humanization. No changes in vernier zone residues are required in either humanization. But invention's FR best fit approach gives the superior result, requiring only 26 amino acid substitutions, and no vernier residues changes. The overall homology between the murine Mc3 and the *in silico* humanized example is 82.2% and 85% for the original humanized Mc3. The best overall is 89.7% for present FR best fit approach.

**Comparison of Humanization Approaches for Mc3 Light Chain**

| | aa Changes | Vernier | %Homology |
|---|---|---|---|
| huMc3 (037000) | 16/107 | 0 | 85% |
| Framework (024300) | 19/107 | 0 | 82.2% |
| FR Best Fit | 11/107 | 0 | 89.7% |
| huMc3 (037000) | 14/117 | 0 | 88% |
| Framework (045903) | 16/117 | 0 | 86.3% |
| FR Best Fit | 15/117 | 0 | 87.2% |

**Overall Comparison of Humanization Approaches for Mc3 Variable Domains**

| | aa Changes | Vernier | %Homology |
|---|---|---|---|
| huMc3(037000) | 30/224 | 0 | 86.6% |
| Framework | 35/224 | 0 | 84.4% |
| FR Best Fit | 26/224 | 0 | 88.4% |

**Table 13. Comparison of humanized Mc3 Light Chain (HuMc3-LC) (SEQ ID NO: 162) and Mc3-LC (SEQ ID NO: 161) using Immunogen's Templates vs. Framework Best Fit (SEQ ID NO: 163) vs. FR Best Fit Approach (SEQ ID NO: 164)**

| | Table 13A | | |
|---|---|---|---|
| Names | | LC-FR1 (23 aa) | |
| Mc3-LC | | | |
| HuMc3-LC | | | |
| Framework | | | |
| FR Best Fit | | | |

| | Table 13B | | |
|---|---|---|---|
| Names | | LC-FR3 (32 aa) | LC-FR4 (10 aa) |
| Mc3-LC | | | |
| HuMc3-LC | | | |
| Framework | | | |
| FR Best Fit | | | |

**Table 14. Comparison of humanized Mc3 Heavy Chain (HuMc3-HC) (SEQ ID NO: 166) and Mc3-HC (SEQ ID NO: 165) using Immunogen's Templates vs. Framework Best Fit (SEQ ID NO: 167) vs. FR Best Fit Approach (SEQ ID NO: 168)**

| | Table 14A | | |
|---|---|---|---|
| Names | | HC-FR1 (30 aa) | |
| Mc3-HC | | | |
| HuMc3-HC | | | |
| Framework | | | |
| FR Best Fit | | | |

| | Table 14B | | |
|---|---|---|---|
| Names | | HC-FR3 (32 aa) | HC-FR4 (11 aa) |
| Mc3-HC | | | |
| HuMc3-HC | | | |
| Framework | | | |
| FR Best Fit | | | |

### Example 8. Comparison in silico of Humanization of anti-TF Antibody: Framework vs. FR Best Fit Approach.

Another comparison of humanization methods can be made using the antibody from Example 1 described above. A comparison is made for the best fit by entire framework approach and compared to the FR best fit approach for the anti-TF antibody. Again this comparison was performed *in silico* more recently compared to the original humanization and the resulting best fit is not always identical to the results presented in Example 1 due to the expansion of the Kabat database providing FRs with better fits. Nonetheless, the conclusion remains that the FR best fit approach provides advantages over the framework best fit approaches.

When the search is performed to identify the best fit framework, the results are as shown below in Tables 15 and 16. The first line in the table is the original murine monoclonal antibody sequence, the second line shows the original FR best fit sequence for the humanized anti-TF antibody from Example 1, the third line is the sequence for the best fit framework and the fourth line is the best fit determined more recently using the FR best fit approach. The best fit framework for the anti-TF antibody light chain (Table 15) is scF11 (041950) which would require 13 amino acids substitutions, changing 1 vernier zone residues and a homology of 87.9 %. The updated FR best fits for the anti-TF antibody light chain are 041950 for FR1, 019308 for FR2, 038233 for FR3, and 036038 for FR4 which would require 10 amino acids substitutions, changing 0 vernier zone residues and a homology of 90.7%. The best fit framework for the anti-TF antibody heavy chain (Table 16) is A10 (045903) which would require 20 amino acids substitutions, changing 1 vernier zone residues and a homology of 82.9%. The FR best fit approach for the anti-TF antibody heavy chain is 000042 for FR1, 023960 for FR2, 045903 for FR3, and 047722 for FR4 which would require 15 amino acids substitutions, changing 0 vernier zone residues and a homology of 87.2 %. Overall the FR best fit approach requires 25 amino acids substitutions, changing no vernier zone residues and a homology of 88.8 % compared to the framework best fit approach which would require 33 amino acids substitutions, changing 2 vernier zone residues and a homology of 85.3 %.

**Overall Comparison of Humanization Approaches for Anti-TF Variable Domains**

| | aa Changes | Vernier | %Homology |
|---|---|---|---|
| Example 1 | 30/224 | 0 | 86.6 |
| Framework | 33/224 | 2 | 85.3 |
| FR Best Fit | 25/224 | 0 | 88.8 |

**Table 15. Comparison of humanized anti-TF Light Chain (cH36-LC) (SEQ ID NO: 72) and (Human-LC) (SEQ ID NO: 79) using Framework Best Fit (SEQ ID NO: 169) vs. FR Best Fit Approach (SEQ ID NO: 170)**

| | Table 15A | | |
|---|---|---|---|
| Names | | LC-FR1 (23 aa) | |
| cH36-LC | | | |
| Human-LC | | | |
| Framework | | | |
| FR Best Fit | | | |

| | Table 15B | | |
|---|---|---|---|
| Names | | LC-FR3 (32 aa) | LC-FR4 (10 aa) |
| cH36-LC | | | |
| Human-LC | | | |
| Framework | | | |
| FR Best Fit | | | |

**Table 16. Comparison of humanized anti-TF Heavy Chain (cH36-HC) (SEQ ID NO: 83) and (Human-HC) (SEQ ID NO: 91) using Framework Best Fit (SEQ ID NO: 171) vs. FR Best Fit Approach (SEQ ID NO: 172)**

| | Table 16A | | |
|---|---|---|---|
| Names | | HC-FR1 (30 aa) | |
| cH36-HC | | | |
| Human-HC | | | |
| Framework | | | |
| FR Best Fit | | | |

| | Table 16B | | |
|---|---|---|---|
| Names | | HC-FR3 (32 aa) | HC-FR4 (11 aa) |
| cH36-HC | | | |
| Human-HC | | | |
| Framework | | | |
| FR Best Fit. | | | |

### Example 9. Comparison in silico of Humanization of anti-LTA Antibody: Framework vs. FR Best Fit Approach.

Another comparison of humanization methods can be made using the antibody from Example 3 described above. A comparison is made for the best fit by entire framework approach and compared to the FR best fit approach for the anti-LTA antibody (A110). As in Example 8, this comparison was performed *in silico* more recently compared to the original humanization and the resulting best fit might not have been identical to the results presented in Example 3 due to the expansion of the Kabat database providing FRs with better fits. In this particular case the FRs with the best fit are identical in both the earlier humanization and in this more recent *in silico* comparison. However the results support the conclusion that the FR best fit approach provides advantages over the framework best fit approaches.

When the search is performed to identify the best fit framework, the results are as shown below in Tables 17 and 18. The first line in the table is the original murine monoclonal antibody sequence, the second line shows the original FR best fit sequence for the humanized anti-LTA antibody from Example 4, which turns out to be the same result when the FR best fit search was conducted more recently, and the third line is the sequence for the best fit framework determined recently. The best fit framework for the anti-LTA antibody light chain variable domain (Table 17) is 036047 which would require 13 amino acids substitutions out of 107 amino acids, changing 2 vernier zone residues and a homology of 87.9%. The FR best fits for the anti-LTA antibody light chain are 036047 for FR1, 037658 for FR2, 036047 for FR3, and 004763 for FR4 which would require 9 amino acids substitutions, changing no vernier zone residues and a homology of 91.6%. The best fit framework for the anti-LTA antibody heavy chain variable domain (Table 18) is 028897 which would require 15 amino acids substitutions out of 123 amino acids, changing 4 vernier zone residues and a homology of 87.8%. The FR best fit approach for the anti-TF antibody heavy chain is 000468 for FR1, 000565 for FR2, 000628 for FR3, and 031571 for FR4 which would require 9 amino acids substitutions, changing 2 vernier zone residues and a homology of 92.7%. Overall the FR best fit approach requires 18 amino acids substitutions, changing 2 vernier zone residues and a homology of 92.2% compared to the framework best fit approach which would require 28 amino acids substitutions, changing 6 vernier zone residues and a homology of 87.8%.

**Table 17. Comparison of humanized anti-LTA Light Chain (A110-LC) (SEQ ID NO: 109) and (Human-LC) (SEQ ID NO: 127) using Framework Best Fit (SEQ ID NO: 173)**

| | Table 17A | |
|---|---|---|
| Names | | LC-FR1 (23 aa) |
| A110-LC | | |
| Human-LC | | |
| Framework | | |

| | Table 17B | |
|---|---|---|
| Names | | LC-FR3 (32 aa) |
| A110-LC | | |
| Human-LC | | |
| Framework | | |

**Table 18. Comparison of humanized anti-LTA Heavy Chain (A110-HC) (SEQ ID NO: 118) and (Human-HC) (SEQ ID NO: 123) using Framework Best Fit (SEQ ID NO: 174)**

| | **Table 18A** | |
|---|---|---|
| **Names** | | **HC-FR1 (25aa)** |
| A110-HC | | |
| Human-HC | | |
| Framework | | |

| | **Table 18B** | |
|---|---|---|
| **Names** | | **HC-FR3 (32aa)** |
| A110-HC | | |
| Human-HC | | |
| Framework | | |

## Claims

1. A method for producing a humanized antibody variable (V) domain, wherein the method comprises:
a) comparing the amino acid sequence of a framework region (FR) of a non-human antibody variable (V) domain to a collection of human antibody framework amino acid sequences,
b) selecting a human FR sequence from the collection having the greatest amino acid sequence identity to the non-human FR,
c) mutagenizing DNA of the non-human FR to encode a humanized FR (huFR) having an amino acid sequence at least 75% identical to the selected human FR from step b),
d) repeating steps a) thru c) for each of the FRs in the non-human V domain to produce a plurality of DNA sequences in which each DNA sequence encodes a humanized FR;
e) substituting into a first vector encoding at least the V domain of the non-human antibody, each of the huFR DNA sequences from step d) for the corresponding non-human FRs encoded by the vector; wherein the substitution operatively links each of the huFRs to its corresponding complimentarity determining region (CDR); and
(f) expressing the first vector in host cells and under conditions conducive to making the humanized antibody V domain.

2. The method of claim 1, wherein the V domain is from a non-human antibody light chain.

3. The method of claim 1, wherein the V domain is derived from non-human antibody heavy chain.

4. The method of claim 2 or claim 3, wherein the framework region (FR) of the light or heavy chain V domain, respectively, of step a) is FR1.

5. The method of claim 4, wherein the sequence identity between the FR1 and the selected human framework FR is at least 70%.

6. The method of claim 5, wherein step d) further comprises comparing a second framework region (FR2) of the non-human light or heavy chain V domain to the collection and selecting a human FR having at least 70% sequence identity.

7. The method of claim 6, wherein step d) further comprises comparing a third framework region (FR3) of the non-human light or heavy chain V domain to the collection and selecting a human FR having at least 70% sequence identity.

8. The method of claim 7, wherein step d) further comprises comparing a fourth framework region (FR4) of the non-human light or heavy chain V domain to the collection and selecting a human FR having at least 70% sequence identity.

9. The method of claim 8, wherein the humanized light or heavy chain V domain comprises covalently linked in sequence: huFR1-CDR1-huFR2-CDR2-huFR3-CDR3-huFR4.

10. The method of claim 2-9, wherein the vernier zone amino acid residues in each FR are identical in the non-human and human FR of the antibody light or heavy chain V domain.

11. The method of claim 3, wherein the first vector further comprises a human heavy chain constant domain or fragment thereof covalently linked to the humanized heavy chain V domain.

12. The method of claim 1, wherein the collection of human amino acid sequences comprises amino acid sequences of fully sequenced human antibodies.

13. The method of claim 12, wherein the collection further comprises amino acid sequences of partially sequenced human antibodies.

14. The method of claim 11, wherein the humanized heavy chain fragment has an amino acid length of between 95 to 540 amino acids.

15. A method as claimed in claim 1 for making a humanized antibody or a fragment thereof, wherein the method comprises:
a) comparing the amino acid sequence of a non-human antibody light chain variable (V) domain framework region (1-FR) to a collection of human antibody light chain amino acid sequences,
b) selecting a human FR sequence from the collection having the greatest amino acid sequence identity to the 1-FR,
c) mutagenizing DNA of the 1-FR to encode a light chain humanized FR (L-huFR) having an amino acid sequence at least 75% identical to the selected human FR from step b),
d) repeating steps a) thru c) for each of the FRs in the light chain V domain to produce a plurality of DNA sequences in which each DNA sequence encodes an L-huFR,
e) substituting into a first vector encoding at least the light chain V domain of the non-human antibody, each of the L-huFR DNA sequences from step d) for the corresponding 1-FRs encoded by the vector; wherein the substitution operatively links each of the L-huFRs to a corresponding complimentarity determining region (CDR),
f) comparing the amino acid sequence of a framework region of a non-human antibody heavy chain variable (V) domain (h-FR) to a collection of human antibody heavy chain amino acid sequences, or fragments thereof,
g) selecting a human FR sequence from the collection having the greatest amino acid sequence identity to the h-FR,
h) mutagenizing DNA of the h-FR to encode a humanized heavy chain FR (H-huFR) having an amino acid sequence at least 75% identical to the selected human FR from step g),
i) repeating steps f) thru h) for each of the h-FRs in the non-human heavy chain V region to produce a plurality of DNA sequences in which each DNA sequence encodes a H-huFR,
j) substituting into said first vector or a second vector encoding at least the heavy chain V domain of the non-human antibody, each of the H-huFR DNA sequences from step i) for the corresponding h-FRs encoded by the vector; wherein the substitution operatively links each of the H-huFRs to a corresponding heavy chain CDR, and
k) expressing the vector or vectors in the same host cells and under conditions conducive to producing humanized light and heavy chains and making the humanized antibody or the fragment thereof.

16. The method of claim 15, wherein the DNAs encoding the humanized light and heavy chains or fragments thereof are contained on said first vector and are co-expressed in the same host.

17. The methods of claims 15 and 16, wherein the host is mammalian, plant, avian or microbial.

18. The method of claim 2 or 15, wherein the first vector further comprises a human light chain constant domain or fragment thereof covalently linked to the humanized light chain V domain.

19. The method of claim 18, wherein the light chain constant domain is Cκ, Cγ or a fragment thereof.

20. The method of claim 19, wherein the humanized light chain fragment has an amino acid length of between from 95 to 235 amino acids.

21. The method of claim 15, wherein the second vector further comprises a human heavy chain constant domain or fragment thereof covalently linked to the humanized heavy chain V domain.

22. The method of claim 11 or 21, wherein the human heavy chain constant domain is one of an IgG1, IgG2, IgG3 or IgG4 isotype.

23. The method of any preceding claim, wherein the method further comprises purifying the humanized antibody or humanized antibody V domain from the host cells to produce a substantially pure preparation of the antibody.

24. The method of claim 23, wherein the substantially purified humanized antibody specifically binds antigen with an affinity not less than one-tenth of that of the parental non-human antibody.

25. The method of claim 24, wherein the parental non-human antibody is chimeric.

26. The method of claim 23, wherein the antibody specifically recognizes and binds lipotechoic acid or human tissue factor.

27. The method of claim 15, wherein the method further comprises making a humanized single-chain antibody (sc-Fv) from the humanized V domains.

28. The method of claim 15, 16 or 23, wherein the fragment of the humanized antibody is one of F(ab')2, Fab' or Fab.

## Patentansprüche

1. Verfahren zum Herstellen einer variablen (V) humanisierten Antikörperdomäne, wobei das Verfahren Folgendes umfasst:
a) Vergleichen der Aminosäuresequenz einer Gerüstregion (GR) einer variablen (V) nicht-humanen Antikörperdomäne mit einer Sammlung von Aminosäuresequenzen von Gerüsten humaner Antikörper,
b) Auswählen einer humanen GR-Sequenz aus der Sammlung, welche die größte Aminosäuresequenzidentität mit der nicht-humanen GR aufweist,
c) Mutagenisieren von DNA der nicht-humanen GR, um eine humanisierte GR (huGR) zu kodieren, die eine Aminosäuresequenz aufweist, die zu mindestens 75 % mit der ausgewählten humanen GR von Schritt b) identisch ist,
d) Wiederholen von Schritt a) bis c) für jede der GR in der nicht-humanen V-Domäne, um mehrere DNA-Sequenzen herzustellen, in denen jede DNA-Sequenz eine humanisierte GR kodiert;
e) Substituieren in einen ersten Vektor, der mindestens die V-Domäne des nicht-humanen Antikörpers kodiert, der entsprechenden, von dem Vektor kodierten, nicht-humanen GR durch jede der huGR-DNA-Sequenzen aus Schritt d), wobei die Substitution jede der huGR mit ihrer entsprechenden Komplementarität-bestimmenden Region (CDR) operativ verknüpft; und
f) Exprimieren des ersten Vektors in Wirtszellen und unter Bedingungen, die dienlich sind, um die humanisierte Antikörper-V-Domäne herzustellen.

2. Verfahren nach Anspruch 1, wobei die V-Domäne von einer leichten Kette eines nicht-humanen Antikörpers stammt.

3. Verfahren nach Anspruch 1, wobei die V-Domäne von einer schweren Kette eines nicht-humanen Antikörpers stammt.

4. Verfahren nach Anspruch 2 oder Anspruch 3, wobei die Gerüstregion (GR) der V-Domäne der leichten bzw. schweren Kette von Schritt a) GR1 ist.

5. Verfahren nach Anspruch 4, wobei die Sequenzidentität zwischen der GR1 und der ausgewählten humanen Gerüst-GR mindestens 70 % beträgt.

6. Verfahren nach Anspruch 5, wobei Schritt d) ferner das Vergleichen einer zweiten Gerüstregion (GR2) der V-Domäne der nicht-humanen leichten oder schweren Kette mit der Sammlung und Auswählen einer humanen GR mit einer Sequenzidentität von mindestens 70 % umfasst.

7. Verfahren nach Anspruch 6, wobei Schritt d) ferner das Vergleichen einer dritten Gerüstregion (GR3) der V-Domäne der nicht-humanen leichten oder schweren Kette mit der Sammlung und Auswählen einer humanen GR mit einer Sequenzidentität von mindestens 70 % umfasst.

8. Verfahren nach Anspruch 7, wobei Schritt d) ferner das Vergleichen einer vierten Gerüstregion (GR4) der V-Domäne der nicht-humanen leichten oder schweren Kette mit der Sammlung und Auswählen einer humanen GR mit einer Sequenzidentität von mindestens 70 % umfasst.

9. Verfahren nach Anspruch 8, wobei die V-Domäne der humanisierten leichten oder schweren Kette in der Sequenz huGR1-CDR1-huGR2-CDR2-huGR3-CDR2-huGR4 kovalent verknüpft ist.

10. Verfahren nach Anspruch 2-9, wobei die Aminosäurereste der Vernier-Zone in jeder GR in der nicht-humanen und humanen GR der V-Domäne der leichten oder schweren Antikörperkette identisch sind.

11. Verfahren nach Anspruch 3, wobei der erste Vektor ferner eine konstante Domäne einer humanen schweren Kette oder ein Fragment davon umfasst, die bzw. das kovalent mit der V-Domäne der humanisierten schweren Kette verknüpft ist.

12. Verfahren nach Anspruch 1, wobei die Sammlung humaner Aminosäuresequenzen Aminosäuresequenzen vollständig sequenzierter humaner Antikörper umfasst.

13. Verfahren nach Anspruch 12, wobei die Sammlung ferner Aminosäuresequenzen partiell sequenzierter humaner Antikörper umfasst.

14. Verfahren nach Anspruch 11, wobei das humanisierte Schwere-Kette-Fragment eine Aminosäurelänge von 95 bis 540 Aminosäuren aufweist.

15. Verfahren, wie in Anspruch 1 beansprucht, zum Herstellen eines humanisierten Antikörpers oder eines Fragments davon, wobei das Verfahren folgendes umfasst:
a) Vergleichen der Aminosäuresequenz einer Gerüstregion einer variablen (V) Domäne der leichten Kette eines nicht-humanen Antikörpers (1-GR) mit einer Sammlung von Aminosäuresequenzen von leichten Ketten humaner Antikörper,
b) Auswählen einer humanen GR-Sequenz aus der Sammlung, welche die größte Aminosäuresequenzidentität mit der 1-GR aufweist,
c) Mutagenisieren von DNA der 1-GR, um eine humanisierte GR einer leichten Kette (1-huGR) zu kodieren, die eine Aminosäuresequenz aufweist, die zu mindestens 75 % mit der ausgewählten humanen GR von Schritt b) identisch ist,
d) Wiederholen von Schritt a) bis c) für jede der GR in der V-Domäne der leichten Kette, um mehrere DNA-Sequenzen herzustellen, in denen jede DNA-Sequenz eine 1-huGR kodiert,
e) Substituieren in einen ersten Vektor, der mindestens die V-Domäne der leichten Kette des nicht-humanen Antikörpers kodiert, der entsprechenden, von dem Vektor kodierten, 1-GR durch jede der 1-huGR-DNA-Sequenzen aus Schritt d), wobei die Substitution jede der 1-huGR mit ihrer entsprechenden Komplementarität-bestimmenden Region (CDR) operativ verknüpft,
f) Vergleichen der Aminosäuresequenz einer Gerüstregion einer variablen (V) Domäne der schweren Kette eines nicht-humanen Antikörpers (h-GR) mit einer Sammlung von Aminosäuresequenzen von schweren Ketten humaner Antikörper oder Fragmenten davon,
g) Auswählen einer humanen GR-Sequenz aus der Sammlung, welche die größte Aminosäuresequenzidentität mit der h-GR aufweist,
h) Mutagenisieren von DNA der h-GR, um eine humanisierte GR einer schweren Kette (h-huGR) zu kodieren, die eine Aminosäuresequenz aufweist, die zu mindestens 75 % mit der ausgewählten humanen GR von Schritt g) identisch ist,
i) Wiederholen von Schritt f) bis h) für jede der h-GR in der V-Domäne der nicht-humanen schweren Kette, um mehrere DNA-Sequenzen herzustellen, in denen jede DNA-Sequenz eine H-huGR kodiert,
j) Substituieren in den ersten Vektor oder einen zweiten Vektor, der mindestens die V-Domäne der schweren Kette des nicht-humanen Antikörpers kodiert, der entsprechenden, von dem Vektor kodierten, h-GR durch jede der H-huGR-DNA-Sequenzen aus Schritt i), wobei die Substitution jede der H-huGR mit einer entsprechenden Schwere-Kette-CDR operativ verknüpft, und
k) Exprimieren des Vektors bzw. der Vektoren in denselben Wirtszellen und unter Bedingungen, die dienlich sind, um humanisierte leichte und schwere Ketten herzustellen und den humanisierten Antikörper oder das Fragment davon herzustellen.

16. Verfahren nach Anspruch 15, wobei die DNAs, welche die humanisierten leichten und schweren Ketten oder Fragmente davon kodieren, auf dem ersten Vektor enthalten sind und im selben Wirt coexprimiert werden.

17. Verfahren nach Anspruch 16 und 16, wobei der Wirt ein Säugetier, eine Pflanze, ein Vogel oder ein Mikroorganismus ist.

18. Verfahren nach Anspruch 2 oder 15, wobei der erste Vektor ferner eine konstante Domäne einer humanen leichten Kette oder ein Fragment davon aufweist, die bzw. das kovalent mit der V-Domäne der humanisierten leichten Kette verknüpft ist.

19. Verfahren nach Anspruch 18, wobei die konstante Domäne der leichten Kette C_{□}, C_{□} oder ein Fragment davon ist.

20. Verfahren nach Anspruch 19, wobei das humanisierte Leichte-Kette-Fragment eine Aminosäurelänge von 95 bis 235 Aminosäuren aufweist.

21. Verfahren nach Anspruch 15, wobei der zweite Vektor ferner eine konstante Domäne einer humanen schweren Kette oder ein Fragment davon aufweist, die bzw. das kovalent mit der V-Domäne der humanisierten schweren Kette verknüpft ist.

22. Verfahren nach Anspruch 11 oder 21, wobei die konstante Domäne der humanen schweren Kette eine aus einem IgG1-, IgG2-, IgG3- oder IgG4-Isotyp ist.

23. Verfahren nach einem der vorherigen Ansprüche, wobei das Verfahren ferner das Reinigen des humanisierten Antikörpers oder der humanisierten Antikörper-V-Domäne aus den Wirtszellen umfasst, um eine im Wesentlichen reine Zubereitung des Antikörpers herzustellen.

24. Verfahren nach Anspruch 23, wobei der im Wesentlichen gereinigte humanisierte Antikörper mit einer Affinität, die nicht geringer ist als ein Zehntel der des nicht-humanen Mutterantikörpers, spezifisch Antigen bindet.

25. Verfahren nach Anspruch 24, wobei der nicht-humane Mutterantikörper chimär ist.

26. Verfahren nach Anspruch 23, wobei der Antikörper spezifisch Lipoteichonsäure oder humanen Gewebefaktor erkennt und bindet.

27. Verfahren nach Anspruch 15, wobei das Verfahren ferner das Herstellen eines humanisierten Einzelkettenantikörpers (sc-Gv) aus humanisierten V-Domänen umfasst.

28. Verfahren nach Anspruch 15, 16 oder 23, wobei das Fragment des humanisierten Antikörpers eines aus F(ab')2, Fab' oder Fab ist.

## Revendications

1. Un procédé de production d'un domaine variable (V) d'anticorps humanisé, le procédé comprenant :
a) la comparaison de la séquence d'acides aminés d'une zone d'infrastructure (FR) d'un domaine variable (V) d'anticorps non humain à une collection de séquences d'acides aminés d'infrastructure d'anticorps humain,
b) la sélection d'une séquence FR humaine dans la collection possédant l'identité de séquence d'acides aminés la plus grande vis-à-vis de la FR non humaine,
c) la mutagénèse de l'ADN de la FR non humaine de façon à coder une FR humanisée (huFR) possédant une séquence d'acides aminés identique à au moins 75% à la FR humaine sélectionnée à l'opération b),
d) la répétition des opérations a) à c) pour chacune des FR dans le domaine V non humain de façon à produire une pluralité de séquences ADN dans lesquelles chaque séquence ADN code une FR humanisée,
e) la substitution dans un premier vecteur codant au moins le domaine V de l'anticorps non humain de chacune des séquences ADN huFR à l'opération d) par les FR non humaines correspondantes codées par le vecteur, où la substitution lie de manière opérationnelle chacune des huFR à sa zone de détermination correspondante complémentaire (CDR), et
f) l'expression du premier vecteur dans des cellules hôtes et dans des conditions propices à fabriquer le domaine V d'anticorps humanisé.

2. Le procédé selon la Revendication 1, où le domaine V provient d'une chaîne légère d'anticorps non humain.

3. Le procédé selon la Revendication 1, où le domaine V est dérivé d'une chaîne lourde d'anticorps non humain.

4. Le procédé selon la Revendication 2 ou 3, où la zone d'infrastructure (FR) du domaine V de chaîne lourde ou légère, respectivement, de l'opération a) est FR1.

5. Le procédé selon la Revendication 4, où l'identité de séquence entre la FR1 et l'infrastructure humaine sélectionnée FR est d'au moins 70%.

6. Le procédé selon la Revendication 5, où l'opération d) comprend en outre la comparaison d'une deuxième zone d'infrastructure (FR2) du domaine V de chaîne lourde ou légère non humain à la collection et la sélection d'une FR humaine possédant une identité de séquence d'au moins 70%.

7. Le procédé selon la Revendication 6, où l'opération d) comprend en outre la comparaison d'une troisième zone d'infrastructure (FR3) du domaine V de chaîne lourde ou légère non humain à la collection et la sélection d'une FR humaine possédant une identité de séquence d'au moins 70%.

8. Le procédé selon la Revendication 7, où l'opération d) comprend en outre la comparaison d'une quatrième zone d'infrastructure (FR4) du domaine V de chaîne lourde ou légère non humain à la collection et la sélection d'une FR humaine possédant une identité de séquence d'au moins 70%.

9. Le procédé selon la Revendication 8, où le domaine V de chaîne lourde ou légère humanisé comprend liés de manière covalente en séquence : huFR1-CDR1-huFR2-CDR2-huFR3-CDR3-huFR4.

10. Le procédé selon la Revendication 2 à 9, où les résidus d'acides aminés de zone de Vernier dans chaque FR sont identiques dans la FR humaine et non humaine du domaine V de chaîne lourde ou légère d'anticorps.

11. Le procédé selon la Revendication 3, où le premier vecteur comprend en outre un domaine constant de chaîne lourde humaine ou un fragment de celui-ci lié de manière covalente au domaine V de chaîne lourde humanisé.

12. Le procédé selon la Revendication 1, où la collection de séquences d'acides aminés humaines comprend des séquences d'acides aminés d'anticorps humains entièrement séquencés.

13. Le procédé selon la Revendication 12, où la collection comprend en outre des séquences d'acides aminés d'anticorps humains partiellement séquencés.

14. Le procédé selon la Revendication 11, où le fragment de chaîne lourde humanisé possède une longueur d'acides aminés se situant entre 95 et 540 acides aminés.

15. Un procédé selon la Revendication 1 destiné à la fabrication d'un anticorps humanisé ou un fragment de celui-ci, où le procédé comprend :
a) la comparaison de la séquence d'acides aminés d'une zone d'infrastructure (1-FR) de domaine variable (V) de chaîne légère d'anticorps non humain à une collection de séquences d'acides aminés de chaîne légère d'anticorps humain,
b) la sélection d'une séquence FR humaine dans la collection possédant l'identité de séquence d'acides aminés la plus grande vis-à-vis de la 1-FR,
c) la mutagénèse de l'ADN de la 1-FR de façon à coder une FR humanisée de chaîne légère (L-huFR) possédant une séquence d'acides aminés identique à au moins 75% à la FR humaine sélectionnée à l'opération b),
d) la répétition des opérations a) à c) pour chacune des FR dans le domaine V de chaîne légère de façon à produire une pluralité de séquences ADN dans lesquelles chaque séquence ADN code une L-huFR,
e) la substitution dans un premier vecteur codant au moins le domaine V de chaîne légère de l'anticorps non humain de chacune des séquences ADN L-huFR à l'opération d) par les 1-FR correspondantes codées par le vecteur, où la substitution lie de manière opérationnelle chacune des L-huFR à une zone de détermination (CDR) complémentaire correspondante,
f) la comparaison de la séquence d'acides aminés d'une zone d'infrastructure d'un domaine variable (V) de chaîne lourde d'anticorps non humain (h-FR) à une collection de séquences d'acides aminés de chaîne lourde d'anticorps humain, ou à des fragments de celui-ci,
g) la sélection d'une séquence FR humaine dans la collection possédant l'identité de séquence d'acides aminés la plus grande vis-à-vis de la h-FR,
h) la mutagénèse de l'ADN de la h-FR de façon à coder une FR de chaîne lourde humanisée (H-huFR) possédant une séquence d'acides aminés identique à au moins 75% à la FR humaine sélectionnée à l'opération g),
i) la répétition des opérations f) à h) pour chacune des h-FR dans la zone V de chaîne lourde non humaine de façon à produire une pluralité de séquences ADN dans lesquelles chaque séquence ADN code une H-huFR,
j) la substitution dans ledit premier vecteur ou un deuxième vecteur codant au moins le domaine V de chaîne lourde de l'anticorps non humain de chacune des séquences ADN H-huFR à l'opération i) par les h-FR correspondantes codées par le vecteur, où la substitution lie de manière opérationnelle chacune des H-huFR à une CDR de chaîne lourde correspondante, et
k) l'expression du vecteur ou des vecteurs dans les mêmes cellules hôtes et dans des conditions propices à la production de chaînes lourdes et légères humanisées et la fabrication de l'anticorps humanisé ou du fragment de celui-ci.

16. Le procédé selon la Revendication 15, où les ADN codant les chaînes lourdes et légères humanisées ou des fragments de celles-ci sont contenus dans ledit premier vecteur et sont co-exprimés dans le même hôte.

17. Les procédés selon les Revendications 15 et 16, où l'hôte est mammifère, végétal, aviaire ou microbien.

18. Le procédé selon la Revendication 2 ou 15, où le premier vecteur comprend en outre un domaine constant de chaîne légère humain ou un fragment de celui-ci lié de manière covalente au domaine V de chaîne légère humanisé.

19. Le procédé selon la Revendication 18, où le domaine constant de chaîne légère est CK, Cγ ou un fragment de celui-ci.

20. Le procédé selon la Revendication 19, où le fragment de chaîne légère humanisé possède une longueur d'acides aminés se situant entre 95 et 235 acides aminés.

21. Le procédé selon la Revendication 15, où le deuxième vecteur comprend en outre un domaine constant de chaîne lourde humain ou un fragment de celui-ci lié de manière covalente au domaine V de chaîne lourde humanisé.

22. Le procédé selon la Revendication 11 ou 21, où the domaine constant de chaîne lourde humain est un isotype parmi IgG1, IgG2, IgG3 ou IgG4.

23. Le procédé selon l'une quelconque des Revendications précédentes, où le procédé comprend en outre la purification de l'anticorps humanisé ou du domaine V d'anticorps humanisé à partir des cellules hôtes de façon à produire une préparation sensiblement pure de l'anticorps.

24. Le procédé selon la Revendication 23, où l'anticorps humanisé sensiblement purifié se lie de manière spécifique à un antigène avec une affinité non inférieure à un dixième de celle de l'anticorps non humain parental.

25. Le procédé selon la Revendication 24, où l'anticorps non humain parental est chimérique.

26. Le procédé selon la Revendication 23, où l'anticorps reconnaît spécifique et se lie à un acide lipotéichoïque ou un facteur tissulaire humain.

27. Le procédé selon la Revendication 15, où le procédé comprend en outre la fabrication d'un anticorps à chaîne unique humanisé (sc-Fv) à partir des domaines V humanisés.

28. Le procédé selon la Revendication 15, 16 ou 23, où le fragment de l'anticorps humanisé est un fragment parmi F(ab') 2, Fab' ou Fab.
